# EUROPEAN PATENT SPECIFICATION

(11) **EP 2 995 305 B1**
(45) Date of publication and mention of the grant of the patent: **04.03.2020**
(21) Application number: 15172998.5
(22) Date of filing: 27.01.2012
(51) Int. Cl.: A61K 31/44, A61P 25/14

(54) **USE OF POTASSIUM CHANNEL BLOCKERS TO TREAT CEREBRAL PALSY**
VERWENDUNG VON KALIUMKANALBLOCKERN ZUR BEHANDLUNG VON ZEREBRALPARESE
UTILISATION DE BLOQUEURS DES CANAUX POTASSIQUES POUR TRAITER UNE PARALYSIE MOTRICE CÉRÉBRALE

(30) Priority: 28.01.2011 US 201161437558 P
(43) Date of publication of application: 16.03.2016
(62) Divisional of application: 12702948.6
(73) Proprietor: Acorda Therapeutics, Inc., Hawthorne, NY 10532 (US)
(72) Inventor: WESSEL, Thomas C., MA 12040 (US); RABINOWICZ, Adrian, L., NJ 07932 (US); HENEY III, Herbert, R., Clarksboro NJ 08020 (US); RUNYAN, Jacob, D., New Milford CT 06776 (US); CAGGIANO, Anthony, Larchmont NY 10538 (US); BLIGHT, Andrew, Mahopac NY 10541 (US)
(74) Representative: Carpmaels & Ransford LLP

(56) References cited:
- WO-A1-94/14439
- WO-A1-2010/093838
- WO-A1-2011/019845
- WO-A2-2005/099701
- WO-A2-2008/036294
- JI HAITAO ET AL: "Prevention of cerebral palsy with de novo designed neuronal nitric oxide synthase selective inhibitors", NITRIC OXIDE, vol. 19, no. Suppl. S, ABS. O69, 2008, page S36, XP002670416, 5TH INTERNATIONAL CONFERENCE ON BIOLOGY, CHEMISTRY, AND THERAPEUTIC APPLICATIONS OF NITRIC OXIDE; BREGENZ, AUSTRIA; AUGUST 24 -28, 2008 ISSN: 1089-8603, DOI: 10.1016/J.NIOX.2008.06.070
- HAWAMDEH Z M ET AL: "Long-term effect of botulinum toxin (A) in the management of calf spasticity in children with diplegic cerebral palsy", EUROPA MEDICOPHYSICA, vol. 43, no. 3, 1 September 2007 (2007-09-01), pages 311-318, XP009156868, MINERVA MEDICA, TORINO, IT ISSN: 0014-2573
- JOHNSTON MICHAEL V ET AL: "Models of cerebral palsy: which ones are best?", JOURNAL OF CHILD NEUROLOGY DEC 2005, vol. 20, no. 12, December 2005 (2005-12), pages 984-987, XP002752592, ISSN: 0883-0738

## Description

### 1. FIELD OF THE INVENTION

The present invention relates to treating pathological conditions that have effects in the central nervous system of mammals. In particular, the invention relates to use of one or more aminopyridines to improve the signs or symptoms of cerebral palsy.

### 2. BACKGROUND OF THE INVENTION

Cerebral palsy (CP) is an umbrella term encompassing a group of non-progressive (Lin, 2003, J Neurol Neurosurg Psychiatry 74: i23-i29), non-contagious motor conditions that cause physical disability in human development, chiefly in the various areas of body movement (Beukelman and Mirenda, 1999, Augmentative and Alternative Communication: Management of severe communication disorders in children and adults (2nd ed.), Baltimore: Paul H Brookes Publishing Co. pp. 246-249). Here, cerebral refers to the cerebrum, which is the affected area of the brain (although the disorder most likely involves connections between the cortex and other parts of the brain such as the cerebellum), and palsy refers to disorder of movement. Cerebral palsy's nature as an umbrella term means it is defined mostly via several different subtypes, and various groupings/mixtures of those subtypes.

Cerebral palsy is caused by damage to the motor control centers of the developing brain and can occur during pregnancy, during childbirth or after birth up to about age three ("Cerebral Palsy - Topic Overview", retrieved 2008-02-06 from WebMD webpage). Resulting limits in movement and posture cause activity limitation and are often accompanied by disturbances of sensation, depth perception and other sight-based perceptual problems, communication ability, and sometimes even cognition; sometimes a form of CP may be accompanied by epilepsy. Patients may have comorbidities including epilepsy (Carlsson et al., 2003, Dev Med Child Neurol 45: 371-376; Humphreys et al., 2007, J Child Neurol 22: 598-605; Zelnik et al., 2010, Eur J Paediatr Neurol 14: 67-72), auditory, visual, or somatic sensory disturbances (Krigger et al., 2006, Am Fam Physician 73: 91-100; Koman et al., 2004, Lancet 363: 1619-1631; Johnston et al., 2006, Neuromolecular Med 8: 435-450), growth problems (Krigger et al., 2006, Am Fam Physician 73: 91-100), and intellectual impairment (Krigger et al., 2006, Am Fam Physician 73: 91-100). Epilepsy affects approximately 30% of the CP population (Zelnik et al., 2010, Eur J Paediatr Neurol 14: 67-72). CP, no matter what the type, is often accompanied by secondary musculoskeletal problems that arise as a result of the underlying etiology (Anonymus, 2007, Dev Med Child Neurol 49: 8).

Of the many types and subtypes of CP, none of them has a known cure. Usually, medical intervention is limited to the treatment and prevention of complications arising from CP's effects. A 2003 study put the economic cost for people with CP in the US at $921,000 per individual, including lost income (Centers for Disease Control and Prevention, 2004, MMWR Morb Mortal Wkly Rep 53: 57-59). In the last 40 years the prevalence of CP has risen to well above 2.0 per 1000 live births (Odding et al., 2006, Disabil Rehabil 28: 183-191). Improvements in neonatology, or the medical specialty which is involved with treatment of neonates, have helped reduce the number of babies who develop cerebral palsy, but the survival of babies with very low birth weights has increased, and these babies are more likely to have cerebral palsy ("Information: Scope", retrieved 2007-12-08 from Scope webpage; and "Medical News: Cerebral Palsy Rates Decline in Very Low Birthweight Children", retrieved 2007-12-08 from Neurology, General Neurology, MedPage Today webpage). WO2010/093838 describes compositions and methods for using aminopyridines for patients with a demyelinating condition such as multiple sclerosis. WO2005/099701 describes methods of using sustained release aminopyridine compositions to treat neurological diseases such as multiple sclerosis. WO94/14439 describes the use of 4-aminopyridine in the treatment of a neurological condition such as spinal cord injury. WO2008/036294 describes NOS inhibitors for treatment of motor deficit disorders.

### 2.1 Etiology and Risk Factors

The known etiologies of CP include intrauterine infection or inflammation (Johnston et al., 2006, Neuromolecular Med 8: 435-450; Bax et al., 2006, JAMA 296: 1602-1608), fetal trauma or stroke, notably intraventricular hemorrhage (Johnston et al., 2006, Neuromolecular Med 8: 435-450; Folkerth et al., 2005, J Child Neurol 20: 940-949; Derrick et al., 2007, Stroke 38:731-735), and less frequently, congenital heart or respiratory disease (Folkerth et al., 2005, J Child Neurol 20: 940-949) or postnatal brain damage (Krigger et al., 2006, Am Fam Physician 73: 91-100). In the developed world, the major risk factor is low-birthweight prematurity (Bax et al., 2006, JAMA 296: 1602-1608).

As CP risk factors, prematurity and low birthweight appear to represent heightened risk of white-matter damage (Volpe et al., 2001, Pediatric Res 50: 553-562; Back et al., 2007, Stroke 38: 724-730). The most common CP-related neuropathology, strongly associated with the spastic diplegia form of CP, is periventricular white-matter injury (Bax et al., 2006, JAMA 296: 1602-1608; Deng et al., 2008, Arch Neurol 65: 1291-1295). Referred to as periventricular leukomalacia (PVL) or cerebral leukoencephalopathy (Johnston et al., 2006, Neuromolecular Med 8: 435-450), it is most often diagnosed in the neonatal intensive care area by head ultrasound (Beaino et al., 2010, Dev Med Child Neurol 52: e119-e125; Deng et al., 2008, Arch Neurol 65: 1291-1295). Magnetic resonance imaging (MRI), while not always feasible in unstable premature infants, is highly sensitive in detecting PVL (Johnston et al., 2006, Neuromolecular Med 8: 435-450; Bax et al., 2006, JAMA 296: 1602-1608; Deng et al., 2008, Arch Neurol 65: 1291-1295) and especially, when done repeatedly and with diffusion tensor imaging, can provide prognostic information by documenting gray matter atrophy and degeneration in white matter tracts (Thomas et al., 2005, Brain 128: 2562-2577; Hoon et al., 2009, Dev Med Child Neurol 51: 697-704; Beckung et al., 2008, Pediatrics 121: e187-e192).

### 2.2 Incidence and Prevalence

The overall prevalence of CP in the developed world is estimated to be approximately 0.2%, or 2 cases per 1,000 (Krigger et al., 2006, Am Fam Physician 73: 91-100; Odding et al., 2006, Disabil Rehabil 28: 183-191; Johnston et al., 2006, Neuromolecular Med 8: 435-450; Wu et al, 2006,. Pediatrics 118: 690-697). The incidence is higher in males than in females; the Surveillance of Cerebral Palsy in Europe (SCPE) reports a M:F ratio of 1.33:1 (Johnson et al., 2002, Dev Med Child Neurol 44: 633-640). Variances in reported rates of incidence across different geographical areas in industrialized countries are thought to be caused primarily by discrepancies in the criteria used for inclusion and exclusion. When such discrepancies are taken into account in comparing two or more registers of patients with cerebral palsy (for example, the extent to which children with mild cerebral palsy are included), the incidence rates converge toward the average rate of 2:1000.

The prevalence of CP increases dramatically with low birthweight, exceeding 10 percent among very-low (<1,500 g) - or extremely-low-birthweights (<1,000 g) (Mikkola et al., 2005, Pediatrics 116: 1391-1400; Robertson et al., 2007, JAMA 297: 2733-2740; Beaino et al., 2010, Dev Med Child Neurol 52: e119-e125). In tandem with the increased survival of low-birthweight infants (O'Shea et al., 1998, Pediatrics 101: 642-647) and the increased longevity of adults with CP (Strauss et al., 1998, Dev Med Child Neurol 40: 369-375; Hemming et al., 2006, Dev Med Child Neurol 48: 90-95), the prevalence of CP throughout the developed world's population may be increasing. In the U.S., an estimated 800,000 persons have CP symptoms; approximately 10,000 newborns per year are diagnosed with CP, and 1200-1500 are diagnosed at preschool age ("Cerebral Palsy: Hope through research," National Institute of Neurological Disorders and Stroke (NINDS) webpage, accessed April 20, 2011). The precise number of adults with CP is unknown. However, it has been established that about 95% of children with diplegia and 75% of children with quadriplegia survive to the age of 30 years. Overall the survival of children with CP to the age of 20 years is 90% (Rapp CE et al., Arch Fam Med. 2000;9:466-472).

Prevalence of cerebral palsy is best calculated around the school entry age of about six years, the prevalence in the U.S. is estimated to be 2.4 out of 1000 children (Hirtz et al., 2007, Neurology 68: 326-337).

The Surveillance of Cerebral Palsy in Europe (SCPE) reported the following incidence of comorbidities in children with CP (the data are from 1980-1990 and included over 4,500 children over age 4 whose CP was acquired during the prenatal or neonatal period):
Mental disadvantage (IQ < 50): 31%
Active seizures: 21%
Mental disadvantage (IQ < 50) and not walking: 20%
Blindness: 11%[76]

The SCPE noted that the incidence of comorbidities is difficult to measure accurately, particularly across centers. For example, the actual rate of an intellectual impairment may be difficult to determine, as the physical and communicational limitations of people with CP would likely lower their scores on an IQ test if they were not given a correctly modified version of the IQ test.

### 2.3 Classifications of CP

Cerebral palsy (CP) is divided into four major classifications to describe different movement impairments. These classifications also reflect the areas of the brain that are damaged. The four major classifications are: Spastic, Ataxic, Athetoid/dyskinetic, and Hypotonic.

### Spastic

Spastic cerebral palsy is by far the most common type of overall cerebral palsy, occurring in 70% to 80% of all cases. Moreover, spastic CP accompanies any of the other types of CP in 30% of all cases.

People with spastic CP are hypertonic and have what is essentially a neuromuscular mobility impairment (rather than hypotonia or paralysis) stemming from an upper motor neuron lesion in the brain as well as the corticospinal tract or the motor cortex, this damage impairs the ability of some nerve receptors in the spine to properly receive gamma amino butyric acid, leading to hypertonia in the muscles signaled by those damaged nerves.

### Ataxic

Ataxia type symptoms can be caused by damage to the cerebellum. The forms of ataxia are less common types of cerebral palsy, occurring in at most 10% of all cases. Some of these individuals have hypotonia and tremors. Motor skills such as writing, typing, or using scissors might be affected, as well as balance, especially while walking. It is common for individuals to have difficulty with visual and/or auditory processing.

### Athetoid/dyskinetic

Athetoid or dyskinetic cerebral palsy is mixed muscle tone - both hypertonia and hypotonia. People with athetoid CP have trouble holding themselves in an upright, steady position for sitting or walking, and often show involuntary motions. For some people with athetoid CP, it takes a lot of work and concentration to get their hand to a certain spot (like scratching their nose or reaching for a cup). Because of their mixed tone and trouble keeping a position, they may not be able to hold onto objects, especially small ones requiring fine motor control (such as a toothbrush or pencil). About one-quarter of all people with CP have athetoid CP; it occurs in 10% to 20% of all cases ("Athetoid/Dyskinetic Cerebral Palsy", retrieved 2009-10-27 from Cerebral Palsy Information webpage). The damage occurs to the extrapyramidal motor system and/or pyramidal tract and to the basal ganglia. In newborn infants, high bilirubin levels in the blood, if left untreated, can lead to brain damage in certain areas (kernicterus), which can lead to athetoid cerebral palsy.

### Hypotonic

Hypotonia is the opposite of hypertonia; people with hypotonic CP have musculature that is limp, and can move only a little or not at all. Although physical therapy is usually attempted to strengthen the muscles (in a similar way to how PT is used to stretch and loosen the tight muscles of hypertonic individuals), it is not always fundamentally effective.

### 2.4 Prognosis

CP is not a progressive disorder (meaning the brain damage neither improves nor worsens), but the symptoms can become more severe over time due to subdural damage. A person with the disorder may improve somewhat during childhood if he or she receives extensive care from specialists, but once bones and musculature become more established, orthopedic surgery may be required for fundamental improvement. People who have CP tend to develop arthritis at a younger age than normal because of the pressure placed on joints by excessively toned and stiff muscles.

The full intellectual potential of a child born with CP will often not be known until the child starts school. People with CP are more likely to have some type of learning disability, but this is not related to a person's intellect or IQ level. Intellectual level among people with CP varies from genius to intellectually impaired, as it does in the general population, and experts have stated that it is important to not underestimate a person with CP's capabilities and to give them every opportunity to learn (Jenks et al., 2007, Dev Neuropsychol 32: 861-879).

The ability to live independently with CP varies widely depending on the severity of each case. Some individuals with CP will require personal assistant services for all activities of daily living. Others can lead semi-independent lives, needing support only for certain activities. Still others can live in complete independence. The need for personal assistance often changes with increasing age and associated functional decline. However, in most cases, persons with CP can expect to have a normal life expectancy; survival has been shown to be associated with the ability to ambulate, roll, and self-feed (Strauss et al., 2008, Dev Med Child Neurol 50: 487-493). As the condition does not directly affect reproductive function, some persons with CP have children and parent successfully.

According to OMIM, only 2% of cases of CP are inherited (with glutamate decarboxylase-1 as one known enzyme involved) ("Mendelian Inheritance in Man" (OMIM) webpage, entry number *603513).

### 2.5 Signs and symptoms

The diagnosis often emerges from observations of abnormally slow motor development, abnormal muscle tone, unusual posture, or persistence of infantile reflexes in the absence of any alternative genetic or metabolic causes (Krigger et al., 2006, Am Fam Physician 73: 91-100). All types of cerebral palsy are characterized by abnormal muscle tone (i.e. slouching over while sitting), reflexes, or motor development and coordination. There can be joint and bone deformities and contractures (permanently fixed, tight muscles and joints). The classical symptoms are spasms, other involuntary movements (e.g. facial gestures or tics), unsteady gait, problems with balance, and/or soft tissue findings consisting largely of decreased muscle mass. Scissor walking (where the knees come in and cross) and toe walking (which can contribute to a gait reminiscent of a marionette) are common among people with CP who are able to walk, but taken overall, CP symptomatology is very diverse. The effects of cerebral palsy fall on a continuum of motor dysfunction that may range from slight clumsiness at the mild end of the spectrum to impairments so severe that they render coordinated movement virtually impossible at the other end the spectrum.

Babies born with severe CP often have an irregular posture; their bodies may be either very floppy or very stiff. Birth defects, such as spinal curvature, a small jawbone, or a small head sometimes occur along with CP. Symptoms may appear or change as a child gets older. Some babies born with CP do not show obvious signs right away. Classically, CP becomes evident when the baby reaches the developmental stage at six and a half to 9 months and is starting to mobilize, where preferential use of limbs, asymmetry or gross motor developmental delay is seen.

Although the underlying insult of CP is static, its complications are not (Krigger et al., 2006, Am Fam Physician 73: 91-100). Among such complications, impairment of walking is common. In a study of 159 five-year-old French children with CP (Beaino et al., 2010, Dev Med Child Neurol 52: e119-e125), 67% could walk unaided, but 14% required an aid to walk, and 19% were unable to walk. In a larger study of 10,004 patients in a European CP database (Beckung et al., 2008, Pediatrics 121: e187-e192), 54% could walk unaided at five years of age, 16% required assistive devices, and 30% were unable to walk. As patients age, a combination of chronic pain, fatigue, osteopenia, osteoporosis, bone fractures, and osteoarthritis may exacerbate their loss of ambulation and independence (Krigger et al., 2006, Am Fam Physician 73: 91-100). In data derived from a California developmental-disabilities database, patients with CP who were mobile when they reached adulthood showed a marked decline in ambulation: at 20 years old, 39% of patients could walk well without support for at least 20 feet, while 35% did not walk even with support. However at 60 years old, only 25% were able to walk well without support and 40% did not walk at all. By contrast, many other skills such as speech and self-feeding seemed to be preserved (Strauss et al., 2004, NeuroRehabilitation 19: 69-78). While the survival rate of ambulatory older adults was only moderately worse than that of the general population, the survival of those with lost mobility was much poorer (Strauss et al., 2004, NeuroRehabilitation 19: 69-78).

Secondary conditions can include seizures, epilepsy, apraxia, dysarthria or other communication disorders, eating problems, sensory impairments, mental retardation, learning disabilities, and/or behavioral disorders.

Speech and language disorders are common in people with Cerebral Palsy. The incidence of dysarthria is estimated to range from 31% to 88%. Speech problems are associated with poor respiratory control, laryngeal and velopharyngeal dysfunction as well as oral articulation disorders that are due to restricted movement in the oral-facial muscles. There are three major types of dysarthria in cerebral palsy: spastic, dyskinetic (athetosis) and ataxic.

Speech impairments in spastic dysarthria involve four major abnormalities of voluntary movement: spasticity, weakness, limited range of motion and slowness of movement. Speech mechanism impairment in athetosis involves a disorder in the regulation of breathing patterns, laryngeal dysfunction (monopitch, low, weak and breathy voice quality). It is also associated with articulatory dysfunction (large range of jaw movements), inappropriate positioning of the tongue, instability of velar elevation.

Athetoid dysarthria is caused by disruption of the internal sensorimotor feedback system for appropriate motor commands, which leads to the generation of faulty movements that are perceived by others as involuntary.

Ataxic dysarthria is uncommon in cerebral palsy. The speech characteristics are: imprecise consonants, irregular articulatory breakdown, distorted vowels, excess and equal stress, prolonged phonemes, slow rate, monopitch, monoloudness and harsh voice (Russle J. Love, 1999, Childhood Motor Speech Disability: Childhood dysarthria). Overall language delay is associated with problems of mental retardation, hearing impairment and learned helplessness (Beukelman and Mirenda, 1999, Augmentative and Alternative Communication: Management of severe communication disorders in children and adults (2nd ed.), Baltimore: Paul H Brookes Publishing Co. pp. 246-249). Children with cerebral palsy are at risk of learned helplessness and becoming passive communicators, initiating little communication (Beukelman and Mirenda, 1999).

### Skeletal Malformations

In order for bones to attain their normal shape and size, they require the stresses from normal musculature. Osseous findings will therefore mirror the specific muscular deficits in a given person with CP. The shafts of the bones are often thin (gracile) and become thinner during growth. When compared to these thin shafts (diaphyses), the centers (metaphyses) often appear quite enlarged (ballooning). With lack of use, articular cartilage may atrophy, leading to narrowed joint spaces. Depending on the degree of spasticity, a person with CP may exhibit a variety of angular joint deformities. Because vertebral bodies need vertical gravitational loading forces to develop properly, spasticity and an abnormal gait can hinder proper and/or full bone and skeletal development. People with CP tend to be shorter in height than the average person because their bones are not allowed to grow to their full potential. Sometimes bones grow to different lengths, so the person may have one leg longer than the other.

### Pain and sleep disorders

Pain is common, which may result from the inherent deficits associated with the condition, along with the numerous therapeutic procedures children typically face (McKearnan et al., 2004, J Neurosci Nurs 26: 252-259). There is also a high likelihood of suffering from chronic sleep disorders associated with both physical and environmental factors (Newman et al., 2006, Dev Med Child Neurol 48: 564-568).

The common signs and symptoms associated with CP can have a significant impact on participation in "occupations." As used herein, occupation is a term related to occupational therapy that refers to all activities a person does throughout their day. These activities may be grouped into the categories of self-care, productivity and leisure activities. Impairments related to CP can impact these activities. For example, children with motor impairments may also experience difficulties moving around their home and community, such as transportation, moving from room to room or transferring from wheelchair to toilet.

### 2.6 Treatment

As noted, there is no cure for cerebral palsy. Many of the existing forms of therapy are directed to helping a person with the disorder to function and live more effectively. In general, the earlier treatment begins the better chance children have of overcoming developmental disabilities or learning new ways to accomplish the tasks that challenge them.

The earliest interventions occur during the infant's recovery in the neonatal intensive care unit (NICU), and may continue throughout development. Treatment may include one or more of the following: physical therapy; occupational therapy; speech therapy; drugs to control seizures, alleviate pain, or relax muscle spasms (e.g. benzodiazepienes, baclofen, tizanadine and intrathecal phenol/baclofen); hyperbaric oxygen; the use of Botox(R) to relax contracting muscles; surgery to correct anatomical abnormalities or release tight muscles; braces and other orthotic devices; rolling walkers; and communication aids such as computers with attached voice synthesizers. For instance, the use of a standing frame can help reduce spasticity and improve range of motion for people with CP who use wheelchairs. Nevertheless, there is only some benefit from therapy. Treatment is usually symptomatic and focuses on helping the person to develop as many motor skills as possible or to learn how to compensate for the lack of them. Non-speaking people with CP are often successful availing themselves of augmentative and alternative communication systems such as Blissymbols.

### Medication

There are limited medications for CP, and the ones that are used are symptomatic. For example, medicinal treatments may include drugs to control seizures, alleviate pain, or relax muscle spasms (e.g. benzodiazepienes, baclofen, tizanadine and intrathecal phenol/baclofen); hyperbaric oxygen; the use of Botox(R) to relax contracting muscles. Botulinum toxin A (Botox(R)) is sometimes injected into muscles that are spastic or have contractures; the goal of such administration is to relax the muscles and lessen the limitations and pain produced by the inappropriately contracting muscle(s) (Hawamdeh et al., 2007, Europa Medicophysica 43: 311-318).

### Surgery and orthoses

Surgery usually involves one or a combination of:
a) Loosening tight muscles and releasing fixed joints, most often performed on the hips, knees, hamstrings, and ankles. In rare cases, this surgery may be used for people with stiffness of their elbows, wrists, hands, and fingers.
b) The insertion of a Baclofen(R) pump usually during the stages while a patient is a young adult. This pump is usually placed in the abdomen and is connected to the spinal cord, where it sends Baclofen with a goal of alleviating continuous muscle flexion. Baclofen is a muscle relaxant and is often given PO "per os" (Latin for "by mouth") to patients to help counter the effects of spasticity.
c) Straightening abnormal twists of the leg bones, i.e. femur (termed femoral anteversion or antetorsion) and tibia (tibial torsion). This is a secondary complication caused by the spastic muscles generating abnormal forces on the bones, and often results in intoeing (pigeon-toed gait). The surgery is called derotation osteotomy, in which the bone is broken (cut) and then set in the correct alignment (Schejbalová et al., 2006, Acta Chir Orthop Traumatol Cech 73: 334-339).
d) Cutting nerves on the limbs most affected by movements and spasms. This procedure, called a rhizotomy, "rhizo" meaning root and "tomy" meaning "a cutting of." The goal of a rhizotomy is to reduce spasms and allow more flexibility and control of the effected limbs and joints (Farmer et al., 2007, Childs Nerv Syst 23: 991).

Orthotic devices such as ankle-foot orthoses (AFOs) are often prescribed to minimize gait irregularities. AFOs have been found to improve several measures of ambulation, including reducing energy expenditure[38] and increasing speed and stride length (White et al., 2002, Dev Med Child Neurol 44: 227-232).

### 2.7 Aminopyridines

An exemplary property of certain aminopyridines is that they are potassium channel blockers.

4-AP is an example of an aminopyridine with such potassium channel blocking properties. At 4-AP plasma concentrations obtained in clinical studies, typically <1 microM (94 ng . mL⁻¹), the potassium channel blocking activity of 4-AP appears to be selective for certain types of these channels. Interestingly, at high concentration 4-AP is a broad-spectrum blocker of potassium channels (such as at millimolar concentrations). The clinical neurologic effects of 4-AP are consistent with the molecular mechanism of potassium channel blockade. At the cellular level, this action may increase neuronal excitability, relieve conduction block in demyelinated axons, and potentiate synaptic and neuromuscular transmission.

Studies of 4-aminopyridine have been conducted using intravenous (i.v.) administration and oral administration with immediate-release (IR), controlled-release (CR) or sustained-release (SR) formulations. Administration of IR capsules resulted in rapid and shortlasting peaks of 4-aminopyridine in the plasma. Early pharmacokinetic studies were conducted using an immediate release (IR) formulation for oral administration, which consisted of 4-aminopyridine powder in a gelatin-based capsule or oral solution. Administration resulted in rapidly changing 4-aminopyridine plasma levels that were not well tolerated. A sustained-release matrix tablet was then developed. The 4-aminopyridine-SR matrix tablet showed improved stability and an appropriate pharmacokinetic profile for dosing twice daily.

Dalfampridine is the United States Adopted Name (USAN) for the chemical 4-aminopyridine (4-AP). It is FDA-approved as an extended release (ER), 10 mg tablet (see Ampyra® package insert) indicated to improve walking in subjects with multiple sclerosis (MS), as demonstrated by an increase in walking speed. The approved therapeutic dose of Dalfampridine is a 10 mg extended release tablet to be taken twice daily, approximately 12 hours apart, with or without food.

The effectiveness of dalfampridine in improving walking in patients with multiple sclerosis was evaluated in two double-blind, placebo-controlled phase 3 trials involving a total of 540 patients (Goodman et al., 2009, Lancet 373: 732-738; Goodman et al., 2010, Ann Neurol 68:494-502. The primary measure of efficacy in both trials was walking speed (in feet per second) as measured by the Timed 25-foot Walk (T25FW), using a responder analysis. A Responder was defined as a patient who showed faster walking speed for at least three visits out of a possible four during the double-blind period than the maximum value achieved in the five non-treatment visits. A statistically significantly greater proportion of patients taking dalfampridine-ER 10 mg twice daily were Responders, compared to patients taking placebo. During the double-blind treatment period, a statistically significantly greater proportion of patients taking dalfampridine had increases in walking speed of at least 10%, 20%, or 30% from baseline, compared to placebo. In both trials, consistent improvements in walking speed were shown to be associated with improvements on a patient self-assessment of ambulatory disability, the 12-item Multiple Sclerosis Walking Scale (MSWS-12).

Leg strength, assessed using the Lower Extremity Manual Muscle Test (LEMMT) was also studied in patients with multiple sclerosis. Leg strength was found to be statistically significantly improved with dalfampridine relative to placebo (p<0.05) (Goodman et al., 2008, Neurology 71: 1134-1141; Goodman et al., 2009, Lancet 373: 732-738; Goodman et al., 2010, Ann Neurol 373: 494-502).

There remains a need in the art for methods of ameliorating the symptoms of cerebral palsy. An unmet need exists in the art for effective treatments for the signs and symptoms of cerebral palsy. In particular, there is as need for medicinal treatments that facilitate performance of or restore function of nerves impacted by cerebral palsy.

Citation of a reference herein shall not be construed as an admission that such is prior art to the present invention.

### 3. SUMMARY OF THE INVENTION

The invention relates to an aminopyridine or a pharmaceutically acceptable salt thereof for use in a method for treating a sign or symptom of cerebral palsy ("CP") or an impairment or alteration consequent to CP, said method comprising administering the aminopyridine or pharmaceutically acceptable salt thereof to a patient with CP, wherein the symptom of CP or the impairment or alteration consequent to CP is not an impairment in mental status, preferably wherein the patient is human. Also described is the use of aminopyridines, such as 4-aminopyridine or 4-aminopyridine-SR, in a dosing regimen that serves to improve a sign or symptom of cerebral palsy (CP) in the patient.

Also disclosed is a method for treating a sign or symptom of cerebral palsy (CP) or an impairment or alteration consequent to CP by administering an aminopyridine or a pharmaceutically acceptable salt thereof to a patient with CP. In specific embodiments, the methods described herein comprise administering a therapeutically effective amount of an aminopyridine or a pharmaceutically acceptable salt thereof. In one embodiment, a composition comprising a therapeutically effective amount of an aminopyridine and a pharmaceutically acceptable carrier is administered to a patient with CP. In some embodiments, the described methods include a step of assessing a CP sign or symptom in a patient before administration of an aminopyridine or a pharmaceutically acceptable salt thereof, and/or a step of assessing the same CP sign or symptom after administration of the aminopyridine or a pharmaceutically acceptable salt thereof. In specific embodiments, a composition administered to a patient does not comprise a pharmaceutically acceptable salt of an aminopyridine. In one embodiment, the aminopyridine is 4-aminopyridine. In another embodiment, the aminopyridine is 3-aminopyridine. In yet another embodiment, the aminopyridine is 3,4-diaminopyridine.

In some embodiments, an aminopyridine or a pharmaceutically acceptable salt thereof is administered to a patient in a sustained release composition. In other embodiments, an aminopyridine or a pharmaceutically acceptable salt thereof is administered to a patient in an immediate release composition. In some embodiments, an aminopyridine or a pharmaceutically acceptable salt thereof is administered to the patient once daily. In other embodiments, an aminopyridine or a pharmaceutically acceptable salt thereof is administered to the patient twice daily. In specific embodiments of all the dosage regimens described herein, an aminopyridine or a pharmaceutically acceptable salt thereof is administered to the patient orally (e.g., as a tablet, a pill or a capsule). In one embodiment, an aminopyridine or a pharmaceutically acceptable salt thereof is formulated in a form of a tablet for administration to a patient. In one embodiment, an aminopyridine or a pharmaceutically acceptable salt thereof is formulated in a form of a capsule for administration to a patient.

In particular embodiments, an aminopyridine or a pharmaceutically acceptable salt thereof is administered to a patient in an amount in the range of 5 to 20 mg, 5 to 15 mg, 5 to 10 mg, or 7.5 to 10 mg once or twice daily, preferably in a sustained release composition. For example, 5, 6, 7, 7.5, 8, 9, 10, 11, 12, 12.5, 13, 14, 15, 16, 17, 17.5, 18, 19, 20, 25, 30, 35, or 40 mg of an aminopyridine or a pharmaceutically acceptable salt thereof can be administered to a patient once daily, preferably in a sustained release composition, or 5, 6, 7, 7.5, 8, 9, 10, 11, 12, 12.5, 13, 14, 15, 16, 17, 17.5, 18, 19, or 20 mg of an aminopyridine or a pharmaceutically acceptable salt thereof can be administered to a patient twice daily, preferably in a sustained release composition. In one embodiment, an aminopyridine or a pharmaceutically acceptable salt thereof is administered in the amount of 10 mg once daily, preferably in a sustained release composition. In another embodiment, an aminopyridine or a pharmaceutically acceptable salt thereof is administered in the amount of 10 mg twice daily, preferably in a sustained release composition.

In specific embodiments, a therapeutically effective amount of an aminopyridine or a pharmaceutically acceptable salt thereof is in the range of 5 to 20 mg, 5 to 15 mg, 5 to 10 mg, or 7.5 to 10 mg once or twice daily, preferably in a sustained release composition. For example, a therapeutically effective amount of an aminopyridine or a pharmaceutically acceptable salt can be 5, 6, 7, 7.5, 8, 9, 10, 11, 12, 12.5, 13, 14, 15, 16, 17, 17.5, 18, 19, 20, 25, 30, 35, or 40 mg once daily, preferably in a sustained release composition, or 5, 6, 7, 7.5, 8, 9, 10, 11, 12, 12.5, 13, 14, 15, 16, 17, 17.5, 18, 19, or 20 mg twice daily, preferably in a sustained release composition.

Also described herein are methods for treating one or more sensorimotor or motor impairments in a patient with CP. In particular, the sign or symptom of CP treated in accordance with the methods described herein can be one or more of walking or ambulation dysfunction, impairment in walking speed, impairment in limb function, motor dysfunction of the upper extremities, motor dysfunction of the lower extremities, sensorimotor impairment in lower extremity function, sensorimotor impairment in upper extremity function, dysfunctional muscle strength, impairment in lower extremity muscle strength, impairment in upper extremity muscle strength, impairment in muscle tone, impairment in hand function, impairment in hand strength, impairment in fine hand coordination, impairment in manual dexterity, impairment in grip strength, impairment in balance or coordination, impairment in global body control, speech dysfunction, dysarthria, impairment in jaw function, impairment in chewing, impairment in jaw articulation, motor dysfunction in muscle groups of the body core, motor dysfunction in postural musculature, impairment in muscle tone, impairment in reflexes, spasticity, involuntary movements, impairment in gait, or decreased muscle mass (e.g., regardless of muscle groups involved). In particular embodiments, the sign or symptom of CP treated in accordance with the methods described herein is not cognition (i.e., not a cognition impairment), and/or not a neuro-psychiatric or a neuro-cognitive impairment.

In certain embodiments, the patient treated in accordance with the methods described herein is a mammal (e.g., a human). In preferred embodiments, the patient treated in accordance with the methods described herein is a human diagnosed with CP.

Described herein is a method for treating a sign or symptom of cerebral palsy (CP) comprising administering a potassium channel blocker to a patient with cerebral palsy (CP). Such method can further include a step of assessing a CP sign or symptom before administration of the potassium channel blocker, and a step of assessing the same CP sign or symptom after administration of the potassium channel blocker. In some embodiments of the described method, the potassium channel blocker is an aminopyridine. In one embodiment of the described method, the potassium channel blocker is a 3-aminopyridine. In one embodiment of the described method, the potassium channel blocker is a 4-aminopyridine. In one embodiment of the described method, the potassium channel blocker is a 3,4-diaminopyridine. The sign or symptom of CP treated in accordance with the method described herein can be one of motor dysfunction, the motor dysfunction can be of the extremities, such as upper or lower extremities or muscle groups of the body core, such as postural musculature. The sign or symptom of CP treated in accordance with the method described herein can be a one of speech dysfunction. The sign or symptom of CP treated in accordance with the method described herein can be one of walking or ambulation dysfunction. The sign or symptom of CP treated in accordance with the method described herein can be one of dysfunctional muscle strength. The sign or symptom of CP treated in accordance with the method described herein can be at least one of: muscle tone, reflexes, coordination, spasticity, involuntary movements, gait, balance or decreased muscle mass, regardless of muscle groups involved.

In some embodiments, the patient with CP treated in accordance with the methods described herein is less than 1, 2, 3, 4, 5, 6, 7, 8, 9, 10, 11 or 12 months old. In other embodiments, the patient with CP is less than 1, 2, 3, 4, 5, 6, 7, 8, 9, 10, 11, 12, 13, 14, 15, 16, 17, 18, 19 or 20 years old. In yet other embodiments, the patient with CP is more than 1, 2, 3, 4, 5, 6, 7, 8, 9, 10, 11, 12, 13, 14, 15, 16, 17, 18, 19 or 20 years old. In specific embodiments, the patient with CP is more than 20, 25, 30, 35, 40, 45 or 50 years old.

In certain embodiments of the methods described herein, wherein a patient is administered an aminopyridine (e.g., 3-aminopyridine, 4-aminopyridine or 3,4-diaminopyridine), the aminopyridine is administered in an amount that elicits a Cₘᵢₙₛₛ of at least 10, 11, 12, 13, 14, 15, 16, 17, 18, 19 or 20 ng/ml. In some embodiments wherein a patient is administered an aminopyridine (e.g., 3-aminopyridine, 4-aminopyridine or 3,4-diaminopyridine), the aminopyridine is administered in an amount that elicits a Cₘₐₓₛₛ of less than 30, 35, 40, 45, 50, 55, 60, 65, 70 75 or 80 ng/ml. In other embodiments wherein a patient is administered an aminopyridine (e.g., 3-aminopyridine, 4-aminopyridine or 3,4-diaminopyridine), the aminopyridine is administered in an amount that elicits a Cₘᵢₙₛₛ selected from the group consisting of at least any of 10, 11, 12, 13, 14, 15, 16, 17, 18, 19 or 20 ng/ml, and which elicits a Cₘₐₓₛₛ selected from the group consisting of less than any of 30, 35, 40, 45, 50, 55, 60, 65, 70 75 or 80 ng/ml.

Described herein is a method of treating a form of CP by administering of a potassium channel blocker as disclosed herein, whereby a sign or symptom of CP as disclosed herein is effectively improved. Also described herein is a method of treating at least one form of CP as disclosed herein by administering an aminopyridine, which is 3-aminopyridine, 4-aminopyridine or 3,4- diaminopyridine, to a patient with a form of CP as disclosed herein, and, whereby a sign or symptom of CP as disclosed herein is effectively improved in the patient.

Described herein is a method of treating CP by administering an aminopyridine or a pharmaceutically acceptable salt thereof to a patient in need thereof, whereby a sign or symptom of CP is effectively improved. In some cases, the method entails administering a composition comprising a therapeutically effective amount of an aminopyridine (e.g., 4-AP, 3-AP, or 3,4-DAP) and a pharmaceutically acceptable carrier.

Described herein is a method for treating a motor dysfunction by administering orally to the patient a sustained release composition of 10 mg of 4-AP once daily. Also described herein is a method for treating a motor dysfunction by administering orally to the patient a sustained release composition of 10 mg of 4-AP twice daily. The motor dysfunctions that can be treated in accordance with these methods include, but are not limited to, dysfunctions or impairments in walking speed, gait, hand strength or manual dexterity.

Also provided herein are compositions comprising an aminopyridine or a pharmaceutically acceptable salt thereof for use in a method of treating a sign or symptom of CP or an impairment or alteration to CP. Accordingly, the invention also provides a composition comprising an aminopyridine or a pharmaceutically acceptable salt thereof for use in a method of treating a form of CP.

### 3.1 DEFINITIONS

In the description, figures and tables herein, a number of terms are used. In order to provide a clear and consistent understanding of the specification and claims, the following definitions are provided:

| **Abbreviation or Specialist Term** | **Explanation** |
|---|---|
| AUC | Area under the concentration-time curve |
| AUC₍₀₋ₜ₎, AUC_{(o-∞)} or AUC_{(0-inf)} | Area under the plasma concentration *versus* time curve, to the last quantifiable level, and extrapolated to infinity |
| AUC₍₀₋₁₂₎, AUC₍₀₋₂₄₎ | Area under the plasma concentration *versus* time curve, 0-12 hours, 0-24 hours |
| b.i.d. | Twice daily |
| ¹⁴C | Radioactive carbon 14 |
| CI | Confidence interval |
| CL/F | Apparent total body clearance after administration |
| Cm | Centimeter |
| Cₘₐₓ | Maximum measured plasma concentration |
| Cₘₐₓₛₛ | Maximum measured plasma concentration at steady state |
| Cₘᵢₙ | Minimum measured plasma concentration |
| Cₘᵢₙₛₛ | Minimum measured plasma concentration at steady state |
| CNS | Central nervous system |
| CR | Controlled-release |
| ER | Extended Release |
| 3-AP | 3-Aminopyridine |
| 4-AP | 4-Aminopyridine |
| 3,4-DAP | 3,4-Diaminopyridine |
| g, kg, mg, µg, ng | Gram, kilogram, milligram, microgram, nanogram |
| h, hr | Hour |
| HDPE | High-density polyethylene |
| HPLC | High performance liquid chromatography |
| IR | Immediate-release |
| i.v. | Intravenous |
| K⁺ | Potassium |
| L, mL | Liter, milliliter |
| LCMS, LC/MS/MS | Liquid chromatography / mass spectrometry |
| Ln | Natural log |
| Min | Minute |
| mM, µM | Millimolar, micromolar |
| MS | Multiple sclerosis |
| NE | Not evaluable |
| NF | National Formulary |
| Norm | Normalized |
| pₐₚₚ | Apparent permeability coefficient |
| p.o. | Oral |
| SCI | Spinal cord injury |
| Sec | Second |
| SEM | Standard error of the mean |
| SR | Sustained-release (which is used interchangeably herein with "extended release") |
| SS | Steady state |
| Tₘₐₓ | Time of the maximum plasma concentration |
| USP | United States Pharmacopeia |
| V_{d} | Volume of distribution |
| V_{dss} | Volume of distribution at steady state |

When used in conjunction with the word "comprising" or other open language in the claims, the words "a" and "an" denote "one or more."

The term "pharmaceutically acceptable salt(s)," with reference to an aminopyridine, as used herein, refers to a salt prepared from a pharmaceutically acceptable non-toxic acid or base, including an inorganic acid or base, or an organic acid or base. In one embodiment, the pharmaceutically acceptable salt is prepared from a pharmaceutically acceptable non-toxic acid which can be an inorganic or organic acid. In one embodiment, non-toxic acids include, but are not limited to, inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. In one embodiment, the non-toxic acid is hydrochloric acid. Suitable pharmaceutically acceptable salts will be apparent to those skilled in the art and include those described in S.M. Barge et al., "Pharmaceutical Salts," 1977, J. Pharm. Sci. 66:1-19, which is incorporated herein by reference in its entirety.

### 4. BRIEF DESCRIPTION OF THE DRAWINGS

The following drawings form part of the present specification and are included to demonstrate certain aspects of the present disclosure. The invention may be better understood by reference to one of these drawings in combination with the detailed description of specific embodiments presented herein.

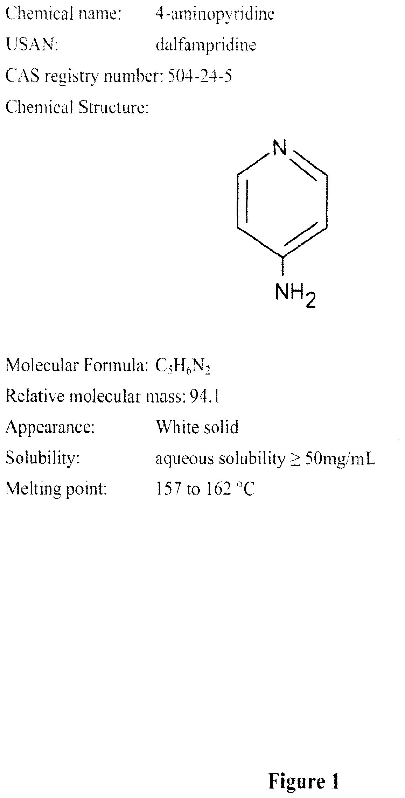

**Figure 1** shows information regarding 4-aminopyridine.
**Figure 2** is a schematic showing the timetable of dosing and behavior testing in a model of ischemia.
**Figure 3** shows the results of the forelimb placing test in a model of ischemia: X axis represents the number of days after the stroke event (i.e., days post-MCAO). The Y axis represents the behavioral score (0 to 12, with 0 being normal function and 12 being maximally impaired). The graph shows an average behavioral score of the animals in each test group (i.e., Groups I-III) at D-1, D1, D7, D14, D21, D28, D30, D32, D42, D44, D46, D56, D58, D60 as described in Section 5.5 ("D"=day).
**Figure 4** shows the results of the hindlimb placing test in a model of ischemia: X axis represents the number of days after the stroke event (i.e., days post-MCAO). The Y axis represents the behavioral score (0 to 6, with 0 being normal function and 6 being maximally impaired). The graph shows an average behavioral score of the animals in each test group (i.e., Groups I-III) at D-1, D1, D7, D14, D21, D28, D30, D32, D42, D44, D46, D56, D58, D60 as described in Section 5.5.
**Figure 5** shows the results of the body swing test in a model of ischemia: X axis represents the number of days after the stroke event (i.e., days post-MCAO). The Y axis represents the behavioral score. The graph shows an average behavioral score of the animals in each test group (i.e., Groups I-III) at D-1, D1, D7, D14, D21, D28, D30, D32, D42, D44, D46, D56, D58, D60 as described in Section 5.5.
**Figure 6** shows the average weight (g) of the animals in each test group (i.e., Groups I-III) at days (i.e., D-1, D1, D7, D14, D21, D28, D30, D32, D42, D44, D46, D56, D58, D60) after the stroke event (i.e., MCAO).
**Figure 7** shows the results of the cylinder test in a model of ischemia: X axis represents the number of days after the stroke event (i.e., days post-MCAO). The Y axis represents the behavioral score. The graph shows an average behavioral score of the animals in each test group (i.e., Groups I-III) at Day -1 (pre-operation), Day 7, Day 21, Day 30, Day 32, Day 44, Day 46, Day 58, Day 60 as described in Section 5.5.
**Figure 8** shows the total movement score of animals subjected to the cylinder test in a model of ischemia: X axis represents the number of days after the stroke event (i.e., days post-MCAO). The Y axis represents the behavioral score. The graph shows an average behavioral score of the animals in each test group (i.e., Groups I-III) at Day -1 (pre-operation), Day 7, Day 21, Day 30, Day 32, Day 44, Day 46, Day 58, Day 60.
**Figure 9** shows the study design for Part A (Cohort 1) of the clinical protocol described in Example 5.
**Figure 10** shows the study design for Part B (Cohort 2) of the clinical protocol described in Example 5.
**Figure 11** shows the study design for Part A (Cohort 1) of the clinical protocol described in Example 6.
**Figure 12** shows the study design for Part B (Cohort 2) of the clinical protocol described in Example 6.

### 5. DETAILED DESCRIPTION OF THE INVENTION

### 5.1 Potassium Channel Blocker Use in Cerebral Palsy

The invention provides potassium channel blockers for treating one or more signs or symptoms of CP. In one embodiment, the potassium channel blocker is one or more aminopyridine.

The application affords a sound prediction that at least one sign or symptom of CP, such as a sensorimotor impairment, can be treated by use of a potassium channel blocker such as an aminopyridine, in particular, 4-AP. In particular, this sound prediction arises from the appreciation of a combination of several factual bases: first, that an aminopyridine is therapeutic in promoting functional recovery after a neuronal ischemic insult as demonstrated by the data hereinbelow, and the knowledge that an ischemic insult is an etiology common to both stroke and most cases of cerebral palsy; second, the knowledge that an aminopyridine is therapeutic for multiple sclerosis, which is a disease that shares certain demyelinating characteristics with CP. This is described in more detail below.

Unlike MS, CP has a predominantly ischemic etiology since it is believed to be commonly caused by hypoxia or lack of oxygen in the developing brain (e.g., during the prenatal period) (Derrick et al., 2007, Stroke 38(2):731-735).

Notably, Acorda Therapeutics, Inc. has generated data showing that an aminopyridine, and specifically, 4-aminopyridine is therapeutic for ischemic central nervous system injury, particularly, for functional recovery of sensorimotor impairments after stroke. In particular, these data have shown that 4-AP is effective in restoring neurological function after middle cerebral artery occlusion in rats, in an accepted model for stroke in humans. As described in Section 5.5, the surprising discovery has been made that 4-aminopyridine is effective in treating chronic and stable sensorimotor impairments following an ischemic event. The data generated by Acorda Therapeutics, Inc. show efficacy when dosing is initiated during a chronic phase following an ischemic event, when stable motor deficits are present. Thus, the data indicate that an aminopyridine can treat impairments due to ischemic neuronal injury long after the ischemic event has occurred. Moreover, CP is characterized by impairments due to ischemic injury long after the ischemic event has occurred.

Further, the inventors have appreciated that CP has some of the neuropathological features seen in multiple sclerosis, and aminopyridine, in particular, 4-AP, is therapeutic for MS. Specifically, both multiple sclerosis and cerebral palsy exhibit white matter abnormalities, such as demyelination (see Stolp et al., 2009, Neuropathol Appl Neurobiol. 35:132-146). Further, inflammatory cells such as macrophages, microglia and astrocytes, that are involved in the pathogenesis of MS, have also been found in white matter lesions in post mortem studies of CP patients (see Stolp et al., 2009). Further, sustained release dalfampridine (i.e., a sustained release composition of 4 aminopyridine) is an FDA-approved drug indicated to improve walking in patients with MS. In addition, sustained release 4-AP has been shown to improve other sensorimotor impairments in MS, such lower extremity muscle strength and spasticity. However, unlike MS, the susceptibility of the brain to white matter damage in CP is considered to occur primarily during the specific stage of brain development, from 24 to 32 weeks of gestation, that corresponds to the early stages of myelination (see Stolp et al., 2009), and is believed to be largely caused by prenatal ischemia (see Derrick et al., 2007).

Thus, the surprising data obtained by Acorda Therapeutics, Inc. showing the effectiveness of an aminopyridine in the treatment of ischemia weeks after the occurrence of the ischemic event, the recognition of this data's relevance to CP, and the appreciation of the above-described similarities between CP and MS, provide a reasonable basis to conclude that an aminopyridine can be effective in the treatment of CP.

Accordingly, in preferred embodiments, an aminopyridine or a pharmaceutically acceptable salt thereof is used in the methods of treating CP provided herein.

The structure of an aminopyridine is well known in the art. As shown in U.S. Patent No. 5,952,357, a mono- or diaminopyridine has the following structure: 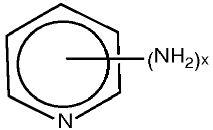 , wherein x is 1 or 2.

Aminopyridines having the above structural formula wherein x is 1 are, e.g., 2-aminopyridine, 3- aminopyridine and 4- aminopyridine. Aminopyridine compounds having the above structural formula wherein x is 2 are, e.g., 2,3-diaminopyridine; 2,5- diaminopyridine; 2,6-diaminopyridine; 3,4- diaminopyridine; 4,5- diaminopyridine and 4,6- diaminopyridine.

In one embodiment, the aminopyridine is a mono- or di-aminopyridine. In one embodiment, the mono- aminopyridine is 3-aminopyridine or 4-aminopyridine. In one embodiment the di- aminopyridine is 3,4-diaminopyridine.

As will be appreciated, a pharmaceutically acceptable salt of an aminopyridine may be used instead of or in addition to an aminopyridine in any or all of the methods of treating discussed herein. Thus, in specific embodiments, a pharmaceutically acceptable salt of an aminopyridine (i.e., any pharmaceutically acceptable salt of any of the aminopyridine compounds listed above) is used in the methods of treating CP provided herein. These salts can be prepared, for example, in situ during the final isolation and purification of the compounds or by separately reacting the purified compound in its free base form with a suitable organic or inorganic acid and isolating the salt thus formed. In some embodiments, a salt of a mono- or di-aminopyridine is used in the invention. In another embodiment, a salt of 3-aminopyridine or 4-aminopyridine is used. In yet another embodiment, a salt of 3,4-diaminopyridine is used. In some embodiments, the pharmaceutically acceptable salt of an aminopyridine is prepared using acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, or p-toluenesulfonic acid. In one embodiment, one equivalent of an aminopyridine, as used herein, may form an acid salt with less than one or with one or more than one equivalent of an acid. In one embodiment an aminopyridine, as used herein, may form a dihydrochloride salt. In one embodiment an aminopyridine, as used herein, may form a phosphate salt. For further description of pharmaceutically acceptable salts that can be used in the methods described herein see, for example, S.M. Barge et al., "Pharmaceutical Salts," 1977, J. Pharm. Sci. 66:1-19, which is incorporated herein by reference in its entirety.

In preferred embodiments, an aminopyridine itself, and not a pharmaceutically acceptable salt thereof, is used in any of the methods of treating CP described herein.

In particular, provided herein are methods for treatment of cerebral palsy in a patient comprising administering an aminopyridine or a pharmaceutically acceptable salt thereof. In certain embodiments, disclosed herein is treatment of a cerebral palsy-related impairment, sign or symptom in a patient. In certain embodiments, disclosed herein is treatment that causes improvement in one or more sensorimotor or motor impairments in a patient who has cerebral palsy.

In certain embodiments, the sign or symptom of CP treated in accordance with the methods described herein is motor dysfunction, language or speech dysfunction, walking or ambulation dysfunction (e.g., decrease in walking speed), dysfunctional muscle strength, dysfunctional muscle tone, abnormal reflexes, abnormal coordination, spasticity, involuntary movements, abnormal gait, abnormal balance, decreased muscle mass, impaired skeletal development, or impaired muscle development. In some embodiments, the sign or symptom of CP treated in accordance with the methods described herein is an impairment in hand strength, impairment in manual dexterity, impairment in walking speed, or impairment in gait. In some embodiments, the sign or symptom of CP treated in accordance with the methods described herein is a sensorimotor impairment, or an impairment in sensorimotor function, such as, but not limited to, ataxia, global body control impairments, coordination or balance impairments, impairment in body sense, impairment in proprioception, endurance impairment, impairment in hand function, impairment in hand strength, fine hand coordination loss or impairment, hyperreflexia, impairment in grip strength, muscle weakness, muscle tone impairment, range of motion impairment, spasticity, strength impairment/weakness, tremor, impairment in limb function, upper extremity function impairment, lower extremity function impairment, impairment in lower extremity muscle strength, walking impairments (e.g., decreased walking speed), impairment in the ability to stand, speech impairments (e.g., dysarthria), impairment in jaw function, impairment in chewing, and impairment in jaw articulation, impairment in dexterity, reflexes, or any other sensorimotor function described herein or known in the art.

In one embodiment, aminopyridine administration restores or improves one or more sensorimotor functions. This is manifest or measured as an improvement, e.g., in walking ability, balance, the ability to stand, hand strength, dexterity, reflexes, answers to art-accepted measures of quality of life, or improvement in any other sensorimotor function described herein or known in the art.

In specific embodiments, the treatment in accordance with the invention is effective to treat (e.g., improve, ameliorate, reduce the severity of, or reduce the duration of) the symptoms of one or more above-identified impairments (e.g., motor, sensory or sensorimotor impairments). In some embodiments, the treatment in accordance with the invention restores one or more functions (e.g., motor, sensory or sensorimotor functions) impaired due to cerebral palsy. Further described are methods for assessing the level of an impairment after (or before and after) repeated administration of an aminopyridine. Such method can be any method for evaluating a function (e.g., a motor, a sensory, or a sensorimotor function) described herein or known in the art.

In certain cases, treating a patient by administering an amount of an aminopyridine or a pharmaceutically acceptable salt thereof is effective to ameliorate a sensorimotor impairment in a patient with CP. In other cases, treating a patient by administering an amount of an aminopyridine or a pharmaceutically acceptable salt thereof is effective to alleviate the symptoms (e.g., decrease the severity) of a motor or sensorimotor impairment in a patient with CP. In a specific case, treating a patient by administering an amount of an aminopyridine or a pharmaceutically acceptable salt thereof is effective to eliminate a motor or sensorimotor impairment in a patient with CP, and/or effective to regain the sensorimotor function impaired by the CP. In certain cases, the administering of an aminopyridine or a pharmaceutically acceptable salt thereof is effective to restore a motor or sensorimotor function impaired by CP.

Also described is a method for maintaining improvement of a sensorimotor function in a patient, wherein such function is impaired due to CP, said method comprising: administering a therapeutically effective amount of an aminopyridine (such as 3,4-diaminopyridine, 4-aminopyridine and the like) or a pharmaceutically acceptable salt thereof to said patient after previously achieving an improvement of such impaired sensorimotor function in said patient during administration of 4-aminopyridine.

In one case, a method for maintaining improvement in a sensorimotor function in a patient with a CP-related impairment of such function comprises administering a therapeutically effective amount of an aminopyridine or a pharmaceutically acceptable salt thereof to said patient over an extended period of time. In another case, a method for achieving sustained improvement in a patient with a CP-related sensorimotor impairment comprises continuing administration of a therapeutically effective amount of an aminopyridine (such as 3,4-diaminopyridine, 4-aminopyridine and the like) or a pharmaceutically acceptable salt thereof to said patient over an extended period of time.

In specific embodiments, the improvement(s) among patients experiencing CP-related sensorimotor impairment occur over periods of at least or more than: 1, 2, 3, 4, 5, 6, 7, 8, 9, 10, 11, 12, 13, 14, 15,16, 17, 18, 19, 20, 21 days; 1, 2, 3, 4, 5, 6, 7, 8, 9, 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 6, or greater than 5 years of treatment.

In a specific embodiment, an aminopyridine of the invention or a pharmaceutically acceptable salt thereof is administered at a therapeutically effective dosage sufficient to treat an impairment associated with CP in a patient. In certain embodiments, the treatment reduces the amount of symptoms of the impairment in the patient by at least about 10%, more preferably 20%, more preferably by at least about 40%, even more preferably by at least about 60%, and still more preferably by at least about 80% relative to untreated subjects. Such percent change quantification is preferably applied to assays of sensorimotor function that provide measurements of results in continuous linear scales, such as T25FW, etc. Other tests of sensorimotor function will not be expressed as percent change but would be predicted to result in a significant change with the appropriate statistical comparison. Such tests include semiquantative measures that assign values to the ability to perform certain skills. In some embodiments, treatment in accordance with the invention results in a statistically significant improvement in a CP-related motor or sensorimotor impairment (e.g., as measured by the patient's ability to perform certain task or skill) in comparison to a control. Such control can be the patient's ability to perform the assessed task or skill prior to the commencement of treatment.

The functions impaired due to CP can be assessed using any method known in the art. For example, assessment tests can include, without limitation the timed 25 foot walk (T25FW), 2 minute walk, 6 minute walk (6MW), Box & Block test, Six Spot Step Test, the Manual Muscle test for lower extremity function, LEMMT, the Ashworth score, the Modified Ashworth Scale, grip strength test, 9-hole peg test, fine finger movement, rapid alternating fingers for upper extremity function, functional system scoring for sensory function, and finger-to-nose and heel-to-shin for ataxia. In particular, T25W can be used to measure walking, LEMMT can be used to measure lower extremity muscle strength, the Modified Ashworth Scale can be used to measure spasticity. In other particular examples, hand strength can be measured by the grip test, tip pinch, key pinch, and/or palmar pinch tests; manual dexterity can be measured by the Box and Block test; and gait can be measured by the following parameters: stride length, cadence, single support time, double support time, stride length variability and/or swing time variability. Art-accepted upper extremity function assessments include, without limitation, performance scale-self-report measures, hand-held dynamometry, and Upper Extremity Index (UEI). Other assessment tests that can be used to measure sensorimotor functions include but are not limited to: Berg Balance Scale (BBS), Kela Coordination Test, Postural Stability Test, Timed 10-meter Gait Test, Shoulder Tug Test, Grip Strength, Maximal isometric force of the knee extensors, muscle endurance tests, passive straight leg raise, TEMPA (upper extremity performance test for the elderly), the Jebsen-Taylor Hand Function Test, The Disabilities of the Arm, Shoulder and Hand (DASH) Questionnaire, and Manual Ability Measure-36 (MAM-36). Such assessments can be performed before and after administration of an aminopyridine to a patient in accordance with the methods disclosed herein. For example, a function of a patient who has cerebral palsy can be assessed before administering an aminopyridine and/or after administering an aminopyridine, e.g., at or after 1, 2, 3, 4, 5, 6, 7, 8, 9, 10 days; 1, 2, 3, 4, 5, 6, 7, 8 weeks; 1, 2, 3, 4, 5, 6, 7, 8, 9, 10 months; or 1, 2, 3, 4, 5 years since the commencement of treatment in accordance with the methods described herein.

In a specific embodiment, a therapeutic outcome in a CP-related sensorimotor impairment is assayed for and detected at any one, two, three, four, five or more, or each, of the following time points, and/or at a time point later than any one of the following time points: 1, 2, 3, 4, 5, 6, 7, 8, 9, 10, 11, 12, 13, 14, 15, 16, 17, 18, 19, 20, 21, days; 1, 2, 3, 4, 5, 6, 7, 8, 9, 10, 11, 12, 13, 14, 15, 16, 17, 18, 19, 20, 21, 22, 23 or 24 weeks; 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 36, 42, 48, 54, 60, and 66 months; .5, 1, 1.5, 2, 2.5, 3, 3.5, 4, 4.5, 5, 5.5, 6 and 6.5 years post-commencement of treatment with an aminopyridine or a pharmaceutically acceptable salt thereof.

In one embodiment, the sign or symptom of CP treated in accordance with the methods described herein is not cognition. In one embodiment, the sign or symptom of CP treated in accordance with the methods described herein is not an impairment in a neuro-cognitive or a neuro-psychiatric parameter, function or condition (e.g., not depression, not altered libido, not euphoria, or not fatigue).

### 5.2 Patient identification or selection

In a preferred embodiment of the invention, a patient is provided, selected, identified or diagnosed who has some form of cerebral palsy.

In one embodiment, a patient is provided, selected, identified or diagnosed who has some form of cerebral palsy and who has a sign or symptom of CP or an impairment or alteration consequent to CP. The sign, symptom impairment or alteration is one or more of: motor dysfunction, language or speech dysfunction, walking or ambulation dysfunction (e.g., decrease in walking speed), dysfunctional muscle strength, dysfunctional muscle tone, abnormal reflexes, abnormal coordination, spasticity, involuntary movements, abnormal gait, abnormal balance, decreased muscle mass, impaired skeletal development, impaired muscle development or impaired mental status. In one embodiment, the sign, symptom impairment or alteration is one or more of: impairment in hand strength, impairment in manual dexterity, impairment in walking speed, and impairment in gait.

In a particular embodiment, a patient is provided, selected, identified or diagnosed who has some form of cerebral palsy and who has a sign, symptom, impairment or alteration consequent to CP which is: motor dysfunction, language or speech dysfunction, walking or ambulation dysfunction, dysfunctional muscle strength, dysfunctional muscle tone, abnormal reflexes, abnormal coordination, spasticity, involuntary movements, abnormal gait, abnormal balance, decreased muscle mass, impaired skeletal development, or impaired muscle development and with a proviso that the patient does not have impaired mental status.

In a particular embodiment, patients are provided, selected, identified or diagnosed who have some form of cerebral palsy and who have an sign, symptom, impairment or alteration consequent to CP which is: motor dysfunction, language or speech dysfunction, walking or ambulation dysfunction, dysfunctional muscle strength, dysfunctional muscle tone, abnormal reflexes, abnormal coordination, spasticity, involuntary movements, abnormal gait, abnormal balance, decreased muscle mass, impaired skeletal development, impaired muscle development, or impaired mental status; with the proviso that the patient does not have at least one of the signs, symptoms, impairments or alterations consequent to CP in the following group other than the one selected for treatment and improvement: motor dysfunction, language or speech dysfunction, walking or ambulation dysfunction, dysfunctional muscle strength, dysfunctional muscle tone, abnormal reflexes, abnormal coordination, spasticity, involuntary movements, abnormal gait, abnormal balance, decreased muscle mass, impaired skeletal development, impaired muscle development, or impaired mental status.

As used herein "mental status" can include a disease-related condition such as depression, libido, euphoria, a decrease in mentation, fatigue or cognitive impairment. These patients are then administered a composition and a dosing regimen in accordance with the present invention. In specific embodiments, the sign, symptom, impairment or alteration consequent to CP treated in accordance with the methods described herein is not an impairment in mental status, for example, not one or more of depression, libido, euphoria, a decrease in mentation, fatigue and cognitive impairment.

In certain embodiments, a patient is provided, selected, identified or diagnosed who has some form of cerebral palsy and who has a sign, symptom, impairment or alteration consequent to CP which is a sensorimotor impairment, such as ataxia, global body control impairments, coordination or balance impairments, impairment in body sense, endurance impairment, impairment in hand function, fine hand coordination loss or impairment, hyperreflexia, impairment in grip strength, muscle weakness, muscle tone impairment, range of motion impairment, spasticity, strength impairment/weakness, tremor, impairment in limb function, upper extremity function impairment, lower extremity function impairment, impairment in lower extremity muscle strength, walking impairments (e.g., decreased walking speed), speech impairments (e.g., dysarthria, such as spastic, athetoid or ataxic dysarthria), impairment in jaw function, impairment in chewing, and impairment in jaw articulation.

The patients or subjects that are treated by the methods described herein include, but are not limited to, humans and non-human vertebrates such as wild, domestic and farm animals. In certain embodiments, the patient treated in accordance with the invention is a mammal, e.g., a human, a cow, a dog, a cat, a goat, a horse, a sheep, or a pig. In a preferred embodiment, the patient to whom a potassium channel blocker as described herein is administered is a human, *e.g.,* a child or adult. Preferably the patient is a human adult (*e.g.,* at least 14, at least 18 or at least 21 years old). In another embodiment, the patient is a child (pediatric treatment) (*e.g.,* under 1, under 3, under 5, under 10, under 14, or under 16 years of age). In another embodiment, the patient is 18 to 55 years old. In another embodiment, the patient is greater than 55 years old.

In certain embodiments, the patient treated in accordance with the methods described herein has (e.g., is diagnosed with) any type of CP, e.g., spastic, ataxic, athetoid/dyskinetic, and/or hypotonic CP. In one embodiment, the patient is diagnosed with spastic CP. In another embodiment, the patient is diagnosed with ataxic CP. In yet another embodiment, the patient is diagnosed with athetoid or dyskinetic CP. In yet another embodiment, the patient is diagnosed with hypotonic CP. In some embodiments, the patient has (e.g., is diagnosed with) spastic hemiplegia, spastic diplegia, spastic monoplegia, spastic triplegia, or spastic quadriplegia.

In some embodiments, the patients treated in accordance with the methods provided herein do not have a clinical history of seizures and/or epilepsy. In specific embodiments, the patients treated in accordance with the methods provided herein do not have a clinical history of seizures and/or epilepsy , with the exception of febrile seizures. For example, the patients have not experienced seizures and/or epilepsy in their lifetime, or have not experienced seizures and/or epilepsy 1, 2, 3, 4 or 5 years, or more than 5 years, prior to administration of an aminopyridine or a pharmaceutically acceptable salt thereof. In yet other embodiments, the patients treated in accordance with the methods provided herein have a clinical history of seizures and/or epilepsy.

### 5.3 Dosing Regimens

In some embodiments, an aminopyridine or a pharmaceutically acceptable salt thereof is administered in a sustained release composition. In other embodiments, an aminopyridine or a pharmaceutically acceptable salt thereof is administered in an immediate release composition. In certain embodiments, the method described herein with the invention comprises administering an aminopyridine or a pharmaceutically acceptable salt thereof once daily, twice daily or thrice daily. In a specific embodiment, an aminopyridine (e.g., 4-AP), or a pharmaceutically acceptable salt thereof, is in a sustained release composition, and is administered once or twice daily, for example, orally. In a specific embodiment, the daily dose of 4-AP is once a day, or is given as two, three, or four equally divided subdoses. In another specific embodiment, an aminopyridine (e.g., 4-AP), or a pharmaceutically acceptable salt thereof, is in an immediate release composition, and is administered one, two, three times or more than three times daily, for example, orally. In one embodiment, a single dose of an aminopyridine or a pharmaceutically acceptable salt thereof (e.g., in an immediate release composition or in a sustained release composition) is administered to a patient.

In a specific embodiment, an aminopyridine or a pharmaceutically acceptable salt thereof is administered to the patient orally, in a sustained release composition b.i.d. (i.e., twice daily). In certain embodiments, twice daily administration comprises administration of a compound every 12 hours.

In some embodiments, an aminopyridine in a sustained release composition provides a Tₘₐₓ of about 2 hours to about 6 hours in a human.

In a specific embodiment, an aminopyridine or a pharmaceutically acceptable salt thereof is administered to the patient orally, in a sustained release composition once daily.

In certain embodiments, an aminopyridine or a pharmaceutically acceptable salt thereof is administered in an amount ranging from about 4 mg to about 20 mg (e.g., about 4, 5, 6, 7, 7.5, 8, 9, 10, 11, 12, 12.5, 13, 14, 15, 16, 17, 17.5 or 20 mg) once or twice daily, preferably in a sustained release composition. In some embodiments, an aminopyridine or a pharmaceutically acceptable salt thereof is administered in an amount ranging from about 5 mg to 15 mg, 5 mg to 10 mg, 5 mg to 7.5 mg, 7.5 mg to 12.5 mg, or 7.5 mg to 10 mg twice daily, or about 10 mg to 30 mg, 10 mg to 20 mg, 10 mg to 15 mg, 15 mg to 30 mg, 15 mg to 40 mg, or 20 mg to 40 mg once daily, preferably in a sustained release composition. In one embodiment, an aminopyridine or a pharmaceutically acceptable salt thereof is administered at a dose of 5 mg once daily or twice daily, preferably in a sustained release composition. In another embodiment, an aminopyridine or a pharmaceutically acceptable salt thereof is administered at a dose of 10 mg once daily or twice daily, preferably in a sustained release composition. In some of these embodiments, the aminopyridine is 4-aminopyridine. In specific embodiments, an aminopyridine or a pharmaceutically acceptable salt thereof is administered in an amount ranging from about 5 mg to 20 mg, 8 mg to 15 mg, 7.5 mg to 12.5 mg, or 10 mg to 15 mg once daily (e.g., in a sustained release composition).

In one embodiment, lower doses, e.g., in the range of 1 mg to 10 mg once daily, or 4 mg to 10 mg once daily, or 1 mg to 5 mg twice daily, are used for pediatric treatment, preferably in a sustained release composition. In one embodiment, doses are for adult treatment and are in the range of 10 mg to 40 mg once daily, or 5 mg to 20 mg twice daily, preferably in a sustained release composition.

In some embodiments, the patient with CP is treated in accordance with the methods described herein for a period of time that is, e.g., for at least 1 week, at least 2 weeks, at least 3 weeks, at least 4 weeks, at least 5 weeks, at least 6 weeks, at least 1 month, at least 2 months, at least 3 months, at least 4 months, at least 5 months, at least 6 months, at least 7 months, at least 8 months, at least 9 months, at least 10 months, at least 11 months, at least 1 year, at least 2 years, at least 3 years, at least 4 years, at least 5 years, at least 10 years, or more than 5 or 10 years. In certain embodiments, the treatment regimen (a particular dose and frequency of administration, which can be selected from any described herein) is stable over a period of time, e.g., for at least 1 week, at least 2 weeks, at least 3 weeks, at least 1 month, at least 2 months, at least 3 months, at least 4 months, at least 6 months, or at least 1 year.

In their most direct embodiment, neuromuscular connections can be straightforward: a cortical motor neuron, a spinal motor neuron and a muscle. Mental status, and the various aspects thereof, are a more diffuse and integrated phenomena. Thus, it is disclosed herein that the need to tightly mange the range of steady state values, in the plasma, inside the blood:brain barrier or in the CSF are particularly important. The requisite control can be achieved by use of 4-aminopyridine-SR on a bid or every 12 hour dosing protocol.

In one embodiment, a patient meeting the conditions set forth herein is provided with 4-aminopyridine-SR. In one embodiment, the patient is instructed to take the drug twice daily. In one embodiment, the patient is instructed to take 4-aminopyridine-SR in a dose selected from 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, or 25 mg bid. In one embodiment, one of the daily doses of 4-aminopyridine-SR is different from the other dose, and in a particular embodiment a morning dose is higher than the evening dose. In a preferred embodiment, at least one of the twice daily doses of 4-aminopyridine-SR is 10 mg.

In another embodiment, the patient is instructed to take 4-aminopyridine-SR once daily. In one embodiment, the patient is instructed to take 4-aminopyridine-SR in a dose selected from 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, or 40 mg once daily. In a preferred embodiment, the once daily dose of 4-aminopyridine-SR is 10 mg.

In specific embodiments, a patient with CP is treated by administering any of the doses and dosage regimens described in this application.

In one dosing embodiment, a sufficient amount of an aminopyridine, such as 4-aminopyridine, is provided such that it elicits the steady state levels that are within the range obtained by use of 4-aminopyridine-SR; in one embodiment these steady state values are a maximum concentration at steady state (Cₘₐₓₛₛ) and minimum concentration at steady state (Cₘᵢₙₛₛ). The steady state values can be plasma levels, levels on the brain side of the blood:brain barrier, or levels in the CSF. Preferably, these are plasma levels.

In another embodiment a sufficient amount of aminopyridine, such as 4-aminopyridine, is provided that it elicits the steady state levels that differ not more than about 15, 14, 13, 12, 11, 10, 9, 8, 7, 6, 5, 4, 3, 2, or 1% from the average steady state level (Cₐᵥₛₛ) obtained by use of 4-aminopyridine-SR. The steady state values can be plasma levels, levels on the brain side of the blood:brain barrier, or levels in the CSF. Preferably, these are plasma levels.

4-aminopyridine is a potassium (K+) channel blocker that was and continues to be evaluated clinically as a treatment for improving neurological and muscular function in patients with Multiple Sclerosis (MS). Dalfampridine is the United States Adopted Name (USAN) for the chemical 4-aminopyridine (4-AP), which has a molecular formula of C₅H₆N₂ and molecular weight of 94.1. The terms "dalfampridine", "4 aminopyridine" and "4-AP" will be used herein to refer to the active drug substance. 4-aminopyridine has been formulated as a sustained-release (SR) matrix tablet in various strengths from 5 to 40 mg.

4-aminopyridine-SR, in a strength of 10 mg, is provided as oval-shaped, white to off-white, sustained-release matrix tablets under the US tradename Ampyra®, Acorda Therapeutics, Hawthorne, NY. The following excipients are included in each Ampyra tablet: hydroxypropyl methylcellulose, USP; microcrystalline cellulose, USP; colloidal silicon dioxide, NF; magnesium stearate, USP; and Opadry White. Ampyra tablets, 10 mg, are packaged in HDPE bottles with child-resistant caps. Each bottle contains a desiccant.

Pharmacologically, the K+ channel blocking properties of 4-aminopyridine and its effects on action potential conduction in demyelinated nerve fiber preparations have been extensively characterized. At low concentrations that are relevant to clinical experience, in the range of 0.2 to 2 µM (18 to 180 ng/mL), 4-AP is able to block certain voltage-dependent K+ channels in neurons. This characteristic appears to explain the ability of the drug to restore conduction of action potentials in some critically demyelinated nerve fibers, though the molecular characteristics of the potassium channels in demyelinated axons that show such high sensitivity have not yet been elucidated. At higher (millimolar) concentrations, 4-aminopyridine affects other types of K+ channels in both neural and non-neural tissues. Blockade of repolarizing K+ currents can increase synaptic transmission throughout the nervous system by increasing the duration of the pre-synaptic action potential. A range of neurological effects consistent with increased excitability of presynaptic nerve terminals occurs with clinically relevant doses of 4-aminopyridine.

### 5.4 Pharmaceutical compositions

The invention also provides pharmaceutical compositions comprising a potassium channel blocker (such as an aminopyridine or a pharmaceutically acceptable salt thereof) as described herein. Such pharmaceutical compositions comprise a therapeutically effective amount of a potassium channel blocker and a pharmaceutically acceptable carrier. In one embodiment, the pharmaceutical composition is suitable for oral administration and can be, for example, a pill, tablet or capsule. Pharmaceutical compositions can be as described, for example, in U.S. Patent Application Publication No. 2005/0276851, published December 15, 2005 and U.S. Patent Application Publication No. 2005/0228030, published October 13, 2005, the contents of each of which are incorporated by reference herein in their entireties. A pharmaceutical composition can be, for example, an immediate release composition, a controlled release composition, or a sustained release composition. In one embodiment, the pharmaceutical composition comprises a sustained release composition of 4-aminopyridine. The pharmaceutical compositions of the invention are administered to a patient for any of the uses described herein.

The potassium channel blocker (such as an aminopyridine or a pharmaceutically acceptable salt thereof) is preferably administered to a patient orally or parenterally in the conventional form of preparations, such as capsules, microcapsules, tablets, granules, powder, troches, pills, suppositories, injections, suspensions, or syrups. Suitable formulations can be prepared by methods commonly employed using conventional, organic or inorganic additives, such as one or more of: an excipient (e.g., sucrose, starch, mannitol, sorbitol, lactose, glucose, cellulose, talc, calcium phosphate or calcium carbonate), a binder (e.g., cellulose, methylcellulose, hydroxymethylcellulose, polypropylpyrrolidone, polyvinylpyrrolidone, gelatin, gum arabic, polyethyleneglycol, sucrose or starch), a disintegrator (e.g., starch, carboxymethylcellulose, hydroxypropylstarch, low substituted hydroxypropylcellulose, sodium bicarbonate, calcium phosphate or calcium citrate), a lubricant (e.g., magnesium stearate, light anhydrous silicic acid, talc or sodium lauryl sulfate), a flavoring agent (e.g., citric acid, menthol, glycine or orange powder), a preservative (e.g., sodium benzoate, sodium bisulfite, methylparaben or propylparaben), a stabilizer (e.g., citric acid, sodium citrate or acetic acid), a suspending agent (e.g., methylcellulose, polyvinyl pyrroliclone or aluminum stearate), a dispersing agent (e.g., hydroxypropylmethylcellulose), a diluent (e.g., water), and base wax (e.g., cocoa butter, white petrolatum or polyethylene glycol). In some embodiments, suitable formulations of an aminopyridine or a pharmaceutically acceptable salt thereof can be prepared using one, two, three or more, or all, of the following additives: colloidal silicon dioxide, hydroxypropyl methylcellulose, magnesium stearate, microcrystalline cellulose, polyethylene glycol, and titanium dioxide.

The amount of a potassium channel blocker (such as an aminopyridine or a pharmaceutically acceptable salt thereof) that is present in the pharmaceutical composition is preferably an amount that will exercise the desired effect.

A potassium channel blocker (such as an aminopyridine or a pharmaceutically acceptable salt thereof) can be administered orally. In some of the embodiments wherein an aminopyridine or a pharmaceutically acceptable salt thereof is administered orally, the composition is formulated in a form of a tablet, a pill or a capsule. The potassium channel blocker (such as an aminopyridine or a pharmaceutically acceptable salt thereof) can also be administered intradermally, intramuscularly, intraperitoneally, percutaneously, intravenously, subcutaneously, intranasally, epidurally, sublingually, intracerebrally, intravaginally, transdermally, rectally, by inhalation, or topically to the ears, nose, eyes, or skin. In one embodiment, an aminopyridine or a pharmaceutically acceptable salt thereof is administered to the patient intravenously. The mode of administration is left to the discretion of the health-care practitioner.

A pharmaceutically acceptable carrier or vehicle can comprise an excipient, diluent, or a mixture thereof.

The compositions can be in the form of tablets, chewable tablets, capsules, solutions, parenteral solutions, troches, suppositories and suspensions and the like. Compositions can be formulated to contain a daily dose, or a convenient fraction of a daily dose, in a dosage unit, which may be, e.g., a single tablet or capsule or convenient volume of a liquid.

Capsules can be prepared by any known method, such as mixing a potassium channel blocker with a suitable carrier or diluent and filling the proper amount of the mixture in capsules. Carriers and diluents include, but are not limited to, inert powdered substances such as starch of many different kinds, powdered cellulose, especially crystalline and microcrystalline cellulose, sugars such as fructose, mannitol and sucrose, grain flours and similar edible powders.

Tablets can be prepared by known methods such as direct compression, by wet granulation, or by dry granulation. Their formulations usually incorporate diluents, binders, lubricants and disintegrators as well as the compound. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders.

In a specific embodiment, one can combine an aminopyridine or a pharmaceutically acceptable salt thereof with one or more other agents and/or physical or occupational therapies for the treatment of CP (or any sign or symptom thereof), for example, for the treatment of a sensorimotor impairment due to CP. In some embodiments, an aminopyridine or a pharmaceutically acceptable salt thereof is administered to a patient concomitantly or sequentially with one or more additional drug or therapy. For example, an aminopyridine or a pharmaceutically acceptable salt thereof can be administered to a patient at the same time, before, or after administration of a drug that controls seizures, a drug that alleviates pain, a drug that relaxes muscle spasms (e.g. benzodiazepienes, baclofen, tizanadine and intrathecal phenol/baclofen); a hyperbaric oxygen; or Botulinum toxin A (Botox(R)). In particular embodiments, the combination of an aminopyridine or a pharmaceutically acceptable salt thereof and one, two or more additional drug(s) is a fixed dose combination. For example, an aminopyridine or a pharmaceutically acceptable salt thereof and one or more additional drug(s) can be formulated in one composition, such as a pill, a tablet or a capsule. In other embodiments, an aminopyridine or a pharmaceutically acceptable salt thereof is administered to a patient concomitantly (e.g., at the same time, before or after) with physical therapy, occupational therapy, speech therapy, or surgery (e.g., to correct anatomical abnormalities or release tight muscles). In some embodiments, an aminopyridine or a pharmaceutically acceptable salt thereof is administered to a patient with CP who uses a brace, standing frame or other orthotic device such as a rolling walker, or communication aid such as a computer with attached voice synthesizer. In a specific embodiment, the aminopyridine (or salt thereof) and other drug or therapy is administered at the same doctor's visit, or within 1, 2, 3, 4, 5, 6, or 12 hours, or within 1, 2, 3, 4, 5, 6, or 7 days, of each other.

In yet other embodiments, an aminopyridine or a pharmaceutically acceptable salt thereof is administered to a patient without an additional drug or therapy, or without one or more of additional treatments (such as those described above). In certain embodiments, treatment in accordance with the invention (either with or without use of an additional drug, therapy or surgery), is more effective than treatment with another drug or therapy known to be used for the treatment of CP.

### 5.5 Aminopyridine promotes functional recovery after an ischemic event: Effects of Oral Administration of 4-AP. Functional Recovery Following MCA occlusion (MCAO) in Rats

4-AP was evaluated for its ability to promote functional sensorimotor improvement following ischemic stroke in rats with stable motor deficits at times remote from their ischemic events. The animal model mimics the conditions in human ischemic stroke and is produced by middle cerebral artery occlusion (MCAO), which results in extensive infarction in cerebral cortex and striatum, including the cortical spinal tract (white matter). In particular, the MCAO model in Sprague Dawley rats that was utilized in the experiments presented below mimics the conditions in human ischemic stroke.

### Experimental Design

In this experiment, Sprague Dawley rats were anesthesized, subjected to a surgery resulting in middle cerebral artery occlusion (MCAO), treated with and without 4-aminopyridine and behavioral assessments were performed as described below.

Animals: 45 male Sprague Dawley Rats, 300-400 g (obtained from Charles River Laboratories, which arrived 7-10 days before surgery at 250-275 g) were used.

Nomenclature: The nomenclature for the days of the study is as follows: Day 0 is the day of the MCAO, and the days following are numbered consecutively (Day 1, Day 2, Day 3, etc.); Day -1 represents the day prior to the MCAO.

Grouping details: The amount of time needed for some procedures in this study necessitated breaking up the 3 treatment groups (as listed above), into 8 working groups (as written in the schedule). Six animals received stroke surgery per day. If an animal died during the 8-day surgical period of the study, it was replaced by a spare. If not, the animal was not replaced. Most animal deaths (<5% overall) occurred in the immediate post-op to 7 day period.

Anesthesia: 1-3% isoflurane in N₂O: O₂ (2:1). Anesthesia was induced in an induction chamber with 2-3% isoflurane in N2O:O2 (2:1), and maintained with 1-1.5% isoflurane via face mask. Adequate depth of anesthesia was assessed by lack of withdrawal to hindlimb pinch and loss of eyeblink reflex. Once anesthetized, animals received cefazolin sodium (40 mg/kg, i.p.) and buprenorphine (0.1 mg/kg, s.c.). Cefazolin was used as a prophylactic antibiotic for this procedure (because it ensures a negligible infection rate). A veterinary ophthalmic ointment, Lacrilube, was applied to the eyes.

Surgical Procedure: A small focal stroke (infarct) was made on the right side of the surface of the brain (cerebral cortex) by middle cerebral artery occlusion (MCAO). The right side of the head was shaved with electric clippers (patch of approx 3 X 5 cm between eye and ear). The region was carefully cleaned with septisol. Using aseptic technique, an incision was made midway between the eye and eardrum canal. The temporalis muscle was isolated, bisected, and reflected. A small window of bone was removed via drill and rongeurs (subtemporal craniectomy) to expose the middle cerebral artery (MCA). Care was taken not to remove the zygomatic arch or to transect the facial nerve that would impair the ability of the animal to chew after surgery. Using a dissecting microscope, the dura was incised, and the MCA was electrocoagulated from just proximal to the olfactory tract to the inferior cerebral vein (taking care not to rupture this vein), using microbipolar electrocauterization. The MCA was then transected. The temporalis muscle was then repositioned, and the incision was closed subcutaneously with sutures. The skin incision was closed with surgical staples (2-3 required). Throughout the procedure, body temperature was maintained at 37.0° ± 1° C, using a self-regulating heating pad connected to a rectal thermometer.

Post-Operative Monitoring: Following surgery, animals remained on a heating pad until they awakened from anesthesia. They were then returned to clean home cages. They were observed frequently on the day of MCAO surgery (Day 0) and at least once daily thereafter.

Handling, surgery, and injections timetable: The animals were housed 2-3 per cage before and after surgery, unless severe aggression was displayed, or death of cage mate(s). The animals were handled for 7 days before the surgery. Cefazolin Sodium i.p. (40 mg/kg) was administered right before surgery. Buprenorphine s.c. (0.1mg/kg) was administered right before surgery.

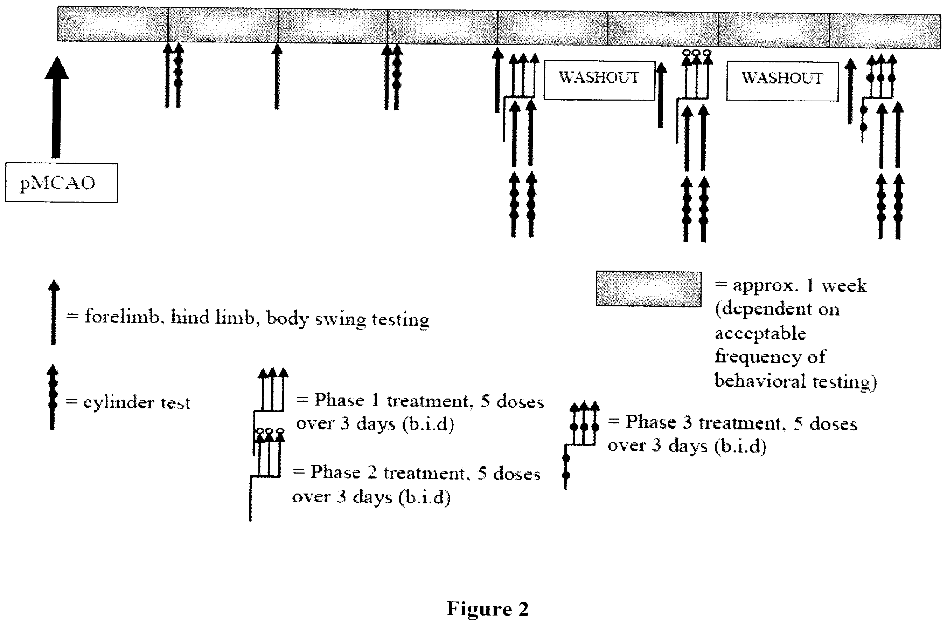

Dosing: Rats were treated in accordance with the dosing schedule shown in Tables 1A, 1B, and Figure 2, with each Phase being a two week period of time. A solution of 4-AP was used in this experiment. Dosing was started at 4 weeks after ischemic event.

**Table 1A:**

| **Treatment ID** | **Treatment (doses TBD)** |
|---|---|
| V | Vehicle (water) |
| L | Low 4-AP (0.63 mg/kg, b.i.d, p.o) |
| H | High 4-AP (2.0 mg/kg, b.i.d, p.o) |

**Table 1B:**

| **Group (n=15)** | **Phase 1 treatment** | **Phase 2 treatment** | **Phase 3 treatment** |
|---|---|---|---|
| 1 | H | L | V |
| 2 | L | V | H |
| 3 | V | H | L |

Treatment groups: Dosing was started 4 weeks after pMCAO surgery. During these 4 weeks, weekly behavioral assessments, as defined below, were performed. Two dose levels of 4-AP plus vehicle control were assessed, with treatment starting at 4 weeks after ischemic event. All dosing was via gastric gavage, volume not to exceed 2 mL/kg. Animals were given the first dose and behavioral assessments were performed starting 60 minutes after dosing. Animals then received the second dose that day at the appropriate time and b.i.d (preferably every 12 hours) thereafter for 2 more days (3 total days of dosing, 5 total doses). One hour after the 5^{th} dose, animals were subjected to behavioral assessments as defined below. Following the final behavioral assessment, drug was withdrawn for 10 or 11 days, behavior was re-assessed, and then animals were re-challenged with a cross-over treatment (Phase II of Table 1B) as described in Tables 1A, 1B, followed by the same behavioral testing and dosing regimen. This cross-over was repeated one more time as well (Phase III of Table 1B) (see Figure 2).

Behavioral test details: Behavioral evaluations were done by investigators blinded to treatment assignment. As described above, behavioral assessments were timed exactly with dosing times. Animals were given the first dose, behavioral assessments were performed starting 60 minutes later, and blood was collected 90 minutes after dosing. Animals then received the second dose that day at the appropriate time and b.i.d (preferably every 12 hours) thereafter for 2 more days (3 total days of dosing, 5 total doses). One hour after the 5^{th} dose, animals were subjected to behavioral testing. Following the final behavioral assessment, drug was withdrawn for 10 or 11 days, behavior was re-assessed, and then animals were re-challenged with a cross-over treatment as described in Tables 1A, 1B, and Figure 2, followed by the same behavioral testing and dosing regimen.

Limb Placing: Evaluated at Day -1 (pre-operation), Day 1, Day 7, Day 14, Day 21, Day 28, Day 30, Day 32, Day 42, Day 44, Day 46, Day 56, Day 58, Day 60. The limb placing tests were divided into forelimb and hindlimb tests. For the forelimb-placing test, the examiner held the rat close to a tabletop and scored the rat's ability to place the forelimb on the tabletop in response to whisker, visual, tactile, or proprioceptive stimulation. Similarly, for the hindlimb placing test, the examiner assessed the rat's ability to place the hindlimb on the tabletop in response to tactile and proprioceptive stimulation. Separate sub -scores were obtained for each mode of sensory input and added to give total scores (for the forelimb placing test: 0 = normal, 12 = maximally impaired; for the hindlimb placing test: 0 = normal; 6 = maximally impaired). Scores were given in half-point increments (see below). Typically, there is a slow and steady recovery of limb placing behavior during the first month after stroke.
Forelimb placing test (0-12):
   whisker placing (0-2);
   visual placing (forward (0-2), sideways (0-2))
   tactile placing (dorsal (0-2), lateral (0-2))
   proprioceptive placing (0-2).
Hindlimb placing test (0-6):
   tactile placing (dorsal (0-2), lateral (0-2))
   proprioceptive placing (0-2).
For each subtest, animals are scored as followed:
   0.0 = immediate response
   0.5 = response within 2 seconds
   1.0 = response of 2-3 seconds
   1.5 = response of >3 seconds
   2.0 = no response

Body Swing: Evaluated at Day -1 (pre-operation), Day 1, Day 7, Day 14, Day 21, Day 28, Day 30, Day 32, Day 42, Day 44, Day 46, Day 56, Day 58, Day 60. For this test, the rat was held approximately one (1) inch from the base of its tail. It was then elevated to an inch above a surface of a table. The rat was held in the vertical axis, defined as no more than 10° to either the left or the right side. A swing was recorded whenever the rat moved its head out of the vertical axis to either side. Before attempting another swing, the rat had to return to the vertical position for the next swing to be counted. Thirty (30) total swings were counted. A normal rat typically has an equal number of swings to either side. Following focal ischemia, the rat tends to swing to the contralateral side (left side in this case). Body swing scores were expressed as a percentage of rightward over total swings. There is a spontaneous partial recovery of body swing scores (toward 50%) during the first month after stroke.

Cylinder Test: Evaluation was performed at Day -1 (pre-operation), Day 7, Day 21, Day 30, Day 32, Day 44, Day 46, Day 58, Day 60. This test assessed limb use asymmetry. Rats were placed in a transparent cylinder (20 cm in diameter and 30 cm in height) for 3-6 minutes. A mirror was placed behind the cylinder to determine forelimb movements when animal was turned away from the camera. The extent of fore limb-use asymmetry displayed by the animals was determined by counting the number of times the left or right forelimbs contact the wall during a full rear. Simultaneous use of both left and right forelimbs while contacting the wall during a full rear was also scored. A total number of 20 forelimb placings were counted during a trial. Data was expressed in terms of percent use of the non-impaired and or impaired forelimb relative to the total number of limb use observations for wall movements.

Regulatory Compliance: This study was conducted in a non-GLP environment, in an AAALAC accredited facility and in accordance with standard good scientific principles and practices.

Quality Assurance (QA): During the progress of the study, data collected was verified by a second scientist in the laboratory who was not involved in collection of that data set. This verification was documented within the raw data, and was maintained with the data package for this study. At the completion of the study, the entire data package (all raw data, measurements, notebooks and calculations) was verified and checked against the final report.

### Results

Table 2 shows the distribution of animals among the treatment groups. Tables 3A-3C show the Forelimb Placing Test Total Score for each of Groups I-III. Tables 4A-4C show Hindlimb Placing Test Total Score for each of Groups I-III. Tables 5A-5C show Body Swing Test Total Score for each of Groups I-III. Tables 6A-6C show the weight of animals at time points tested in each of Groups I-III. Tables 7A-7C show Cylinder Test Total Score (% of overall asymmetry) for each of Groups I-III. Tables 8A-8C show Cylinder Test Total Movement Score for each of Groups I-III.

MCAO resulted in substantial acute loss of sensorimotor function which recovered partially, approaching a plateau of stable deficits in all animals by the end of the 4-week pre-treatment period.

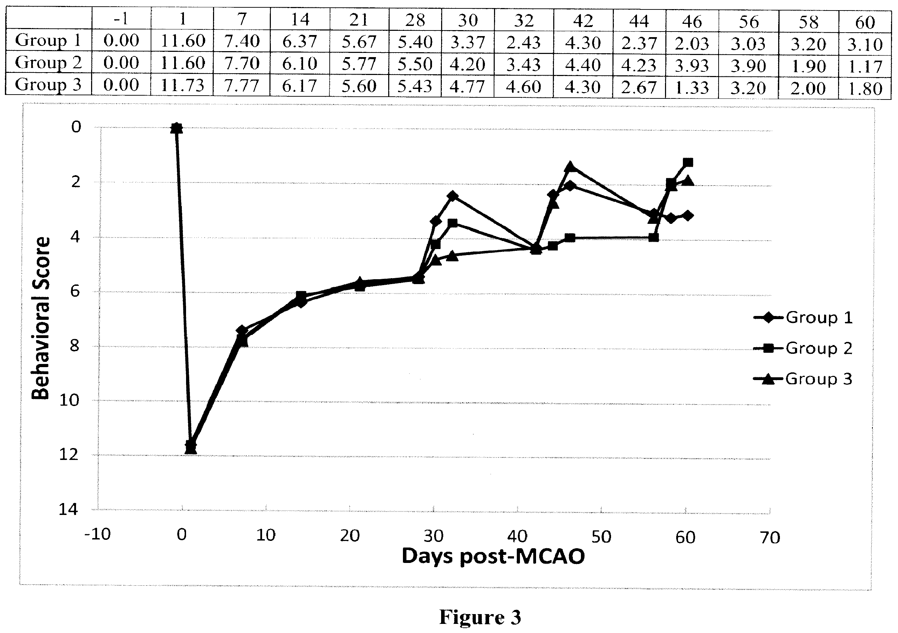

The forelimb placing test shows the effect of the treatment on forelimb function. Figure 3 indicates that the treatment with either low dose or high dose 4-aminopyridine, 4 weeks post ischemic brain injury, is effective to improve forelimb function in rats. Figure 3 also indicates that the effect is dose-responsive. This effect is also reversible as it diminishes upon withdrawal of the drug.

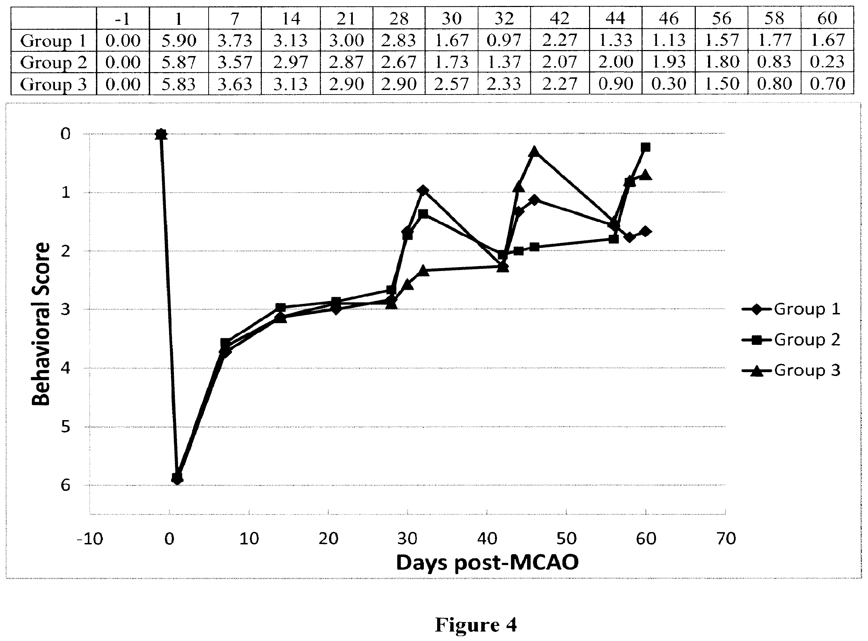

The hindlimb placing test shows the effect of the treatment on hindlimb function. Figure 4 indicates that the treatment with either low dose or high dose 4-aminopyridine, 4 weeks post ischemic brain injury, is effective to improve hindlimb function in rats. Figure 4 also indicates that the effect is reversible. Notably, the effect is dose responsive as the treatment with a higher dose results in an improved behavioral score, relative to the treatment with a lower dose or vehicle control.

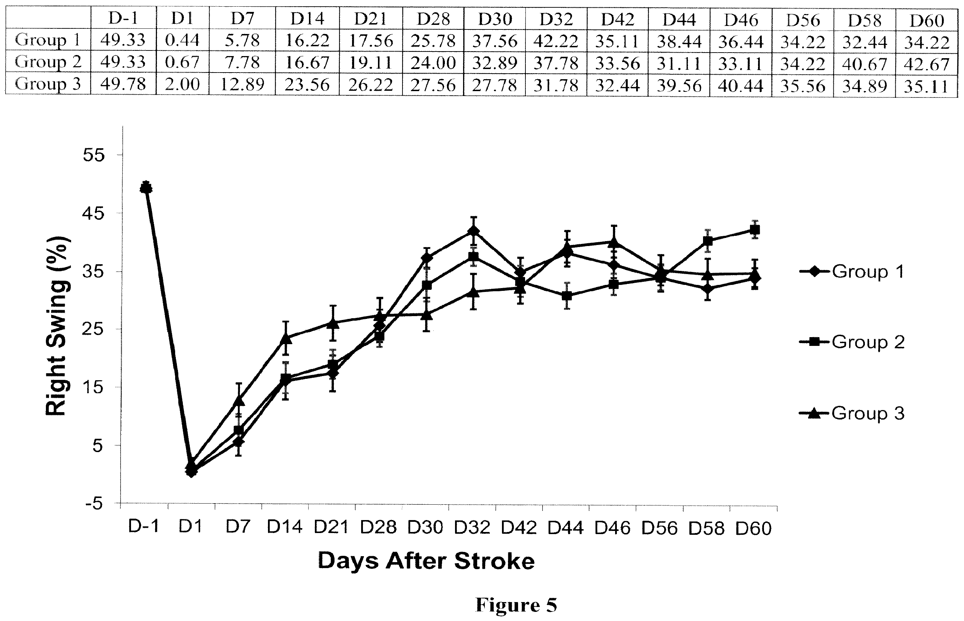

The body swing test shows the effect of the treatment on global body control. Figure 5 shows that the treatment with either low dose or high dose 4-aminopyridine is effective to improve the percentage of rightward over total swings in rats, and thus, effective to improve one of the symptoms of ischemic stroke. Thus, Figure 5 shows that 4-aminopyridine is effective to improve global body control in rats. Figure 5 also demonstrates that this effect is reversible and dose-dependent.

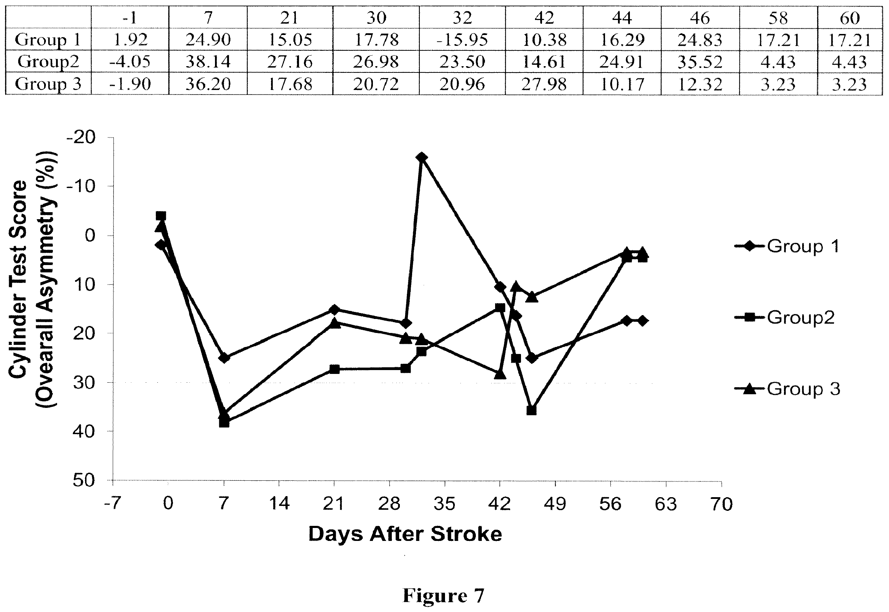

The cylinder test shows the effect of the treatment on aspects of the global body control such as body symmetry and coordination. Figure 7 shows that the treatment with 4-aminopyridine is effective to improve the asymmetry in limb usage resulting from the stroke by showing increase in percentage of use of the impaired forelimb relative to the total limb use in rats. Thus, Figure 7 shows that 4-aminopyridine is effective to improve body symmetry and coordination in rats. Figure 7 also demonstrates that this effect is reversible and dose-dependent.

The data show that during each separate treatment phase and overall, 4-aminopyridine treatment resulted in significant improvement in forelimb, hindlimb and body-swing function. Further, several crossover statistical models utilized by the inventors demonstrated that the high dose was significantly better (p<0.0001 for limb placing tests, and p<0.001 for body swing) than both the vehicle control and the low dose on a consistent basis. The low dose showed either a strong trend or reached significance for improvement compared with vehicle control. Additionally, scores during the second assessment within a dosing phase were significantly better than the first on-drug assessment.

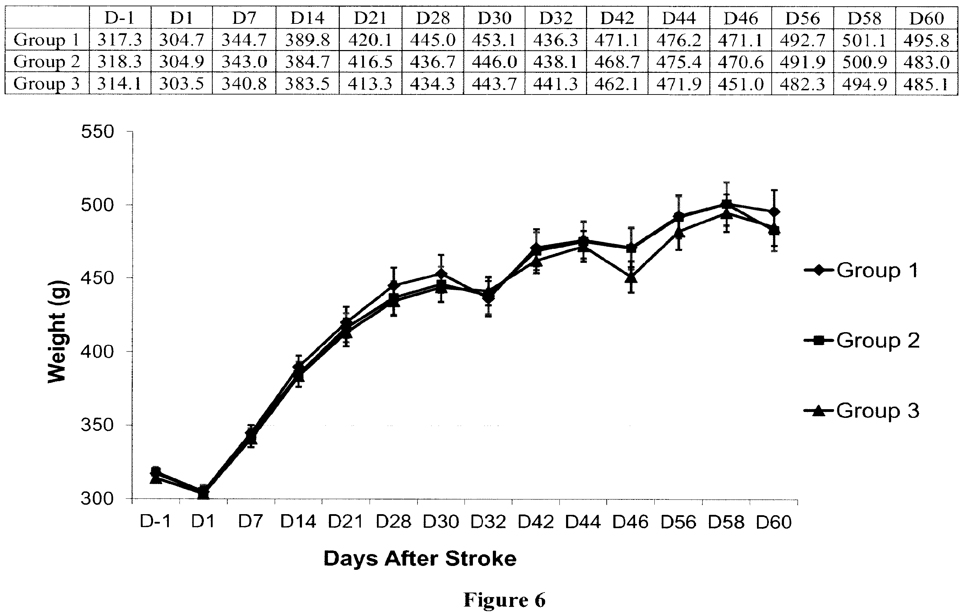

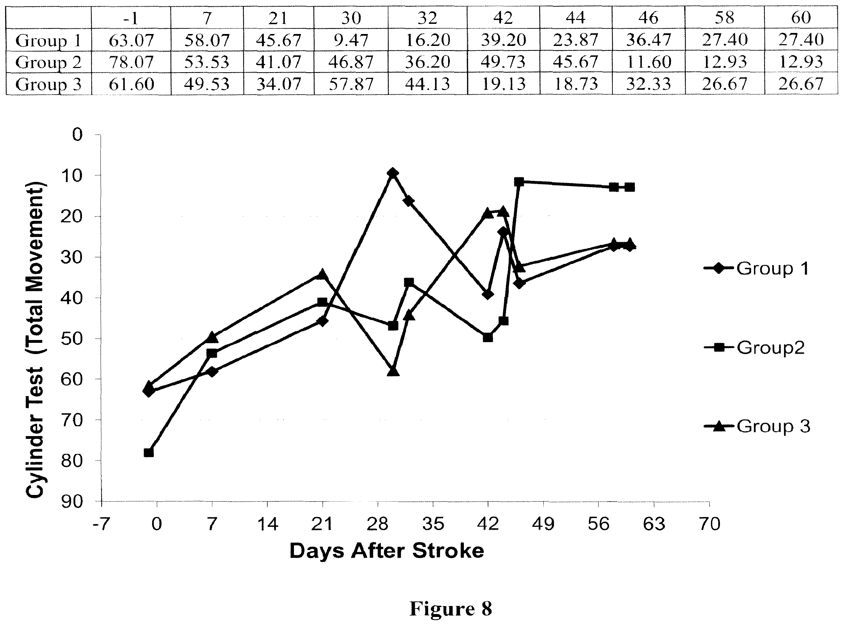

In addition, the results in Figures 3-8 show that continued treatment with 4-aminopyridine may yield further improvement in sensorimotor behavioral outcome. In particular, behavioral scores after administration of multiple doses of 4-aminopyridine are, on average, higher than behavioral scores after a single dose of 4-aminopyridine.

These results indicate that treatment with 4-aminopyridine is effective to improve sensorimotor functions in mammals suffering from ischemia-related impairment of such functions. These results also demonstrate improvements in ischemia-related sensorimotor impairments when the treatment is initiated during a chronic period, with stable motor deficits, following the stroke event. Based on this data it can be concluded that 4-aminopyridine significantly improves chronic sensorimotor deficits post-stroke.

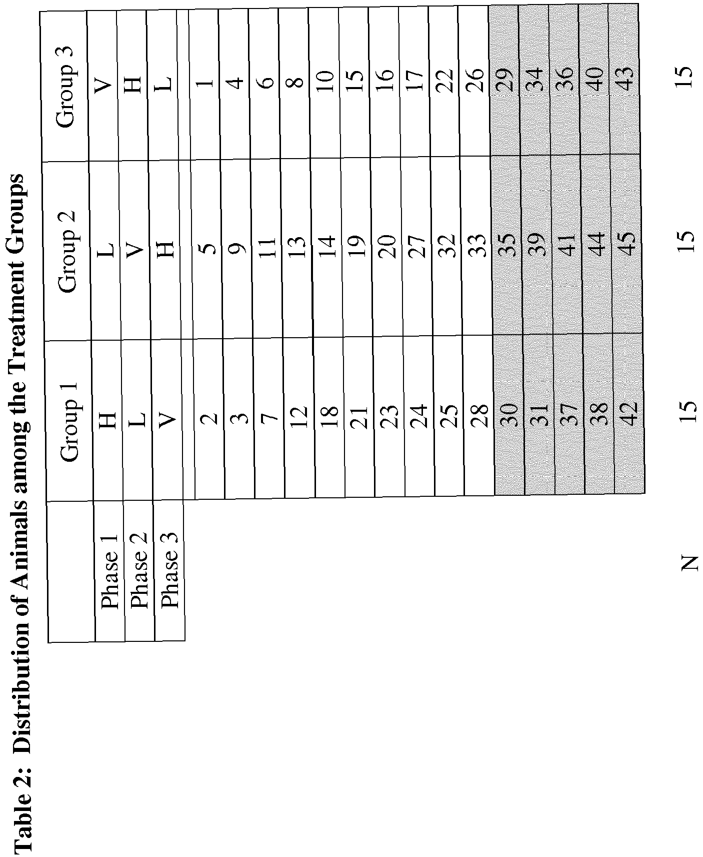

**Table 3A: Forelimb Placing Total Score Group I**

| **Group 1** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 2 | 0.00 | 12.00 | 9.00 | 8.50 | 7.00 | 6.50 | 4.50 | 4.00 | 6.00 | 4.00 | 3.00 | 3.50 | 4.50 | 6.00 |
| No. 3 | 0.00 | 11.50 | 9.00 | 7.50 | 6.50 | 6.00 | 4.50 | 3.00 | 5.00 | 3.00 | 2.50 | 2.50 | 2.50 | 2.50 |
| No. 7 | 0.00 | 11.00 | 7.00 | 6.00 | 6.00 | 6.00 | 3.50 | 3.50 | 4.50 | 0.00 | 0.00 | 2.00 | 3.00 | 3.00 |
| No. 12 | 0.00 | 11.50 | 6.00 | 4.50 | 4.50 | 4.00 | 1.50 | 0.50 | 1.50 | 0.50 | 0.00 | 1.50 | 1.50 | 1.50 |
| No. 18 | 0.00 | 12.00 | 7.00 | 6.50 | 5.50 | 4.50 | 3.00 | 2.00 | 4.00 | 1.00 | 1.00 | 2.50 | 2.00 | 2.00 |
| No. 21 | 0.00 | 12.00 | 9.50 | 7.00 | 6.50 | 6.00 | 4.50 | 3.00 | 5.00 | 3.50 | 3.50 | 4.00 | 4.00 | 3.00 |
| No. 23 | 0.00 | 11.50 | 6.00 | 5.00 | 5.00 | 5.00 | 3.50 | 2.00 | 2.50 | 1.50 | 1.50 | 1.50 | 1.50 | 1.50 |
| No. 24 | 0.00 | 11.50 | 7.50 | 7.00 | 6.50 | 6.00 | 4.00 | 3.50 | 4.50 | 4.00 | 3.00 | 3.00 | 3.50 | 3.50 |
| No. 25 | 0.00 | 11.50 | 7.00 | 6.00 | 6.00 | 6.00 | 5.50 | 2.00 | 6.00 | 4.00 | 1.50 | 5.50 | 5.50 | 5.50 |
| No. 28 | 0.00 | 11.50 | 8.00 | 7.00 | 6.00 | 6.00 | 4.50 | 3.00 | 5.00 | 3.00 | 3.50 | 5.00 | 5.50 | 4.00 |
| No. 30 | 0.00 | 12.00 | 7.50 | 6.50 | 6.00 | 6.00 | 3.00 | 3.00 | 5.50 | 4.00 | 4.00 | 4.50 | 4.00 | 4.00 |
| No. 31 | 0.00 | 11.50 | 7.00 | 6.50 | 6.00 | 6.00 | 3.50 | 2.50 | 5.00 | 2.00 | 2.50 | 3.50 | 3.50 | 3.00 |
| No. 37 | 0.00 | 12.00 | 6.50 | 6.00 | 6.00 | 6.00 | 3.50 | 3.50 | 6.00 | 3.50 | 3.00 | 5.00 | 5.50 | 5.50 |
| No. 38 | 0.00 | 10.50 | 7.00 | 5.00 | 3.50 | 3.50 | 0.50 | 0.50 | 2.00 | 1.50 | 1.50 | 1.50 | 1.50 | 1.50 |
| No. 42 | 0.00 | 12.00 | 7.00 | 6.50 | 4.00 | 3.50 | 1.00 | 0.50 | 2.00 | 0.00 | 0.00 | 0.00 | 0.00 | 0.00 |
| | | | | | | | | | | | | | | |
| N | 15 | 15 | 15 | 15 | 15 | 15 | 15 | 15 | 15 | 15 | 15 | 15 | 15 | 15 |
| Mean | 0.00 | 11.60 | 7.40 | 6.37 | 5.67 | 5.40 | 3.37 | 2.43 | 4.30 | 2.37 | 2.03 | 3.03 | 3.20 | 3.10 |
| Sem | 0.00 | 0.11 | 0.27 | 0.26 | 0.26 | 0.26 | 0.36 | 0.30 | 0.40 | 0.39 | 0.35 | 0.41 | 0.44 | 0.44 |
| SD | 0.00 | 0.43 | 1.06 | 1.03 | 0.99 | 1.02 | 1.41 | 1.16 | 1.56 | 1.52 | 1.36 | 1.59 | 1.69 | 1.70 |

**Table 3B: Forelimb Placing Total Score Group II**

| **Group 2** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 5 | 0.00 | 11.50 | 9.50 | 6.50 | 6.00 | 5.50 | 4.00 | 3.00 | 3.50 | 3.50 | 3.00 | 2.50 | 0.50 | 0.50 |
| No. 9 | 0.00 | 11.00 | 8.50 | 7.00 | 6.50 | 6.00 | 5.00 | 4.50 | 6.00 | 6.00 | 5.00 | 5.50 | 3.00 | 2.50 |
| No. 11 | 0.00 | 12.00 | 7.00 | 6.00 | 5.50 | 5.50 | 3.00 | 3.00 | 4.50 | 4.00 | 3.50 | 3.50 | 1.00 | 0.00 |
| No. 13 | 0.00 | 11.50 | 8.50 | 7.00 | 6.00 | 6.00 | 4.50 | 4.00 | 5.00 | 4.50 | 4.00 | 4.00 | 2.50 | 2.50 |
| No. 14 | 0.00 | 12.00 | 9.00 | 6.50 | 7.00 | 6.00 | 6.00 | 5.50 | 6.00 | 6.00 | 6.00 | 5.50 | 3.50 | 3.00 |
| No. 19 | 0.00 | 12.00 | 7.00 | 6.00 | 6.00 | 6.00 | 5.50 | 5.50 | 6.00 | 5.00 | 5.00 | 5.00 | 2.50 | 1.00 |
| No. 20 | 0.00 | 12.00 | 9.00 | 6.00 | 4.00 | 4.00 | 4.00 | 2.00 | 2.00 | 1.50 | 1.00 | 0.50 | 0.00 | 0.00 |
| No. 27 | 0.00 | 12.00 | 7.50 | 6.00 | 6.00 | 6.00 | 3.50 | 2.00 | 5.00 | 4.50 | 4.50 | 4.50 | 1.50 | 1.00 |
| No. 32 | 0.00 | 12.00 | 8:00 | 6.50 | 6.50 | 6.50 | 5.50 | 4.00 | 5.00 | 4.50 | 3.50 | 5.00 | 2.50 | 1.50 |
| No. 33 | 0.00 | 12.00 | 8.50 | 6.50 | 6.00 | 6.00 | 5.00 | 5.00 | 6.00 | 6.00 | 5.50 | 5.00 | 3.00 | 0.00 |
| No. 35 | 0.00 | 12.00 | 7.50 | 6.50 | 6.00 | 6.00 | 4.00 | 3.50 | 3.00 | 3.00 | 3.00 | 3.00 | 1.50 | 1.00 |
| No. 39 | 0.00 | 11.00 | 7.00 | 6.00 | 6.00 | 6.00 | 4.50 | 4.50 | 4.50 | 5.00 | 5.00 | 5.00 | 5.00 | 3.50 |
| No. 41 | 0.00 | 11.00 | 6.00 | 4.50 | 4.50 | 4.00 | 1.50 | 1.00 | 2.50 | 3.00 | 3.00 | 2.50 | 0.00 | 0.00 |
| No. 44 | 0.00 | 11.00 | 6.50 | 4.50 | 4.50 | 3.00 | 1.50 | 1.00 | 1.00 | 1.00 | 1.00 | 1.00 | 0.00 | 0.00 |
| No. 45 | 0.00 | 11.00 | 6.00 | 6.00 | 6.00 | 6.00 | 5.50 | 3.00 | 6.00 | 6.00 | 6.00 | 6.00 | 2.00 | 1.00 |
| | | | | | | | | | | | | | | |
| N | 15 | 15 | 15 | 15 | 15 | 15 | 15 | 15 | 15 | 15 | 15 | 15 | 15 | 15 |
| Mean | 0.00 | 11.60 | 7.70 | 6.10 | 5.77 | 5.50 | 4.20 | 3.43 | 4.40 | 4.23 | 3.93 | 3.90 | 1.90 | 1.17 |
| Sem | 0.00 | 0.12 | 0.29 | 0.19 | 0.21 | 0.26 | 0.35 | 0.38 | 0.42 | 0.41 | 0.41 | 0.43 | 0.38 | 0.31 |
| SD | 0.00 | 0.47 | 1.11 | 0.74 | 0.82 | 1.00 | 1.37 | 1.47 | 1.64 | 1.59 | 1.58 | 1.68 | 1.45 | 1.19 |

**Table 3C: Forelimb Placing Total Score Group III**

| **Group 3** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 1 | 0.00 | 12.00 | 9.00 | 7.00 | 6.50 | 6.00 | 6.00 | 6.00 | 6.00 | 5.00 | 1.00 | 4.50 | 3.00 | 3.00 |
| No. 4 | 0.00 | 12.00 | 7.00 | 6.00 | 6.00 | 6.00 | 4.00 | 4.00 | 4.00 | 1.50 | 1.00 | 3.00 | 1.50 | 1.00 |
| No. 6 | 0.00 | 11.50 | 8.00 | 5.00 | 5.00 | 5.00 | 5.00 | 5.00 | 4.50 | 3.00 | 1.50 | 3.50 | 3.00 | 3.00 |
| No. 8 | 0.00 | 11.00 | 6.00 | 4.50 | 4.50 | 4.50 | 3.00 | 3.00 | 1.50 | 0.00 | 0.00 | 0.00 | 0.00 | 0.00 |
| No. 10 | 0.00 | 11.50 | 8.00 | 6.00 | 6.00 | 6.00 | 6.00 | 6.00 | 5.50 | 4.50 | 2.50 | 4.50 | 3.00 | 2.50 |
| No. 15 | 0.00 | 12.00 | 8.00 | 7.00 | 6.00 | 6.00 | 6.00 | 6.00 | 5.50 | 4.00 | 3.00 | 4.00 | 3.00 | 3.00 |
| No. 16 | 0.00 | 12.00 | 7.00 | 6.00 | 5.50 | 5.00 | 3.50 | 3.00 | 2.00 | 1.00 | 0.00 | 0.50 | 0.50 | 1.00 |
| No. 17 | 0.00 | 11.50 | 8.50 | 7.00 | 6.50 | 6.00 | 5.50 | 6.00 | 5.50 | 4.00 | 2.00 | 3.00 | 2.00 | 2.00 |
| No. 22 | 0.00 | 12.00 | 9.00 | 7.00 | 6.50 | 6.50 | 6.50 | 6.50 | 6.00 | 4.00 | 3.00 | 6.00 | 3.50 | 3.50 |
| No. 26 | 0.00 | 12.00 | 9.00 | 6.00 | 5.00 | 5.50 | 5.00 | 4.00 | 3.00 | 2.50 | 1.50 | 3.00 | 1.50 | 0.50 |
| No. 29 | 0.00 | 12.00 | 8.50 | 7.00 | 6.50 | 6.50 | 6.00 | 6.00 | 6.00 | 3.00 | 0.00 | 6.00 | 2.50 | 2.00 |
| No. 34 | 0.00 | 12.00 | 8.50 | 6.50 | 6.00 | 6.00 | 6.00 | 6.00 | 6.00 | 4.50 | 1.50 | 6.00 | 4.00 | 3.00 |
| No. 36 | 0.00 | 12.00 | 7.50 | 6.50 | 5.00 | 5.50 | 4.50 | 4.50 | 6.00 | 3.00 | 3.00 | 4.00 | 2.50 | 2.50 |
| No. 40 | 0.00 | 11.00 | 6.50 | 6.00 | 5.00 | 4.00 | 3.00 | 1.50 | 2.00 | 0.00 | 0.00 | 0.00 | 0.00 | 0.00 |
| No. 43 | 0.00 | 11.50 | 6.00 | 5.00 | 4.00 | 3.00 | 1.50 | 1.50 | 1.00 | 0.00 | 0.00 | 0.00 | 0.00 | 0.00 |
| | | | | | | | | | | | | | | |
| N | 15 | 15 | 15 | 15 | 15 | 15 | 15 | 15 | 15 | 15 | 15 | 15 | 15 | 15 |
| Mean | 0.00 | 11.73 | 7.77 | 6.17 | 5.60 | 5.43 | 4.77 | 4.60 | 4.30 | 2.67 | 1.33 | 3.20 | 2.00 | 1.80 |
| Sem | 0.00 | 0.10 | 0.27 | 0.21 | 0.21 | 0.25 | 0.38 | 0.44 | 0.49 | 0.45 | 0.30 | 0.56 | 0.35 | 0.33 |
| SD | 0.00 | 0.37 | 1.05 | 0.82 | 0.81 | 0.98 | 1.47 | 1.70 | 1.89 | 1.76 | 1.18 | 2.18 | 1.35 | 1.26 |

**Table 4A: Hindlimb Placing Total Score Group I**

| **Group 1** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 2 | 0.00 | 6.00 | 3.50 | 3.00 | 3.00 | 3.00 | 1.50 | 1.50 | 2.50 | 2.50 | 2.00 | 2.50 | 2.50 | 2.50 |
| No. 3 | 0.00 | 6.00 | 3.50 | 3.00 | 3.00 | 2.50 | 1.50 | 0.00 | 0.00 | 0.00 | 0.00 | 0.00 | 0.00 | 0.00 |
| No. 7 | 0.00 | 6.00 | 3.50 | 3.50 | 3.00 | 2.50 | 1.50 | 1.50 | 2.50 | 0.00 | 0.00 | 1.50 | 1.50 | 1.50 |
| No. 12 | 0.00 | 5.00 | 3.00 | 3.00 | 2.50 | 2.00 | 0.50 | 0.00 | 1.50 | 0.50 | 0.50 | 0.50 | 1.00 | 1.00 |
| No. 18 | 0.00 | 6.00 | 4.00 | 3.50 | 3.00 | 3.00 | 1.50 | 0.50 | 2.50 | 1.00 | 1.00 | 1.50 | 1.50 | 1.50 |
| No. 21 | 0.00 | 6.00 | 4.50 | 3.00 | 3.00 | 3.00 | 1.50 | 1.00 | 2.50 | 2.00 | 2.00 | 2.00 | 2.00 | 2.00 |
| No. 23 | 0.00 | 6.00 | 4.50 | 3.50 | 3.50 | 3.00 | 3.00 | 3.00 | 3.00 | 3.00 | 3.00 | 2.00 | 3.00 | 2.00 |
| No. 24 | 0.00 | 6.00 | 3.50 | 3.00 | 3.00 | 3.00 | 1.50 | 1.50 | 3.00 | 2.00 | 1.50 | 1.50 | 2.00 | 2.00 |
| No. 25 | 0.00 | 6.00 | 4.00 | 3.00 | 3.00 | 3.00 | 2.50 | 1.50 | 3.00 | 2.00 | 0.00 | 2.00 | 2.50 | 2.50 |
| No. 28 | 0.00 | 6.00 | 4.00 | 3.50 | 3.00 | 3.00 | 3.00 | 1.50 | 2.50 | 1.50 | 1.50 | 2.50 | 3.00 | 2.50 |
| No. 30 | 0.00 | 6.00 | 3.50 | 3.00 | 3.00 | 3.00 | 1.50 | 0.00 | 2.50 | 2.00 | 2.00 | 3.00 | 3.00 | 3.00 |
| No. 31 | 0.00 | 6.00 | 4.00 | 3.00 | 3.00 | 3.00 | 2.50 | 0.50 | 3.00 | 1.50 | 1.50 | 1.50 | 1.50 | 1.50 |
| No. 37 | 0.00 | 6.00 | 4.00 | 3.00 | 3.00 | 3.00 | 2.00 | 1.50 | 2.50 | 1.50 | 1.50 | 2.50 | 2.50 | 2.50 |
| No. 38 | 0.00 | 5.50 | 3.50 | 3.00 | 3.00 | 3.00 | 1.00 | 0.50 | 1.50 | 0.50 | 0.50 | 0.50 | 0.50 | 0.50 |
| No. 42 | 0.00 | 6.00 | 3.00 | 3.00 | 3.00 | 2.50 | 0.00 | 0.00 | 1.50 | 0.00 | 0.00 | 0.00 | 0.00 | 0.00 |
| | | | | | | | | | | | | | | |
| N | 15 | 15 | 15 | 15 | 15 | 15 | 15 | 15 | 15 | 15 | 15 | 15 | 15 | 15 |
| Mean | 0.00 | 5.90 | 3.73 | 3.13 | 3.00 | 2.83 | 1.67 | 0.97 | 2.27 | 1.33 | 1.13 | 1.57 | 1.77 | 1.67 |
| Sem | 0.00 | 0.07 | 0.12 | 0.06 | 0.05 | 0.08 | 0.22 | 0.22 | 0.21 | 0.25 | 0.24 | 0.24 | 0.27 | 0.24 |
| SD | 0.00 | 0.28 | 0.46 | 0.23 | 0.19 | 0.31 | 0.84 | 0.85 | 0.82 | 0.96 | 0.93 | 0.94 | 1.03 | 0.94 |

**Table 4B: Hindlimb Placing Total Score Group II**

| **Group 2** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 5 | 0.00 | 6.00 | 3.50 | 3.00 | 3.00 | 3.00 | 2.00 | 1.00 | 2.50 | 2.00 | 2.00 | 2.00 | 0.50 | 0.50 |
| No. 9 | 0.00 | 6.00 | 3.50 | 3.00 | 3.00 | 3.00 | 1.50 | 1.50 | 2.50 | 3.00 | 3.00 | 3.00 | 1.50 | 0.00 |
| No. 11 | 0.00 | 6.00 | 3.50 | 2.50 | 2.50 | 2.00 | 1.50 | 1.50 | 1.50 | 1.50 | 2.00 | 1.50 | 0.00 | 0.00 |
| No. 13 | 0.00 | 6.00 | 4.00 | 3.00 | 3.00 | 3.00 | 2.00 | 2.00 | 2.50 | 2.50 | 2.50 | 2.50 | 2.00 | 0.50 |
| No. 14 | 0.00 | 5.50 | 3.00 | 3.00 | 3.00 | 3.00 | 3.00 | 1.50 | 2.00 | 2.00 | 2.00 | 2.00 | 0.50 | 0.00 |
| No. 19 | 0.00 | 6.00 | 3.50 | 3.00 | 3.00 | 3.00 | 2.50 | 2.00 | 3.00 | 2.50 | 2.50 | 2.50 | 2.00 | 0.50 |
| No. 20 | 0.00 | 6.00 | 4.50 | 3.00 | 3.00 | 3.00 | 1.50 | 1.00 | 2.50 | 1.50 | 0.50 | 0.50 | 0.00 | 0.00 |
| No. 27 | 0.00 | 6.00 | 3.00 | 3.00 | 2.50 | 2.50 | 2.00 | 1.50 | 2.00 | 2.00 | 2.00 | 2.00 | 1.00 | 1.00 |
| No. 32 | 0.00 | 6.00 | 4.00 | 3.00 | 3.00 | 2.50 | 2.00 | 1.50 | 2.00 | 2.00 | 2.00 | 2.00 | 1.50 | 0.50 |
| No. 33 | 0.00 | 6.00 | 4.50 | 3.50 | 3.00 | 3.00 | 2.00 | 1.50 | 3.00 | 3.00 | 3.00 | 3.00 | 1.50 | 0.00 |
| No. 35 | 0.00 | 6.00 | 3.50 | 3.00 | 2.50 | 2.50 | 1.50 | 1.50 | 1.50 | 1.50 | 1.50 | 0.50 | 0.00 | 0.00 |
| No. 39 | 0.00 | 5.50 | 3.00 | 3.00 | 3.00 | 3.00 | 1.50 | 1.00 | 1.50 | 2.00 | 1.50 | 1.50 | 1.50 | 0.50 |
| No. 41 | 0.00 | 5.00 | 3.00 | 2.50 | 2.50 | 2.00 | 1.00 | 1.00 | 1.50 | 1.50 | 1.50 | 1.00 | 0.00 | 0.00 |
| No. 44 | 0.00 | 6.00 | 3.50 | 3.00 | 3.00 | 1.50 | 0.50 | 0.50 | 1.00 | 1.00 | 1.00 | 1.00 | 0.00 | 0.00 |
| No. 45 | 0.00 | 6.00 | 3.50 | 3.00 | 3.00 | 3.00 | 1.50 | 1.50 | 2.00 | 2.00 | 2.00 | 2.00 | 0.50 | 0.00 |
| | | | | | | | | | | | | | | |
| N | 15 | 15 | 15 | 15 | 15 | 15 | 15 | 15 | 15 | 15 | 15 | 15 | 15 | 15 |
| Mean | 0.00 | 5.87 | 3.57 | 2.97 | 2.87 | 2.67 | 1.73 | 1.37 | 2.07 | 2.00 | 1.93 | 1.80 | 0.83 | 0.23 |
| Sem | 0.00 | 0.08 | 0.13 | 0.06 | 0.06 | 0.13 | 0.15 | 0.10 | 0.15 | 0.15 | 0.18 | 0.21 | 0.20 | 0.08 |
| SD | 0.00 | 0.30 | 0.50 | 0.23 | 0.23 | 0.49 | 0.59 | 0.40 | 0.59 | 0.57 | 0.68 | 0.80 | 0.77 | 0.32 |

**Table 4C: Hindlimb Placing Total Score Group III**

| **Group 3** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 1 | 0.00 | 6.00 | 4.00 | 4.00 | 3.50 | 3.00 | 3.00 | 3.00 | 3.00 | 3.00 | 1.50 | 3.00 | 1.50 | 1.50 |
| No. 4 | 0.00 | 6.00 | 3.50 | 3.00 | 3.00 | 3.00 | 2.50 | 2.00 | 2.00 | 0.00 | 0.00 | 1.50 | 0.00 | 0.00 |
| No. 6 | 0.00 | 6.00 | 3.50 | 3.00 | 3.00 | 3.00 | 3.00 | 3.00 | 2.50 | 0.50 | 0.00 | 0.50 | 0.50 | 0.50 |
| No. 8 | 0.00 | 5.50 | 4.00 | 3.50 | 2.50 | 2.50 | 2.50 | 1.50 | 2.00 | 0.00 | 0.00 | 0.00 | 0.00 | 0.00 |
| No. 10 | 0.00 | 5.50 | 3.00 | 3.00 | 3.00 | 3.00 | 3.00 | 3.00 | 2.50 | 1.50 | 0.50 | 2.00 | 1.00 | 1.00 |
| No. 15 | 0.00 | 6.00 | 3.50 | 3.50 | 3.00 | 3.00 | 3.00 | 3.00 | 3.00 | 2.00 | 1.00 | 2.00 | 1.50 | 1.50 |
| No. 16 | 0.00 | 6.00 | 3.00 | 3.00 | 3.00 | 3.00 | 2.50 | 2.00 | 1.50 | 0.00 | 0.00 | 1.00 | 0.50 | 1.00 |
| No. 17 | 0.00 | 5.50 | 3.50 | 3.00 | 3.00 | 3.00 | 3.00 | 3.00 | 3.00 | 2.00 | 0.50 | 1.50 | 1.00 | 1.00 |
| No. 22 | 0.00 | 6.00 | 4.50 | 3.00 | 3.00 | 3.00 | 3.00 | 3.00 | 3.00 | 1.50 | 0.50 | 1.50 | 0.50 | 0.50 |
| No. 26 | 0.00 | 6.00 | 3.50 | 3.00 | 2.50 | 3.00 | 2.50 | 2.00 | 1.50 | 1.00 | 0.50 | 1.50 | 1.00 | 0.50 |
| No. 29 | 0.00 | 6.00 | 4.50 | 3.50 | 3.00 | 3.00 | 3.00 | 3.00 | 3.00 | 1.00 | 0.00 | 3.00 | 3.00 | 2.00 |
| No. 34 | 0.00 | 6.00 | 3.50 | 3.00 | 3.00 | 3.00 | 2.00 | 2.00 | 2.50 | 0.50 | 0.00 | 2.50 | 1.00 | 1.00 |
| No. 36 | 0.00 | 6.00 | 3.00 | 2.50 | 2.50 | 2.50 | 1.50 | 1.50 | 2.00 | 0.50 | 0.00 | 1.50 | 0.50 | 0.00 |
| No. 40 | 0.00 | 5.50 | 4.00 | 3.00 | 2.50 | 2.50 | 2.00 | 1.50 | 1.50 | 0.00 | 0.00 | 0.00 | 0.00 | 0.00 |
| No. 43 | 0.00 | 5.50 | 3.50 | 3.00 | 3.00 | 3.00 | 2.00 | 1.50 | 1.00 | 0.00 | 0.00 | 1.00 | 0.00 | 0.00 |
| | | | | | | | | | | | | | | |
| N | 15 | IS | 15 | 15 | 15 | 15 | 15 | 15 | 15 | 15 | 15 | 15 | 15 | 15 |
| Mean | 0.00 | 5.83 | 3.63 | 3.13 | 2.90 | 2.90 | 2.57 | 2.33 | 2.27 | 0.90 | 0.30 | 1.50 | 0.80 | 0.70 |
| Sem | 0.00 | 0.06 | 0.12 | 0.09 | 0.07 | 0.05 | 0.13 | 0.17 | 0.18 | 0.24 | 0.12 | 0.24 | 0.21 | 0.17 |
| SD | 0.00 | 0.24 | 0.48 | 0.35 | 0.28 | 0.21 | 0.50 | 0.67 | 0.68 | 0.93 | 0.46 | 0.93 | 0.80 | 0.65 |

**Table 5A: Body Swing Test (% right Swing) Group I**

| **Group 1** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 2 | 50.00 | 0.00 | 6.67 | 13.33 | 23.33 | 36.67 | 33.33 | 33.33 | 40.00 | 46.67 | 53.33 | 50.00 | 46.67 | 43.33 |
| No. 3 | 46.67 | 0.00 | 3.33 | 6.67 | 10.00 | 33.33 | 26.67 | 30.00 | 23.33 | 30.00 | 20.00 | 16.67 | 26.67 | 40.00 |
| No. 7 | 50.00 | 0.00 | 10.00 | 16.67 | 10.00 | 13.33 | 36.67 | 40.00 | 16.67 | 40.00 | 20.00 | 26.67 | 13.33 | 20.00 |
| No. 12 | 50.00 | 6.67 | 33.33 | 30.00 | 26.67 | 36.67 | 36.67 | 33.33 | 26.67 | 30.00 | 33.33 | 36.67 | 26.67 | 36.67 |
| No. 18 | 50.00 | 0.00 | 0.00 | 23.33 | 26.67 | 33.33 | 46.67 | 50.00 | 30.00 | 43.33 | 40.00 | 43.33 | 30.00 | 26.67 |
| No. 21 | 53.33 | 0.00 | 0.00 | 6.67 | 13.33 | 20.00 | 33.33 | 43.33 | 26.67 | 33.33 | 36.67 | 33.33 | 30.00 | 26.67 |
| No. 23 | 50.00 | 0.00 | 20.00 | 46.67 | 46.67 | 46.67 | 53.33 | 50.00 | 46.67 | 56.67 | 43.33 | 43.33 | 40.00 | 43.33 |
| No. 24 | 50.00 | 0.00 | 0.00 | 6.67 | 23.33 | 16.67 | 33.33 | 53.33 | 40.00 | 43.33 | 43.33 | 40.00 | 36.67 | 36.67 |
| No. 25 | 46.67 | 0.00 | 6.67 | 26.67 | 10.00 | 16.67 | 40.00 | 53.33 | 33.33 | 26.67 | 33.33 | 23.33 | 30.00 | 33.33 |
| No. 28 | 50.00 | 0.00 | 6.67 | 3.33 | 6.67 | 33.33 | 30.00 | 26.67 | 33.33 | 33.33 | 36.67 | 40.00 | 33.33 | 33.33 |
| No. 30 | 50.00 | 0.00 | 0.00 | 13.33 | 30.00 | 23.33 | 36.67 | 43.33 | 33.33 | 33.33 | 36.67 | 30.00 | 30.00 | 30.00 |
| No. 31 | 46.67 | 0.00 | 0.00 | 10.00 | 6.67 | 16.67 | 36.67 | 33.33 | 33.33 | 26.67 | 30.00 | 23.33 | 33.33 | 43.33 |
| No. 37 | 50.00 | 0.00 | 0.00 | 26.67 | 20.00 | 13.33 | 43.33 | 53.33 | 50.00 | 50.00 | 43.33 | 33.33 | 33.33 | 33.33 |
| No. 38 | 46.67 | 0.00 | 0.00 | 10.00 | 6.67 | 33.33 | 40.00 | 50.00 | 50.00 | 43.33 | 43.33 | 40.00 | 40.00 | 36.67 |
| No. 42 | 50.00 | 0.00 | 0.00 | 3.33 | 3.33 | 13.33 | 36.67 | 40.00 | 43.33 | 40.00 | 33.33 | 33.33 | 36.67 | 30.00 |
| | | | | | | | | | | | | | | |
| N | 15 | 15 | 15 | 15 | 15 | 15 | 15 | 15 | 15 | 15 | 15 | 15 | 15 | 15 |
| Mean | 49.33 | 0.44 | 5.78 | 16.22 | 17.56 | 25.78 | 37.56 | 42.22 | 35.11 | 38.44 | 36.44 | 34.22 | 32.44 | 34.22 |
| Sem | 0.48 | 0.44 | 2.44 | 3.15 | 3.08 | 2.81 | 1.70 | 2.38 | 2.54 | 2.30 | 2.29 | 2.33 | 1.96 | 1.77 |
| SD | 1.87 | 1.72 | 9.47 | 12.21 | 11.92 | 10.87 | 6.60 | 9.23 | 9.83 | 8.90 | 8.86 | 9.04 | 7.61 | 6.84 |

**Table 5B: Body Swing Test (% right Swing) Group II**

| **Group 2** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 5 | 46.67 | 0.00 | 3.33 | 3.33 | 0.00 | 33.33 | 30.00 | 43.33 | 26.67 | 30.00 | 30.00 | 33.33 | 36.67 | 40.00 |
| No. 9 | 50.00 | 0.00 | 6.67 | 13.33 | 20.00 | 30.00 | 43.33 | 46.67 | 33.33 | 33.33 | 36.67 | 40.00 | 50.00 | 40.00 |
| No. 11 | 46.67 | 0.00 | 36.67 | 36.67 | 36.67 | 33.33 | 53.33 | 43.33 | 36.67 | 40.00 | 43.33 | 43.33 | 46.67 | 50.00 |
| No. 13 | 50.00 | 3.33 | 0.00 | 20.00 | 16.67 | 26.67 | 26.67 | 33.33 | 10.00 | 23.33 | 30.00 | 26.67 | 33.33 | 40.00 |
| No. 14 | 46.67 | 0.00 | 0.00 | 23.33 | 26.67 | 30.00 | 26.67 | 30.00 | 20.00 | 26.67 | 33.33 | 33.33 | 43.33 | 50.00 |
| No. 19 | 50.00 | 0.00 | 6.67 | 16.67 | 26.67 | 23.33 | 20.00 | 33.33 | 33.33 | 33.33 | 33.33 | 36.67 | 46.67 | 43.33 |
| No. 20 | 46.67 | 0.00 | 16.67 | 26.67 | 26.67 | 13.33 | 40.00 | 33.33 | 36.67 | 33.33 | 30.00 | 30.00 | 33.33 | 40.00 |
| No. 27 | 53.33 | 0.00 | 0.00 | 16.67 | 16.67 | 16.67 | 16.67 | 33.33 | 26.67 | 16.67 | 33.33 | 23.33 | 26.67 | 33.33 |
| No. 32 | 53.33 | 0.00 | 3.33 | 6.67 | 10.00 | 16.67 | 30.00 | 33.33 | 33.33 | 33.33 | 30.00 | 26.67 | 40.00 | 46.67 |
| No. 33 | 50.00 | 0.00 | 0.00 | 16.67 | 16.67 | 23.33 | 36.67 | 33.33 | 30.00 | 20.00 | 20.00 | 33.33 | 33.33 | 36.67 |
| No. 35 | 46.67 | 0.00 | 0.00 | 26.67 | 23.33 | 13.33 | 33.33 | 40.00 | 43.33 | 26.67 | 26.67 | 43.33 | 43.33 | 50.00 |
| No. 39 | 50.00 | 0.00 | 23.33 | 23.33 | 26.67 | 30.00 | 23.33 | 43.33 | 46.67 | 43.33 | 43.33 | 43.33 | 46.67 | 50.00 |
| No. 41 | 50.00 | 6.67 | 6.67 | 13.33 | 16.67 | 13.33 | 33.33 | 30.00 | 33.33 | 26.67 | 33.33 | 26.67 | 46.67 | 40.00 |
| No. 44 | 50.00 | 0.00 | 13.33 | 6.67 | 20.00 | 30.00 | 53.33 | 46.67 | 46.67 | 50.00 | 46.67 | 50.00 | 50.00 | 46.67 |
| No. 45 | 50.00 | 0.00 | 0.00 | 0.00 | 3.33 | 26.67 | 26.67 | 43.33 | 46.67 | 30.00 | 26.67 | 23.33 | 33.33 | 33.33 |
| | | | | | | | | | | | | | | |
| N | 15 | 15 | 15 | 15 | 15 | 15 | 15 | 15 | 15 | 15 | 15 | 15 | 15 | 15 |
| Mean | 49.33 | 0.67 | 7.78 | 16.67 | 19.11 | 24.00 | 32.89 | 37.78 | 33.56 | 31.11 | 33.11 | 34.22 | 40.67 | 42.67 |
| Sem | 0.58 | 0.48 | 2.75 | 2.56 | 2.47 | 1.93 | 2.81 | 1.58 | 2.65 | 2.25 | 1.82 | 2.14 | 1.90 | 1.53 |
| SD | 2.25 | 1.87 | 10.67 | 9.92 | 9.55 | 7.47 | 10.90 | 6.13 | 10.27 | 8.70 | 7.07 | 8.31 | 7.37 | 5.94 |

**Table 5C: Body Swing Test (% right Swing) Group III**

| **Group 3** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 1 | 50.00 | 0.00 | 20.00 | 23.33 | 30.00 | 23.33 | 30.00 | 33.33 | 33.33 | 43.33 | 43.33 | 33.33 | 30.00 | 26.67 |
| No. 4 | 50.00 | 3.33 | 30.00 | 23.33 | 16.67 | 16.67 | 13.33 | 16.67 | 26.67 | 40.00 | 20.00 | 30.00 | 20.00 | 16.67 |
| No. 6 | 46.67 | 0.00 | 6.67 | 10.00 | 3.33 | 10.00 | 13.33 | 10.00 | 6.67 | 23.33 | 23.33 | 26.67 | 20.00 | 23.33 |
| No. 8 | 50.00 | 6.67 | 30.00 | 20.00 | 43.33 | 33.33 | 20.00 | 43.33 | 40.00 | 46.67 | 40.00 | 46.67 | 46.67 | 50.00 |
| No. 10 | 53.33 | 0.00 | 0.00 | 23.33 | 20.00 | 30.00 | 26.67 | 30.00 | 26.67 | 33.33 | 30.00 | 33.33 | 33.33 | 36.67 |
| No. 15 | 50.00 | 0.00 | 6.67 | 33.33 | 33.33 | 36.67 | 30.00 | 36.67 | 36.67 | 40.00 | 40.00 | 36.67 | 30.00 | 30.00 |
| No. 16 | 46.67 | 0.00 | 30.00 | 30.00 | 36.67 | 40.00 | 33.33 | 20.00 | 26.67 | 43.33 | 43.33 | 43.33 | 46.67 | 43.33 |
| No. 17 | 50.00 | 6.67 | 16.67 | 43.33 | 36.67 | 36.67 | 36.67 | 40.00 | 36.67 | 46.67 | 40.00 | 43.33 | 40.00 | 36.67 |
| No. 22 | 53.33 | 0.00 | 0.00 | 10.00 | 26.67 | 13.33 | 16.67 | 33.33 | 30.00 | 53.33 | 50.00 | 43.33 | 40.00 | 36.67 |
| No. 26 | 53.33 | 0.00 | 6.67 | 33.33 | 33.33 | 36.67 | 30.00 | 26.67 | 33.33 | 33.33 | 30.00 | 26.67 | 30.00 | 33.33 |
| No. 29 | 50.00 | 0.00 | 6.67 | 6.67 | 10.00 | 16.67 | 26.67 | 16.67 | 20.00 | 13.33 | 53.33 | 10.00 | 20.00 | 33.33 |
| No. 34 | 50.00 | 0.00 | 0.00 | 30.00 | 23.33 | 16.67 | 33.33 | 33.33 | 36.67 | 36.67 | 43.33 | 33.33 | 33.33 | 36.67 |
| No. 36 | 50.00 | 3.33 | 20.00 | 36.67 | 36.67 | 40.00 | 40.00 | 46.67 | 46.67 | 50.00 | 53.33 | 46.67 | 53.33 | 50.00 |
| No. 40 | 46.67 | 10.00 | 13.33 | 20.00 | 33.33 | 46.67 | 53.33 | 50.00 | 46.67 | 50.00 | 53.33 | 46.67 | 46.67 | 43.33 |
| No. 43 | 46.67 | 0.00 | 6.67 | 10.00 | 10.00 | 16.67 | 13.33 | 40.00 | 40.00 | 40.00 | 43.33 | 33.33 | 33.33 | 30.00 |
| | | | | | | | | | | | | | | |
| N | 15 | 15 | 15 | 15 | 15 | 15 | 15 | 15 | 15 | 15 | 15 | 15 | 15 | 15 |
| Mean | 49.78 | 2.00 | 12.89 | 23.56 | 26.22 | 27.56 | 27.78 | 31.78 | 32.44 | 39.56 | 40.44 | 35.56 | 34.89 | 35.11 |
| Sem | 0.61 | 0.85 | 2.83 | 2.84 | 3.07 | 3.06 | 2.90 | 3.05 | 2.67 | 2.74 | 2.72 | 2.60 | 2.72 | 2.39 |
| SD | 2.35 | 3.29 | 10.97 | 11.02 | 11.88 | 11.85 | 11.25 | 11.81 | 10.35 | 10.61 | 10.53 | 10.05 | 10.53 | 9.25 |

**Table 6A: Weight (g) Group I**

| **Group 1** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 2 | 301 | 284 | 319 | 340 | 355 | 358 | 360 | 351 | 392 | 400 | 386 | 419 | 426 | 415 |
| No. 3 | 305 | 297 | 322 | 358 | 388 | 413 | 422 | 404 | 436 | 440 | 442 | 457 | 463 | 470 |
| No. 7 | 318 | 301 | 334 | 380 | 405 | 407 | 425 | 412 | 444 | 456 | 446 | 463 | 477 | 470 |
| No. 12 | 326 | 323 | 359 | 421 | 457 | 487 | 496 | 477 | 490 | 494 | 490 | 516 | 516 | 513 |
| No. 18 | 295 | 277 | 301 | 334 | 357 | 366 | 372 | 360 | 387 | 390 | 379 | 400 | 405 | 396 |
| No. 21 | 305 | 291 | 328 | 375 | 399 | 427 | 425 | 411 | 448 | 455 | 448 | 476 | 480 | 475 |
| No. 23 | 317 | 317 | 361 | 403 | 419 | 451 | 450 | 441 | 491 | 501 | 490 | 509 | 520 | 511 |
| No. 24 | 311 | 306 | 359 | 394 | 416 | 436 | 435 | 417 | 449 | 447 | 440 | 452 | 460 | 456 |
| No. 25 | 332 | 315 | 375 | 421 | 444 | 484 | 490 | 470 | 499 | 511 | 494 | 523 | 530 | 527 |
| No. 28 | 340 | 323 | 369 | 436 | 494 | 525 | 544 | 515 | 565 | 566 | 570 | 612 | 625 | 615 |
| No. 30 | 324 | 294 | 340 | 382 | 428 | 457 | 470 | 454 | 479 | 480 | 488 | 496 | 512 | 509 |
| No. 31 | 330 | 317 | 366 | 422 | 469 | 506 | 512 | 496 | 543 | 545 | 552 | 576 | 585 | 587 |
| No. 37 | 313 | 300 | 335 | 382 | 380 | 423 | 434 | 422 | 447 | 454 | 457 | 467 | 473 | 467 |
| No. 38 | 322 | 315 | 359 | 404 | 440 | 466 | 482 | 453 | 499 | 502 | 494 | 524 | 530 | 521 |
| No. 42 | 320 | 311 | 344 | 395 | 450 | 469 | 480 | 461 | 497 | 502 | 491 | 500 | 515 | 505 |
| | | | | | | | | | | | | | | |
| N | 15 | 15 | 15 | 15 | 15 | 15 | 15 | 15 | 15 | 15 | 15 | 15 | 15 | 15 |
| Mean | 317.3 | 304.7 | 344.7 | 389.8 | 420.1 | 445.0 | 453.1 | 436.3 | 471.1 | 476.2 | 471.1 | 492.7 | 501.1 | 495.8 |
| Sem | 3.2 | 3.7 | 5.5 | 7.7 | 10.4 | 12.3 | 13.0 | 11.9 | 12.7 | 12.5 | 13.4 | 14.3 | 14.6 | 14.7 |
| SD | 12.4 | 14.2 | 21.4 | 30.0 | 40.3 | 47.6 | 50.2 | 46.1 | 49.2 | 48.6 | 51.9 | 55.2 | 56.6 | 57.1 |

**Table 6B: Weight (g) Group II**

| **Group 2** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 5 | 303 | 278 | 329 | 364 | 409 | 418 | 434 | 424 | 460 | 470 | 465 | 486 | 499 | 488 |
| No. 9 | 304 | 299 | 334 | 364 | 389 | 405 | 420 | 403 | 430 | 440 | 431 | 445 | 456 | 444 |
| No. 11 | 311 | 305 | 330 | 370 | 398 | 415 | 416 | 412 | 454 | 455 | 447 | 464 | 470 | 447 |
| No. 13 | 311 | 309 | 318 | 328 | 359 | 379 | 380 | 374 | 401 | 405 | 394 | 428 | 432 | 417 |
| No. 14 | 316 | 304 | 339 | 381 | 399 | 413 | 431 | 428 | 460 | 455 | 461 | 480 | 483 | 471 |
| No. 19 | 322 | 307 | 353 | 406 | 438 | 479 | 474 | 473 | 492 | 505 | 496 | 524 | 530 | 517 |
| No. 20 | 296 | 278 | 315 | 333 | 364 | 373 | 380 | 369 | 394 | 402 | 391 | 415 | 425 | 410 |
| No. 27 | 319 | 297 | 341 | 383 | 394 | 410 | 414 | 410 | 428 | 441 | 432 | 455 | 461 | 444 |
| No. 32 | 330 | 292 | 338 | 388 | 418 | 434 | 444 | 426 | 458 | 460 | 448 | 472 | 477 | 460 |
| No. 33 | 307 | 286 | 325 | 360 | 390 | 405 | 410 | 406 | 429 | 426 | 427 | 443 | 455 | 441 |
| No. 35 | 332 | 308 | 353 | 401 | 436 | 466 | 480 | 455 | 500 | 505 | 499 | 529 | 540 | 519 |
| No. 39 | 345 | 332 | 388 | 454 | 494 | 525 | 540 | 541 | 577 | 596 | 591 | 620 | 635 | 614 |
| No. 41 | 323 | 322 | 362 | 412 | 444 | 467 | 475 | 467 | 500 | 510 | 510 | 530 | 535 | 508 |
| No. 44 | 318 | 315 | 358 | 424 | 472 | 491 | 512 | 508 | 533 | 540 | 545 | 550 | 570 | 535 |
| No. 45 | 338 | 342 | 362 | 402 | 443 | 470 | 480 | 476 | 515 | 521 | 522 | 538 | 546 | 530 |
| | | | | | | | | | | | | | | |
| N | 15 | 15 | 15 | 15 | 15 | 15 | 15 | 15 | 15 | 15 | 15 | 15 | 15 | 15 |
| Mean | 318.3 | 304.9 | 343.0 | 384.7 | 416.5 | 436.7 | 446.0 | 438.1 | 468.7 | 475.4 | 470.6 | 491.9 | 500.9 | 483.0 |
| Sem | 3.5 | 4.7 | 5.1 | 8.6 | 9.8 | 11.3 | 12.0 | 12.4 | 13.1 | 13.8 | 14.5 | 14.3 | 14.9 | 14.1 |
| SD | 13.7 | 18.1 | 19.6 | 33.5 | 37.9 | 43.8 | 46.4 | 48.0 | 50.6 | 53.5 | 56.1 | 55.5 | 57.6 | 54.5 |

**Table 6C: Weight (g) Group III**

| **Group 3** | D-1 | D1 | D7 | D14 | D21 | D28 | D30 | D32 | D42 | D44 | D46 | D56 | D58 | D60 |
|---|---|---|---|---|---|---|---|---|---|---|---|---|---|---|
| | | | | | | | | | | | | | | |
| No. 1 | 311 | 306 | 345 | 384 | 418 | 438 | 445 | 445 | 455 | 463 | 446 | 480 | 492 | 468 |
| No. 4 | 303 | 289 | 309 | 357 | 389 | 403 | 419 | 412 | 440 | 442 | 430 | 455 | 462 | 453 |
| No. 6 | 307 | 290 | 337 | 372 | 403 | 413 | 429 | 427 | 440 | 445 | 430 | 452 | 469 | 456 |
| No. 8 | 306 | 316 | 332 | 362 | 392 | 405 | 419 | 416 | 444 | 453 | 436 | 460 | 476 | 470 |
| No. 10 | 306 | 287 | 309 | 350 | 381 | 397 | 411 | 413 | 438 | 445 | 412 | 446 | 461 | 447 |
| No. 15 | 312 | 298 | 335 | 375 | 397 | 425 | 422 | 418 | 451 | 460 | 440 | 461 | 475 | 466 |
| No. 16 | 319 | 316 | 341 | 371 | 392 | 414 | 427 | 425 | 451 | 461 | 436 | 467 | 486 | 479 |
| No. 17 | 311 | 317 | 338 | 376 | 394 | 425 | 440 | 441 | 454 | 469 | 455 | 476 | 485 | 484 |
| No. 22 | 315 | 296 | 339 | 382 | 405 | 430 | 430 | 430 | 455 | 451 | 435 | 462 | 467 | 463 |
| No. 26 | 296 | 284 | 333 | 369 | 376 | 404 | 405 | 401 | 415 | 423 | 402 | 425 | 440 | 430 |
| No. 29 | 307 | 298 | 327 | 371 | 405 | 430 | 436 | 429 | 460 | 462 | 442 | 466 | 472 | 465 |
| No. 34 | 323 | 303 | 346 | 394 | 414 | 425 | 433 | 433 | 460 | 463 | 435 | 483 | 490 | 484 |
| No. 36 | 332 | 317 | 370 | 422 | 467 | 498 | 500 | 504 | 515 | 532 | 502 | 535 | 550 | 550 |
| No. 40 | 346 | 338 | 401 | 456 | 508 | 521 | 540 | 530 | 530 | 572 | 567 | 613 | 625 | 613 |
| No. 43 | 318 | 297 | 350 | 412 | 458 | 487 | 500 | 496 | 523 | 538 | 497 | 553 | 573 | 549 |
| | | | | | | | | | | | | | | |
| N | 15 | 15 | 15 | 15 | 15 | 15 | 15 | 15 | 15 | 15 | 15 | 15 | 15 | 15 |
| Mean | 314.1 | 303.5 | 340.8 | 383.5 | 413.3 | 434.3 | 443.7 | 441.3 | 462.1 | 471.9 | 451.0 | 482.3 | 494.9 | 485.1 |
| Sem | 3.2 | 3.8 | 5.8 | 7.1 | 9.4 | 9.7 | 9.9 | 9.8 | 8.6 | 10.7 | 10.7 | 12.5 | 12.8 | 12.5 |
| SD | 12.4 | 14.8 | 22.4 | 27.7 | 36.5 | 37.5 | 38.5 | 37.9 | 33.4 | 41.4 | 41.6 | 48.6 | 49.5 | 48.4 |

**Table 7A: Cylinder Test Score (Overall Asymmetry (%)) Group I**

| **Group 1** | D-1 | D7 | D21 | D30 | D32 | D44 | D46 | D58 | D60 |
|---|---|---|---|---|---|---|---|---|---|
| | | | | | | | | | |
| No. 2 | 24.49 | 36.51 | 53.85 | 50.00 | | 40.00 | 50.00 | 28.57 | 50.00 |
| No. 3 | 28.36 | -38.89 | | | 50.00 | 33.33 | 33.33 | 43.75 | 53.13 |
| No. 7 | 0.97 | -6.45 | -56.25 | -100.00 | -100.00 | 9.30 | 7.69 | 10.87 | -10.81 |
| No. 12 | 11.90 | 60.87 | -32.00 | 80.00 | 16.67 | 10.00 | -8.33 | 0.00 | -9.52 |
| No. 18 | -11.76 | 34.62 | 28.13 | | -56.52 | -28.57 | | 75.00 | 28.57 |
| No. 21 | 25.00 | 51.91 | 52.94 | | 36.36 | 60.71 | 63.64 | 57.14 | 71.43 |
| No. 23 | 0.00 | 40.00 | 24.49 | 9.33 | 24.07 | 25.81 | 14.89 | 27.74 | 32.10 |
| No. 24 | -1.23 | 48.05 | 41.94 | 0.00 | 22.73 | 39.58 | 30.23 | 33.33 | 39.39 |
| No. 25 | -5.77 | 22.81 | 28.36 | 50.00 | 5.13 | 22.81 | 33.33 | 32.10 | 29.55 |
| No. 28 | 6.00 | 24.46 | 43.60 | | -30.00 | 31.91 | 36.96 | 39.62 | 42.86 |
| No. 30 | -9.43 | -8.57 | 28.57 | 16.67 | -100.00 | 33.33 | -8.33 | 12.50 | 11.11 |
| No. 31 | 0.00 | 36.84 | 34.29 | 50.00 | 50.00 | 39.02 | 33.33 | 43.24 | 36.47 |
| No. 37 | -7.14 | 26.67 | -12.50 | 38.46 | -60.00 | -45.45 | 25.00 | | |
| No. 38 | -6.45 | 37.50 | -1.92 | | | -60.00 | | 9.09 | -33.33 |
| No. 42 | -26.09 | 7.14 | -22.73 | -16.67 | -65.79 | -56.06 | -100.00 | -65.38 | -100.00 |
| | | | | | | | | | |
| N | 15 | 15 | 14 | 10 | 13 | 15 | 13 | 14 | 14 |
| Mean | 1.92 | 24.90 | 15.05 | 17.78 | -15.95 | 10.38 | 16.29 | 24.83 | 17.21 |
| Sem | 3.90 | 6.84 | 9.15 | 15.94 | 15.28 | 10.00 | 11.25 | 8.76 | 11.76 |
| SD | 15.09 | 26.48 | 34.24 | 50.40 | 55.10 | 38.73 | 40.58 | 32.79 | 43.99 |

**Table 7B: Cylinder Test Score (Overall Asymmetry (%)) Group II**

| **Group 2** | D-1 | D7 | D21 | D30 | D32 | D44 | D46 | D58 | D60 |
|---|---|---|---|---|---|---|---|---|---|
| | | | | | | | | | |
| No. 5 | -2.70 | 14.29 | -25.00 | 20.00 | -33.33 | -20.00 | 2.86 | | -100.00 |
| No. 9 | -5.66 | 31.82 | 16.67 | 12.50 | 9.09 | 0.00 | -11.54 | 42.86 | 0.00 |
| No. 11 | -9.52 | 40.54 | 0.00 | 6.90 | 50.00 | -9.76 | 10.00 | -6.67 | 23.08 |
| No. 13 | -14.08 | 20.00 | -19.35 | 23.53 | 4.35 | 2.63 | -16.67 | 33.33 | -22.22 |
| No. 14 | -10.59 | 57.89 | 60.00 | 38.10 | 0.00 | 0.00 | 42.86 | | -100.00 |
| No. 19 | -15.58 | 55.47 | 56.25 | 51.72 | 63.29 | 58.33 | 56.67 | 29.17 | 64.00 |
| No. 20 | -1.96 | 28.85 | -23.17 | 0.00 | 18.18 | 14.04 | 28.57 | 50.00 | 20.00 |
| No. 27 | -24.39 | 25.00 | 66.67 | 4.17 | 33.33 | 38.96 | 34.85 | 66.67 | 14.29 |
| No. 32 | 2.38 | 51.56 | 49.06 | 56.64 | 54.78 | 52.53 | 31.40 | 54.76 | 62.50 |
| No. 33 | 0.00 | 50.00 | 46.43 | 27.59 | 22.73 | 11.54 | 19.05 | 30.00 | 10.00 |
| No. 35 | 1.65 | 58.33 | 66.67 | 75.00 | 62.50 | 54.93 | 29.33 | 88.00 | 76.47 |
| No. 39 | -2.20 | 42.36 | 25.32 | 20.99 | 16.00 | 7.14 | 40.35 | -7.69 | 13.95 |
| No. 41 | -7.58 | 28.57 | 47.83 | 27.50 | 20.00 | 18.18 | 46.15 | | |
| No. 44 | 21.05 | 43.14 | 22.22 | 23.33 | 3.85 | -27.08 | 16.95 | -16.67 | 0.00 |
| No. 45 | 8.45 | 24.32 | 17.86 | 16.67 | 27.66 | 17.65 | 42.86 | 62.50 | 0.00 |
| | | | | | | | | | |
| N | 15 | 15 | 15 | 15 | 15 | 15 | 15 | 12 | 14 |
| Mean | -4.05 | 38.14 | 27.16 | 26.98 | 23.50 | 14.61 | 24.91 | 35.52 | 4.43 |
| Sem | 2.78 | 3.75 | 8.37 | 5.35 | 6.81 | 6.82 | 5.51 | 9.35 | 13.97 |
| SD | 10.76 | 14.51 | 32.41 | 20.70 | 26.38 | 26.40 | 21.35 | 32.38 | 52.26 |

**Table 7C: Cylinder Test Score (Overall Asymmetry (%)) Group III**

| **Group 3** | D-1 | D7 | D21 | D30 | D32 | D44 | D46 | D58 | D60 |
|---|---|---|---|---|---|---|---|---|---|
| | | | | | | | | | |
| No. 1 | -17.65 | 28.57 | 25.00 | 35.59 | 16.67 | 48.28 | 19.23 | 23.91 | 16.00 |
| No. 4 | 6.41 | 40.00 | 41.67 | 50.00 | 42.11 | 36.36 | 0.00 | 5.00 | 31.25 |
| No. 6 | 0.00 | 28.13 | 12.96 | 21.57 | 42.11 | | 36.36 | 14.29 | -6.67 |
| No. 8 | 0.00 | 25.81 | 8.33 | 8.57 | 0.00 | -33.33 | 6.90 | 21.43 | -5.71 |
| No. 10 | 10.00 | 0.00 | 0.00 | -7.41 | 57.14 | 28.57 | -22.22 | 14.29 | 0.00 |
| No. 15 | -4.05 | 54.47 | 46.30 | 28.57 | 34.15 | 56.67 | 32.35 | 24.24 | 6.25 |
| No. 16 | 4.82 | 46.67 | -80.00 | -21.31 | -46.15 | -16.67 | -28.57 | 5.56 | 9.09 |
| No. 17 | 3.75 | 41.89 | 15.52 | 30.89 | 29.68 | 40.00 | 32.73 | 17.95 | 16.13 |
| No. 22 | -7.55 | 44.74 | 64.81 | 56.82 | 46.88 | 20.00 | 50.00 | 55.56 | 52.70 |
| No. 26 | -4.55 | 36.51 | 0.00 | -17.74 | 20.00 | 37.50 | | 0.00 | -23.33 |
| No. 29 | -20.00 | 42.35 | 46.94 | 49.18 | 23.91 | 100.00 | -60.00 | 21.74 | -25.00 |
| No. 34 | 8.33 | 51.09 | 42.86 | 47.69 | 15.00 | 0.00 | 55.56 | 0.00 | 0.00 |
| No. 36 | -6.25 | 58.46 | 30.00 | 35.21 | 15.22 | 35.90 | 17.07 | 26.03 | 22.45 |
| No. 40 | -2.80 | 18.18 | -10.87 | -9.09 | -10.81 | 10.53 | -7.14 | -8.82 | -36.36 |
| No. 43 | 0.97 | 26.09 | 21.74 | 2.27 | 28.57 | | | -36.36 | -8.33 |
| | | | | | | | | | |
| N | 15 | 15 | 15 | 15 | 15 | 13 | 13 | 15 | 15 |
| Mean | -1.90 | 36.20 | 17.68 | 20.72 | 20.96 | 27.98 | 10.17 | 12.32 | 3.23 |
| Sem | 2.23 | 3.96 | 8.84 | 6.80 | 6.63 | 9.37 | 9.25 | 5.24 | 5.93 |
| SD | 8.65 | 15.32 | 34.25 | 26.33 | 25.68 | 33.78 | 33.36 | 20.29 | 22.96 |

**Table 8A: Cylinder Test (Total Movement) Group I**

| **Group 1** | D-1 | D7 | D21 | D30 | D32 | D44 | D46 | D58 | D60 |
|---|---|---|---|---|---|---|---|---|---|
| | | | | | | | | | |
| No. 2 | 49.00 | 63.00 | 13.00 | 4.00 | 0.00 | 25.00 | 2.00 | 7.00 | 4.00 |
| No. 3 | 67.00 | 18.00 | 0.00 | 0.00 | 2.00 | 6.00 | 6.00 | 16.00 | 32.00 |
| No. 7 | 103.00 | 31.00 | 16.00 | 5.00 | 1.00 | 43.00 | 39.00 | 46.00 | 37.00 |
| No. 12 | 42.00 | 46.00 | 25.00 | 5.00 | 6.00 | 20.00 | 24.00 | 16.00 | 21.00 |
| No. 18 | 68.00 | 26.00 | 32.00 | 0.00 | 23.00 | 7.00 | 0.00 | 32.00 | 14.00 |
| No. 21 | 64.00 | 131.00 | 51.00 | 0.00 | 11.00 | 28.00 | 11.00 | 14.00 | 14.00 |
| No. 23 | 152.00 | 130.00 | 98.00 | 75.00 | 54.00 | 93.00 | 47.00 | 137.00 | 81.00 |
| No. 24 | 81.00 | 77.00 | 31.00 | 2.00 | 22.00 | 48.00 | 43.00 | 18.00 | 33.00 |
| No. 25 | 52.00 | 57.00 | 67.00 | 10.00 | 39.00 | 57.00 | 48.00 | 81.00 | 44.00 |
| No. 28 | 100.00 | 139.00 | 172.00 | 0.00 | 10.00 | 47.00 | 46.00 | 53.00 | 28.00 |
| No. 30 | 53.00 | 35.00 | 28.00 | 6.00 | 8.00 | 9.00 | 12.00 | 16.00 | 9.00 |
| No. 31 | 47.00 | 57.00 | 70.00 | 10.00 | 14.00 | 123.00 | 63.00 | 74.00 | 85.00 |
| No. 37 | 14.00 | 15.00 | 8.00 | 13.00 | 15.00 | 11.00 | 4.00 | 0.00 | 0.00 |
| No. 38 | 31.00 | 32.00 | 52.00 | 0.00 | 0.00 | 5.00 | 0.00 | 11.00 | 3.00 |
| No. 42 | 23.00 | 14.00 | 22.00 | 12.00 | 38.00 | 66.00 | 13.00 | 26.00 | 6.00 |
| | | | | | | | | | |
| N | 15 | 15 | 15 | 15 | 15 | 15 | 15 | 15 | 15 |
| Mean | 63.07 | 58.07 | 45.67 | 9.47 | 16.20 | 39.20 | 23.87 | 36.47 | 27.40 |
| Sem | 9.10 | 11.12 | 11.33 | 4.83 | 4.20 | 8.95 | 5.56 | 9.50 | 6.80 |
| SD | 35.25 | 43.05 | 43.89 | 18.71 | 16.27 | 34.66 | 21.53 | 36.78 | 26.32 |

**Table 8B: Cylinder Test (Total Movement) Group II**

| **Group 2** | D-1 | D7 | D21 | D30 | D32 | D44 | D46 | D58 | D60 |
|---|---|---|---|---|---|---|---|---|---|
| | | | | | | | | | |
| No. 5 | 74.00 | 14.00 | 8.00 | 35.00 | 9.00 | 35.00 | 35.00 | 0.00 | 1.00 |
| No. 9 | 53.00 | 44.00 | 6.00 | 8.00 | 11.00 | 31.00 | 26.00 | 7.00 | 1.00 |
| No. 11 | 42.00 | 37.00 | 14.00 | 29.00 | 2.00 | 41.00 | 30.00 | 15.00 | 13.00 |
| No. 13 | 71.00 | 50.00 | 31.00 | 17.00 | 23.00 | 38.00 | 24.00 | 3.00 | 9.00 |
| No. 14 | 85.00 | 19.00 | 5.00 | 21.00 | 1.00 | 6.00 | 7.00 | 0.00 | 1.00 |
| No. 19 | 77.00 | 137.00 | 64.00 | 87.00 | 79.00 | 60.00 | 60.00 | 24.00 | 25.00 |
| No. 20 | 51.00 | 104.00 | 82.00 | 17.00 | 11.00 | 57.00 | 35.00 | 12.00 | 10.00 |
| No. 27 | 41.00 | 4.00 | 15.00 | 24.00 | 3.00 | 77.00 | 66.00 | 9.00 | 7.00 |
| No. 32 | 126.00 | 64.00 | 53.00 | 143.00 | 115.00 | 99.00 | 86.00 | 42.00 | 16.00 |
| No. 33 | 46.00 | 24.00 | 56.00 | 29.00 | 22.00 | 26.00 | 21.00 | 10.00 | 20.00 |
| No. 35 | 182.00 | 60.00 | 21.00 | 28.00 | 32.00 | 71.00 | 75.00 | 25.00 | 17.00 |
| No. 39 | 91.00 | 144.00 | 79.00 | 81.00 | 75.00 | 84.00 | 114.00 | 13.00 | 43.00 |
| No. 41 | 66.00 | 14.00 | 46.00 | 40.00 | 35.00 | 22.00 | 26.00 | 0.00 | 0.00 |
| No. 44 | 95.00 | 51.00 | 108.00 | 120.00 | 78.00 | 48.00 | 59.00 | 6.00 | 28.00 |
| No. 45 | 71.00 | 37.00 | 28.00 | 24.00 | 47.00 | 51.00 | 21.00 | 8.00 | 3.00 |
| | | | | | | | | | |
| N | 15 | 15 | 15 | 15 | 15 | 15 | 15 | 15 | 15 |
| Mean | 78.07 | 53.53 | 41.07 | 46.87 | 36.20 | 49.73 | 45.67 | 11.60 | 12.93 |
| Sem | 9.49 | 11.16 | 8.23 | 10.59 | 9.08 | 6.54 | 7.68 | 2.95 | 3.17 |
| SD | 36.77 | 43.22 | 31.87 | 41.02 | 35.18 | 25.34 | 29.74 | 11.41 | 12.26 |

**Table 8C: Cylinder Test (Total Movement) Group III**

| **Group 3** | D-1 | D7 | D21 | D30 | D32 | D44 | D46 | D58 | D60 |
|---|---|---|---|---|---|---|---|---|---|
| | | | | | | | | | |
| No. 1 | 51.00 | 14.00 | 12.00 | 59.00 | 18.00 | 29.00 | 52.00 | 46.00 | 50.00 |
| No. 4 | 78.00 | 50.00 | 36.00 | 80.00 | 57.00 | 11.00 | 11.00 | 20.00 | 16.00 |
| No. 6 | 52.00 | 32.00 | 54.00 | 51.00 | 19.00 | 0.00 | 11.00 | 28.00 | 30.00 |
| No. 8 | 36.00 | 31.00 | 24.00 | 35.00 | 52.00 | 27.00 | 29.00 | 28.00 | 35.00 |
| No. 10 | 20.00 | 16.00 | 3.00 | 27.00 | 7.00 | 7.00 | 9.00 | 7.00 | 5.00 |
| No. 15 | 74.00 | 123.00 | 54.00 | 63.00 | 82.00 | 60.00 | 34.00 | 33.00 | 32.00 |
| No. 16 | 83.00 | 15.00 | 10.00 | 61.00 | 13.00 | 12.00 | 7.00 | 18.00 | 11.00 |
| No. 17 | 80.00 | 74.00 | 58.00 | 123.00 | 155.00 | 60.00 | 55.00 | 78.00 | 31.00 |
| No. 22 | 53.00 | 38.00 | 54.00 | 44.00 | 32.00 | 5.00 | 4.00 | 63.00 | 74.00 |
| No. 26 | 22.00 | 63.00 | 71.00 | 62.00 | 10.00 | 8.00 | 0.00 | 12.00 | 30.00 |
| No. 29 | 85.00 | 85.00 | 49.00 | 61.00 | 46.00 | 4.00 | 5.00 | 23.00 | 4.00 |
| No. 34 | 48.00 | 92.00 | 7.00 | 65.00 | 60.00 | 6.00 | 9.00 | 11.00 | 10.00 |
| No. 36 | 32.00 | 65.00 | 10.00 | 71.00 | 46.00 | 39.00 | 41.00 | 73.00 | 49.00 |
| No. 40 | 107.00 | 22.00 | 46.00 | 22.00 | 37.00 | 19.00 | 14.00 | 34.00 | 11.00 |
| No. 43 | 103.00 | 23.00 | 23.00 | 44.00 | 28.00 | 0.00 | 0.00 | 11.00 | 12.00 |
| | | | | | | | | | |
| N | 15 | 15 | 15 | 15 | 15 | 15 | 15 | 15 | 15 |
| Mean | 61.60 | 49.53 | 34.07 | 57.87 | 44.13 | 19.13 | 18.73 | 32.33 | 26.67 |
| Sem | 7.18 | 8.54 | 5.80 | 6.25 | 9.64 | 5.17 | 4.80 | 5.90 | 5.12 |
| SD | 27.82 | 33.06 | 22.46 | 24.22 | 37.32 | 20.03 | 18.58 | 22.86 | 19.83 |

### 6. EXAMPLES

### 6.1 Example 1: Single dose administration of 4-aminopyridine-SR

A set of patients are provided each of which has a form of cerebral palsy. These patients are provided with a single dose of the same amount of a sustained release aminopyridine.

### 6.2 Example 2: Single dose administration of 4-aminopyridine-SR along with matched control group

A set of patients are provided each of which has a form of cerebral palsy. These patients are provided with a single dose of the same amount of a sustained release aminopyridine.

As a matched control group, a set of patients are provided each of which has a form of cerebral palsy matched for meaningful parameters to the patient group in the previous paragraph. The matched control patients are provided with a single dose of a placebo.

### 6.3 Example 3: Single dose administration of 4-aminopyridine-SR in patients of same CP type, with control group

A set of patients are provided each of which has the same form of cerebral palsy. These patients are provided with a single dose of the same amount of a sustained release aminopyridine.

As a matched control group, a set of patients are provided each of which has the same form of cerebral palsy matched for meaningful parameters to the patient group in the previous paragraph. The matched control patients are provided with a single dose of a placebo.

### 6.4 Example 4: Single dose administration of 4-aminopyridine-SR in patients with various CP types, with control groups

A plurality of CP patient groups is provided. The patients in each respective CP patient group has the same type of CP as defined by a unique set of disease parameters, and the groups amongst the plurality of CP patient groups differ in their respective set of disease parameters. Each patient in these groups is provided with a single dose of the same amount of a sustained release aminopyridine. Types of CP patient groups can be selected from Spastic, Ataxic, Athetoid/dyskinetic, and Hypotonic.

Matched control groups are provided for each of the patient groups in the plurality of CP patient groups of the previous paragraph. Each control group has the same set of disease parameters as a patient group in the previous paragraph to which the particular control group is matched. The matched control patients are provided with a single dose of a placebo.

### 6.5 Example 5: A double-blind, placebo-controlled, crossover study in patients with CP to evaluate the effect on sensorimotor function of Dalfampridine

### 6.5.1 LIST OF ABBREVIATIONS

The following abbreviations and specialist terms are used in this study protocol (see Table 9).

**Table 9: Abbreviations and Specialist Terms**

| **Abbreviation or Specialist Term** | **Explanation** |
|---|---|
| AE | Adverse event |
| AST | Aspartate aminotransferase |
| BMI | Body mass index |
| BUN | Blood urea nitrogen |
| CFB | Change from baseline |
| CP | Cerebral palsy |
| CRF | Case Report Form |
| EEG | Electroencephalogram |
| ER | Extended release |
| FAP | Full Analysis Population |
| g | gram |
| GABA | Gamma-aminobutyric acid |
| GCP | Good Clinical Practice |
| HDPE | High density polyethylene |
| ICH | International Conference on Harmonization |
| IEC | Independent Ethics Committee |
| IND | Investigational New Drug |
| INN | International Nonproprietary Name |
| IRB | Institutional Review Board |
| LEMMT | Lower Extremity Manual Muscle Testing |
| MRI | Magnetic resonance imaging |
| MS | Multiple sclerosis |
| MSWS-12 | 12-item Multiple Sclerosis Walking Scale |
| PI | Principal Investigator |
| PPP | Per-Protocol Population |
| PVL | Periventricular leukomalacia |
| RBC | Red blood cell |
| SAE | Serious adverse event |
| T25FW | Timed 25 Foot Walk |
| USAN | United States Adopted Name |
| UTI | Urinary tract infection |
| WBC | White blood cell |

### 6.5.2 STUDY OBJECTIVES

The effects of dalfampridine-ER 10 mg (i.e., a sustained release formulation of 10 mg 4-aminopyridine) given as a single dose or b.i.d. (twice daily, approximately once every 12 hours) will be reported on the following clinical functions:
- Hand strength as measured by a composite Z-score derived from the grip test, and key, tip and palmar pinch tests
- Manual dexterity as measured by the Box and Block Test
- Walking speed as measured by the Timed 25 Foot Walk (T25FW)
- Gait as measured by the following parameters: stride length, cadence, single support time, and double support time. Stride length variability and swing time variability may also be assessed;
in order to provide an indication of the efficacy of dalfampridine-ER 10 mg given once or twice daily.

### 6.5.3 INVESTIGATIONAL PLAN

This is a multi-center double-blind, placebo-controlled, crossover study. The study will be divided into two parts, A and B, with different subjects enrolled into each part. In Part A, subjects will receive a single dose of investigational product; in Part B, subjects will receive multiple doses of investigational product.

For both parts of the study, eligibility will be determined at a screening visit (Visit 1) through a review of medical history, and results from physical examination, vital sign measurements, SMA-12 chemistry tests including calculated creatinine clearance, urinalysis, and a urine pregnancy test for women.

At screening, a battery of tests will be administered to determine manual dexterity, hand strength, and walking speed using the Box and Block, hand strength as measured by the grip test, tip pinch, key pinch and palmar pinch tests, and T25FW, respectively. If a subject meets eligibility requirements, the test which captures his or her most pronounced functional deficit as determined by a pre-specified algorithm will be assigned as the subject's key variable for analysis; all the other tests will become the subject's additional variables for analysis. An assessment of gait analysis will also be performed but will not be used to determine the subject's key functional variable.

### PART A (Cohort 1):

Within a week after the screening visit, qualified subjects in Cohort 1 will check into the clinic to be enrolled into study Period 1. On this study Day 1, Visit 2, subjects will be randomized 1:1 to one of two treatment sequences: AB (dalfampridine followed by placebo), or BA (placebo followed by dalfampridine). Subjects will undergo the full battery of tests to provide pre-dose baseline measurements. The tests will be administered by an evaluator who is blind to the Evaluator's assessment of the subject's most pronounced functional deficit at the screening visit. Each subject will then take a single witnessed dose of double-blind investigational product (dalfampridine or placebo tablet) with water. The first meal of the day will be provided about one half hour after dosing; water will be permitted ad lib. Box and Block, hand strength tests, and T25FW will be re-administered at three more time points at 3, 4, and 5 hours following the dose of investigational product, to correspond to approximate peak central nervous system concentration of dalfampridine. There will be a ±20 min window from each time point to administer the tests. Gait analysis will be performed only at pre-dose and at the 5-hour post-dose time point. Subjects will be discharged after completion of Day 1 study procedures.

There will be a no-treatment washout period (Day 2).

On Day 3, Visit 3, subjects will return to the clinic. Subjects will undergo the full battery of tests, prior to dosing. They will receive a single witnessed dose of their Period 2 (cross-over) treatment, followed by a meal. The battery of tests will be repeated at 3, 4, and 5 hours post-dose. Gait analysis will be performed only at pre-dose and at the last time point after dosing, as described for Day 1. Subjects will be discharged after completion of study procedures. Visit 3 will be the last clinic visit for Cohort 1.

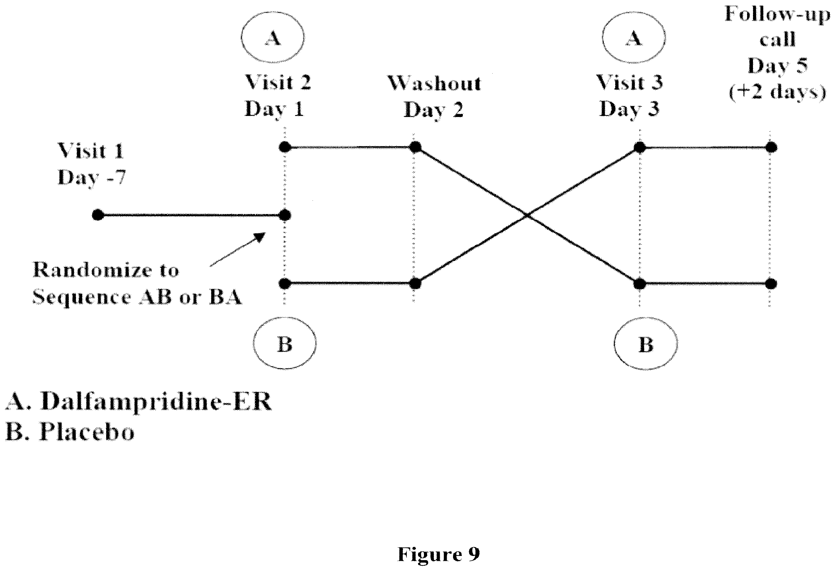

The study design for Part A is displayed graphically in Figure 9. The timing of assessments is presented in Table 10 and details of the study measurements and procedures can be found in Section 6.5.7.

**Table 10: Schedule of Assessments Part A (Cohort 1)**

| | | **Period 1** | | **Period 2** | |
|---|---|---|---|---|---|
| **Procedure** | **Screening** | **Treatment A or B** | **Wash-out** | **Treatment B or A** | **Follow-up call** |
| | **Visit 1** | **Visit 2** | | **Visit 3** | |
| | **Day -7 to Day -1** | **Day 1** | **Day 2** | **Day 3** | **Day 5 (+2 days)** |
| Written informed consent | X | | | | |
| Medical history | X | | | | |
| Concomitant meds/therapy | X | | | | X |
| Physical exam¹ | X | X | | X | |
| Vital signs² | X | X | | X | |
| SMA-12³ and creatinine clearance calculation | X | | | | |
| Urinal ysis | X | | | | |
| Urine pregnancy test | X | | | | |
| Adverse event review | X | X | | X | X |
| Box and Block ⁴ | X | X | | X | |
| Hand strength ^{4,5} | X | X | | X | |
| T25FW^{4,6} | X | X | | X | |
| Gait analysis⁷ | X | X | | X | |
| Evaluator Assessment | X | X | | X | |
| Randomize | | X | | | |
| Administer dose | | X | | X | |
| Discharge subject | | X | | X | |
| Final status assessment | | | | | X |

| | | | | | |
|---|---|---|---|---|---|
| ¹ Full physical only at screening. Brief physical on the treatment days: pre-dose and after the 5 hr (± 20 min) post-dose time point for functional assessments. ² Measured at 2 time points on the treatment days: pre-dose and after the 5 hr (± 20 min) post-dose time point for functional assessments. Height and weight only at Screening. ³ Labs can also be performed at other visits to follow up on an adverse event. ⁴ Administered at 4 time points on the treatment days: pre-dose, and post-dose hours 3, 4, and 5 (± 20 min). ⁵Three trials of the grip test, tip pinch, key pinch and palmar tests. ⁶ Two trials of the T25FWT. ⁷ Performed at 2 time points on the treatment days: pre-dose and 5 hr (± 20 min) post-dose. | | | | | |

The plan for Part B of the study, with a second cohort of new subjects, follows.

### PART B (Cohort 2):

Within a week after the screening visit, qualified subjects from Cohort 2 will check into the clinic to be enrolled into study Period 1. On this study Day 1, Visit 2, subjects will be randomized 1:1 to one of two treatment sequences: AB (dalfampridine then placebo) or BA (placebo then dalfampridine). Subjects will undergo the full battery of functional tests to provide pre-dose baseline measurements for Period 1. The tests will be administered by an evaluator who is blind to the Evaluator's assessment of the subject's most pronounced functional deficit at the screening visit. A single witnessed dose of double-blind investigational product (dalfampridine or placebo tablet) will be administered after the baseline assessments. The first meal of the day will be provided about one half hour after dosing; water will be permitted ad lib. Box and Block, hand strength, and T25FW tests will be re-administered three more times at 3, 4, and 5 hours following the dose of investigational product, to correspond to approximate peak central nervous system concentration of dalfampridine. For each time point, all the tests are to be done within a ± 20 minutes time window. Gait analysis will be performed only at pre-dose and the 5-hour post-dose time point. Subjects will be discharged after completion of Day 1 study procedures. They will receive a one-week supply of their assigned Period 1 treatment, with instructions to take one dose approximately every 12 hours at home, beginning the evening of Day 1 (next dose to be taken approximately 12 hours after the witnessed dose in the clinic).

Subjects will return to the clinic for Visit 3 on Day 8, after taking the last dose of their Period 1 treatment in the morning, no more than 2 hours prior to arrival. Subjects will undergo one set of the full battery of functional tests. Subjects will then be discharged with a supply of placebo, to begin a washout period of 6 days (Days 9-14), They will be instructed to take one dose of placebo approximately every 12 hours at home, beginning the evening of Day 8.

Subjects will return to the clinic for Visit 4 on Day 15, the start of Period 2. They will undergo the same schedule of baseline and post-dose assessments (including meal schedule), as described for Day 1. They will receive a single witnessed dose of their Period 2 (cross-over) treatment, per the sequence established at randomization. After completion of all post-dose assessments, subjects will be discharged with a one-week supply of their assigned treatment, to be taken at home, beginning the evening of Day 15 (next dose to be taken approximately 12 hours after the witnessed dose in the clinic).

Subjects will return to the clinic for Visit 5 on Day 22, after taking the last dose of their Period 2 treatment in the morning, no more than 2 hours prior to arrival. Subjects will undergo one set of the full battery of functional assessments. Subjects will then be discharged, and Visit 5 will be the last clinic visit for the Cohort 2.

Cohort 2 will be instructed to have a dosing schedule which results, approximately, in a morning dose of investigational product being taken no more than 2 hours prior to arriving at the clinic for study assessments at Visits 3 and 5.

A blood sample will be obtained for pharmacokinetic (PK) analysis of study drug concentration at the beginning and end of Periods 1 and 2.

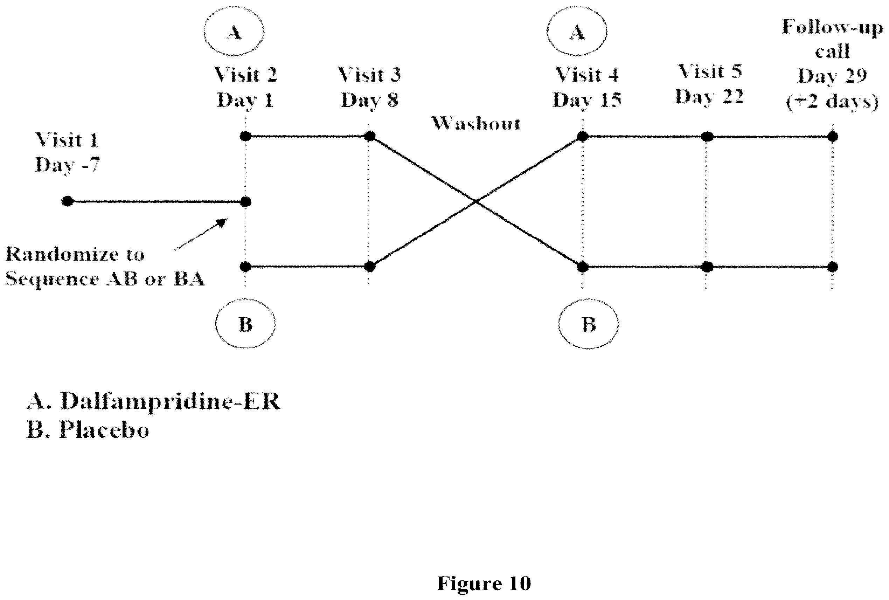

The study design for Part B is displayed graphically in Figure 10. The timing of assessments is presented in Table 11 and details of the study measurements and procedures can be found in Section 6.5.7.

**Table 11: Schedule of Assessments Part B (Cohort 2)**

| | | **Period 1** | | **Wash-out** | **Period 2** | | |
|---|---|---|---|---|---|---|---|
| **Procedure** | **Screening** | **Treatment A or B** | | **Placebo** | **Treatment B or A** | | **Follow-up call** |
| | **Visit 1** | **Visit 2** | **Visit 3** | | **Visit 4** | **Visit 5** | |
| | **Day -7 to Day -1** | **Day 1** | **Day 8¹** | | **Day 15** | **Day 22¹** | **Day 29 (±2 days)** |
| Written informed consent | X | | | | | | |
| Medical history | X | | | | | | |
| Concomitant | X | | | | | | X |
| meds/therapy | | | | | | | |
| Physical exam | X² | X³ | X⁴ | | X³ | X⁴ | |
| Vital signs | X⁵ | X³ | X⁴ | | X³ | X⁴ | |
| SMA-12^{6,7} and creatinine clearance calculation | X | | | | | | |
| PK plasma sample⁷ | | X | X | | X | X | |
| Urinalysis⁷ | X | | | | | | |
| Urine pregnancy test⁷ | X | | | | | | |
| Adverse event review | X | X | X | | X | X | X |
| Box and Block | X | X⁸ | X | | X⁸ | X | |
| Hand strength ⁹ | X | X⁸ | X | | X⁸ | X | |
| T25FW ¹⁰ | X | X⁸ | X | | X⁸ | X | |
| Gait analysis | X | X¹¹ | X | | X¹¹ | X | |
| Evaluator Assessment | X | X | X | | X | X | |
| Randomize | | X | | | | | |
| Administer dose | | X | | | X | | |
| Dispense 1-wk supply of investigational product | | X | X | | X | | |
| Discharge subject | | X | X | | X | X | |
| Final status assessment | | | | | | | X |

| | | | | | | | |
|---|---|---|---|---|---|---|---|
| ¹All measurements will be performed one time after the last dose of Period 1 treatment on Day 8 and last dose of Period 2 treatment on Day 22. ² Full physical only at screening; brief physical at subsequent visits. ³Measured at 2 time points: pre-dose (after functional assessments) and after the 5 hr (± 20 min) post-dose time point for functional assessments. ⁴Measured after functional assessments. ⁵Height and weight only at Screening. ⁶Labs can also be performed at other visits to follow up on an adverse event. ⁷All sampling is to be drawn after the functional assessments have been performed ⁸Administered at 4 time points: pre-dose, and post-dose hours 3, 4, and 5 (± 20 min) ⁹Three trials of the grip test, tip pinch, key pinch and palmar tests. ¹⁰Two trials of the T25FWT. ¹¹Performed at 2 time points: pre-dose and 5 hr (± 20 min) post-dose. | | | | | | | |

Twelve (12) subjects are expected to be randomized in Part A in order to ensure that approximately 5 subjects will complete each sequence. Twenty-five (25) subjects are expected to be randomized in Part B in order to ensure that approximately 10 subjects will complete each sequence.

### 6.5.4 SELECTION AND WITHDRAWAL OF SUBJECTS

### (a) Inclusion Criteria

Subjects must fulfill all of the inclusion criteria listed below to be eligible for participation in the study:
- A diagnosis of CP
- Man or woman 18 to 55 years of age, inclusive
- Body mass index (BMI) ranging between 18.0 - 25.0 kg/m,² inclusive
- No previous use of any dalfampridine formulation
- Mental competence to understand and sign the IRB-approved informed consent prior to the performance of any study-specific procedures, as determined by the Evaluator
- Ability to perform all the required study procedures. Subjects be capable of fully extending and flexing both hands.

### (b) Exclusion Criteria

Subjects who meet any of the following exclusion criteria are not eligible for participation in the study:
- Presence of any progressive neurological disease
- Severe CP defined as the requirement to use a wheelchair at all times and a care taker for constant assistance in daily activities. This definition includes spastic quadriplegia.
- Sexually active women of childbearing potential defined as: not surgically sterile or < two years postmenopause who is not using effective birth control methods
- Pregnant or breastfeeding
- History of seizures, excluding simple febrile seizure, which by definition is a single, generalized seizure of less than 15 minutes of duration occurring between the ages of 6 months to 5 years.
- Moderate or severe renal impairment as defined by a calculated creatinine clearance of ≤ 50 mL/minute using the Cockcroft-Gault Equation
- Presence of active urinary tract infection (UTI) at the Screening Visit or within the 4 weeks prior to the Screening Visit
- Initiation of concomitant prescription medication regimen or therapy within the 3 weeks prior to the Screening Visit
- Initiation of baclofen, tizanidine or any other antispasticity medications, or any change in the dosing regimen of these drugs, within the 30 days prior to the Screening Visit
- Botulinum toxin administered within two months prior to the Screening Visit
- History of drug or alcohol abuse within the past year
- An abnormal laboratory value at the Screening Visit that, in the Evaluator's judgment, is both clinically significant and has the potential to affect the subject's ability to safely complete the study
- Any medical condition that would interfere with conduct of study or interpretation of the study results
- Participation in an investigational trial within 30 days prior to the Screening Visit.

### (c) Withdrawal Criteria

The withdrawal criteria, which are optional, include one or more of the following reasons:Subject experiences an adverse event
- Pregnancy
- Subject is non-compliant with the protocol
- Subject is lost to follow-up
- Evaluator decision, which may include: subject abuses alcohol or drugs or no longer meets another eligibility criterion, to the extent that, in the judgment of the Evaluator, it would affect assessments of clinical status to a significant degree, require discontinuation of the investigational product, or both.

### 6.5.5 TREATMENT OF SUBJECTS

### (a) Treatments to be Administered

### PART A (Cohort 1):

Each subject will receive a single witnessed dose of (A) dalfampridine-ER 10 mg, and a single witnessed dose of (B) placebo. The tablets will be taken with water. The order of treatment will be determined as described below in Section 6.5.5(b).

### PART B (Cohort 2):

Each subject will receive 14 doses of (A) dalfampridine-ER 10 mg, and 28 doses of (B) placebo (including 14 doses to be taken during the placebo washout period). The tablets will be taken at with water. The order of treatment will be determined as described below in Section 6.5.5(b).

At Visit 2 (start of Period 1) and Visit 4 (start of Period 4), the first dose of investigational product will be a witnessed dose in the clinic. A bottle containing a one-week supply of investigational product (14 tablets plus 2 extra tablets) will also be dispensed to the subject at these visits after assessments have been completed. Subjects will be instructed to take the first dose from the bottle in the evening of the visit, approximately 12 hours after the witnessed dose in the clinic, and the next dose the following morning, approximately 12 hours later. Subjects will be instructed to continue dosing every 12 hours at times that are as consistent as possible. Subjects will be told that they must not make up for missed doses.

Visit 3 and Visit 5 will be scheduled to occur no more than 2 hours after the last (morning) dose of investigational product is expected to be taken. At Visit 3, the start of the washout period, subjects will receive a bottle containing a one-week supply (14 tablets plus 2 extra) of placebo, with the same instructions to take a dose approximately every 12 hours beginning the evening of Visit 3.

### (b) Method of Assigning Subject to Treatment Group

Subjects will be randomized at Visit 1 to one of two treatment sequences in a 1:1 ratio according to a randomization created prior to the start of the study:
AB: Dalfampridine then Placebo
BA: Placebo then Dalfampridine

### (c) Blinding

Drug administration for Periods 1 and 2 will be double-blind, meaning that the treatment sequence is not known to the subject or the study site personnel.

The washout period will be single-blind, meaning that the study site personnel, but not the subject, will know that placebo is being administered during this period.

### (d) Treatment Compliance

In Parts A and B, the time of administration of the witnessed dose in the source document will be recorded. In Part B, subjects will be encouraged to take all doses at home as prescribed. Any reasons for non-compliance will be documented.

### (e) Prior and Concomitant Medications

The following medications are excluded for the duration of the study, and also for some period of time prior to the study, for the purpose of maintaining stable symptoms:
- Baclofen, tizanidine or any other antispasticity medications initiated or dosing modified within less than 30 days before the Screening Visit
- Botulinum toxin administered fewer than two months before the Screening Visit
- Other prescription medications or therapies initiated or changed fewer than 3 weeks before the Screening Visit

No changes will be made to the concomitant treatment during the study, except as required for the safety of the subject.

### 6.5.6 DESCRIPTION OF INVESTIGATIONAL PRODUCT

*Active*: Dalfampridine-ER will be supplied as unmarked, film coated, white to off-white, biconvex, oval-shaped, non-scored tablets with a flat edge, containing 10 mg of dalfampridine. Inactive ingredients consist of colloidal silicon dioxide, hydroxypropyl methylcellulose, magnesium stearate, microcrystalline cellulose, polyethylene glycol, and titanium dioxide.

*Placebo*: The placebo tablets will be identical in appearance to the dalfampridine tablets and contain the same inactive ingredients.

### 6.5.7 STUDY PROCEDURES

The following sections describe the baseline and clinical functional measurements that will be obtained in this study. A detailed schedule of procedures by study visit is provided in Section 6.5.7(c) and summarized in Table 10 for Part A and Table 11 for Part B.

Prior to engaging in any study procedure, subjects must provide written informed consent.

### (a) Plasma Dalfampridine Concentration

For Part B, only, blood samples for determination of plasma dalfampridine concentration will be obtained Visits 2, 3, 4, and 5. At each of these visits, a single blood sample will be obtained after all sets of functional assessments have been performed.

A minimum of 7 mL of whole blood will be collected into an appropriately labeled heparin tube and kept cold (i.e., on wet ice) until centrifuged. Immediately after collection, the tube will be centrifuged at low speed and approximately 3 mL of plasma will be transferred from each sample into a labeled tube. The plasma will be stored at -20° C until shipment to the central laboratory is requested. At that time, frozen plasma samples will be collected together and sent in an insulated container, on dry ice, overnight by express carrier to the designated central laboratory.

### (b) Functional Assessments

This section describes the clinical assessments of CP to be used in this study.

For a given subject, the Evaluator administering the T25FW, Box and Block, and Grip and Pinch tests at the screening visit (Visit 1) will not be the same person who administers these tests at subsequent visits. For a given subject, evaluations at all subsequent visits will be performed by the same person (different from the person performing the evaluations at screening).

### Timed 25 Foot Walk (T25FW)

The T25FW test is a quantitative measure of ambulatory function. The subject is instructed to walk as quickly as he or she can from one end to the other end of a clearly marked, unobstructed, 25-foot course. The T25FW will be performed according to the detailed instructions provided in the Administration and Scoring Manual published by the National Multiple Sclerosis Society (Fischer J, et al. The Multiple Sclerosis Functional Composite Administration and Scoring Manual. National Multiple Sclerosis Society. 2001; 1-41). The instruction booklet will be provided to study centers, and is summarized here. The subject will stand with the tip of their shoes on a marked starting line, and timing will begin when any part of the subject's foot crosses the line. Timing will end when any part of the subject's foot crosses the marked finish line. Time will be recorded in seconds and rounded to the nearest tenth of a second using a stopwatch provided for this study. The task is administered again, with a maximum five-minute rest period allowed between trials, by having the subject walk back the same distance. If required, the subject may use an appropriate assistive device. The subject will be instructed to maintain his or her normal activities without rehearsal or practice measures to unfairly improve their performance scores between visits. Every effort will be made to use the same testing room and the same designated area for the T25FW at each assessment. Potential for external distractions will be kept to a minimum as much as possible.

Normative data for walking speed are available (Bohannon, 1997, Age Ageing 26: 15-19). For subjects ≥18 years of age and <20 years of age, the normative data for the 20s decade age group will be used.

### Box and Block

The Box and Block test (Mathiowetz et al., 1985, Am J Occup Ther 36: 386-391) has been used as a valid and reliable measure of manual dexterity. The subject is instructed to quickly pick up blocks one at a time from one side of a box, transport each block over a partition to the other side of the box, and drop it. Specific instructions will be provided in the Site Instruction Manual. The test was originally developed to evaluate the gross manual dexterity of adults with cerebral palsy. Data for normal adults are available (see Bohannon, 1997, Age Ageing 26:15-19). For subjects ≥18 years of age and <20 years of age, the normative data for the 20-24 years age group will be used.

The Box and Block test will be consistently performed before the Pinch and Grip tests to minimize the effects of fatigue.

### Hand Strength by the Grip and Pinch tests

The Grip Test (Mathiowetz et al., 1985, Arch Phys Med Rehabil 66: 69-72) is used as a simple, valid and reliable measure to identify hand strength problems, to detect the change which may result from an occupational therapy program, the course of a disease or injury, or to show the relation of the patient's strength to the general population. Hand strength is measured using a dynamometer.

The Pinch Tests (Mathiowetz et al., 1985, Arch Phys Med Rehabil 66: 69-72) are used as a simple, valid and reliable measure to indentify pinch strength problems, to detect the change which may result from an occupational therapy program, the course of a disease or injury, or to show the relation of the patient's strength to the general population. It has three components, the tip, key and palmar pinch. Pinch strength is measured using a pinch gauge.

Normative data on the Grip and Pinch tests in adults are available (see Mathiowetz et al., 1985, Arch Phys Med Rehabil 66: 69-72). For subjects ≥18 years of age and <20 years of age, the normative data for the 20-24 years age group will be used.

### Gait Analysis

The parameters of interest for gait analysis are; stride length, cadence, single support time, and double support time. Stride length variability and swing time variability may also be assessed.

### Evaluator Assessment

The following question will be answered about each subject after administration of the battery of tests at the Screening Visit: Which test, according to the evaluator's assessment, captures the subject's most pronounced functional deficit?
A. Box and Block
B. Grip and Pinch Tests
C. T25FW

The following question will be answered about each subject after last administration of the battery of tests on dosing days: On which test, according to evaluator's assessment, does the subject show the most improvement?
A. Box and Block
B. Grip and Pinch Tests
C. T25FW
D. None

### (c) Study Sequence

### Part A (Cohort 1):

The following sections describe the assessments to be performed at each clinic visit during Part A of the study.

### Section 1: Day -7 to Day -1 (Visit 1, Screening)

The eligibility will be assessed after the following procedures have been performed in the order presented:
- Obtain signed informed consent
- Assign subject number
- Obtain medical history, demographic information, and CP history including date of initial diagnosis and current clinical presentation
- Record all concomitant medications and therapies taken within three weeks of this visit
- Complete a full physical examination
- Obtain sitting vital sign measurements and height and weight
- Take blood and urine samples for laboratory evaluations (SMA-12, calculated creatinine clearance, urinalysis, and urine pregnancy test for women of childbearing potential).
- Administer Box and Block test (dominant and non-dominant hand, the dominant first)
- Administer grip test, tip pinch, key pinch and palmar pinch tests. These tests will be administered three times with each hand.
- Administer two trials of the T25FW
- Perform gait analysis
- Complete Evaluator Assessment
- Review adverse events
- Discharge subject and schedule a date and time for the next visit to occur within one week

### Section 2: Day 1, Visit 2 (Period 1)

The following assessments and procedures will be performed on Day 1:

### Pre-dose

- Box and Block test (dominant and non-dominant hand, the dominant first)
- Grip test, tip pinch, key pinch and palmar pinch tests. These tests will be administered three times with each hand.
- Two trials of the T25FW
- Gait analysis
- Brief physical examination
- Sitting vital sign measurements
- Review adverse events
- Randomize to one of two treatment sequences

### Administer single dose of Period 1 double-blind investigational drug

### Post-dose

- Provide meal approximately 1/2 hour after dosing
- Box and Block test at 3 hr (±20 min), 4 hr (±20 min), and 5 hr (±20 min) (dominant and non-dominant hand, the dominant first)
- Grip test, tip pinch, key pinch and palmar pinch tests at 3 hr (±20 min), 4 hr (±20 min), and 5 hr (±20 min). Each test will be administered three times per each hand at each time point.
- Two trials of the T25FW test at 3 hr (±20 min), 4 hr (±20 min), and 5 hr (±20 min)
- Gait analysis at 5 hr (±20 min)
- Brief physical examination after the 5 hr (± 20 min) post-dose time point for the functional assessments.
- Sitting vital sign measurements after the 5 hr (± 20 min) post-dose time point for the functional assessments.
- Complete Evaluator Assessment
- Review adverse events
- Discharge subject after completing all 5-hour post-dose procedures

### Section 3: Day 2 (Washout)

There will be no study assessments on Day 2.

### Section 4: Day 3, Visit 3 (Period 2)

The following assessments and procedures will be performed on Day 3:

### Pre-dose

- Box and Block test (dominant and non-dominant hand, the dominant first)
- Grip test, tip pinch, key pinch and palmar pinch tests. Each test will be administered three times with each hand.
- Two trials of the T25FW
- Gait analysis
- Brief physical examination
- Sitting vital sign measurements
- Review adverse events

### Administer single dose of Period 2 double-blind investigational drug

### Post-dose

- Provide meal approximately 1/2 hour after dosing
- Box and Block at 3 hr (±20 min), 4 hr (±20 min), and 5 hr (±20 min) (dominant and non-dominant hand, the dominant first)
- Grip test, tip pinch, key pinch and palmar pinch tests at 3 hr (±20 min), 4 hr (±20 min), and 5 hr (±20 min). Each test will be administered three times per each hand at each time point.
- Two trials of the T25FW test at 3 hr (±20 min), 4 hr (±20 min), and 5 hr (±20 min)
- Gait analysis at 5 hr (±20 min)
- Brief physical examination after the 5 hr (± 20 min) post-dose time point for the functional assessments
- Sitting vital sign measurements after the 5 hr (± 20 min) post-dose time point for the functional assessments
- Complete Evaluator Assessment
- Review adverse events
- Discharge subject after completing all 5-hour post-dose procedures

### Section 5: Day 5 (+ 3 days) (Telephone Follow-up)

The site will make a follow-up telephone call to review any adverse events or changes in medication. The follow-up call may be performed up to 3 days after Day 5 to account for the weekend and/or holidays.
- Final status assessment

### Part B (Cohort 2):

The following sections describe the assessments to be performed at each clinic visit during Part B of the study.

### Section 1: Day -7 to Day -1 (Visit 1, Screening)

The eligibility will be assessed after the following procedures have been performed in the order presented:
- Obtain signed informed consent
- Assign subject number
- Obtain medical history, demographic information, and CP history including date of initial diagnosis and current clinical presentation
- Record all concomitant medications and therapies taken within three weeks of this visit
- Complete a full physical examination
- Obtain sitting vital sign measurements and height and weight
- Take blood and urine samples for laboratory evaluations (SMA-12, calculated creatinine clearance, urinalysis, and urine pregnancy test for women of childbearing potential).
- Administer Box and Block test (dominant and non-dominant hand, the dominant first)
- Administer grip test, tip pinch, key pinch and palmar pinch tests. Each test will be administered three times with each hand.
- Administer two trials of the T25FW
- Perform gait analysis
- Complete Evaluator Assessment
- Review adverse events
- Discharge subject and schedule a date and time for the next visit to occur within one week

### Section 2: Day 1, Visit 2 (start of Period 1)

The following assessments and procedures will be performed on Day 1:

### Pre-dose

- Box and Block test (dominant and non-dominant hand, the dominant first)
- Grip test, tip pinch, key pinch and palmar pinch tests. Each test will be administered three times with each hand.
- Two trials of the T25FW
- Gait analysis
- Brief physical examination
- Sitting vital sign measurements
- Evaluator Assessment
- Review adverse events
- Randomize to one of two treatment sequences

### Administer single dose of Period 1 double-blind investigational drug

### Post-dose

- Provide meal approximately 1/2 hour after dosing
- Box and Block test at 3 hr (±20 min), 4 hr (±20 min), and 5 hr (±20 min) (dominant and non-dominant hand, the dominant first)
- Grip test, tip pinch, key pinch and palmar pinch tests at 3 hr (±20 min), 4 hr (±20 min), and 5 hr (±20 min). Each test will be administered three times per each hand at each time point.
- Two trials of the T25FW test at 3 hr (±20 min), 4 hr (±20 min), and 5 hr (±20 min)
- Gait analysis at 5 hr (±20 min)
- Brief physical examination after the 5 hr (± 20 min) post-dose time point for the functional assessments.
- Sitting vital sign measurements after the 5 hr (± 20 min) post-dose time point for the functional assessments.
- Complete Evaluator Assessment
- Review adverse events
- Dispense a one-week supply of Period 1 double-blind investigational product with instructions to take the next dose that evening, approximately 12 hours after time of first dose
- Discharge subject and schedule a date and time for the next visit to occur in one week

### Section 3: Day 8, Visit 3 (end of Period 1, start of washout)

The following assessments and procedures will be performed on Day 8. Assessments will be performed no more than 2 hours after morning dose of investigational product.
- Box and Block (dominant and non-dominant hand, the dominant first)
- Grip test, tip pinch, key pinch and palmar pinch tests. Each test will be administered three times with each hand.
- Two trials of the T25FW test
- Gait analysis
- Brief physical examination after the functional assessments
- Sitting vital sign measurements after the functional assessments
- Evaluator Assessment
- Review adverse events
- Perform investigational product accountability
- Dispense a one-week supply of placebo with instructions to take first dose that evening at approximately the same time as the evening doses taken during Period 1.
- Discharge subject and schedule a date and time for the next visit to occur in one week

### Section 4: Day 15, Visit 4 (end of washout, start of Period 2)

The following assessments and procedures will be performed on Day 15:

### Pre-dose

- Box and Block test (dominant and non-dominant hand, the dominant first)
- Grip test, tip pinch, key pinch and palmar pinch tests. Each test will be administered three times with each hand.
- Two trials of the T25FW
- Gait analysis
- Brief physical examination
- Sitting vital sign measurements
- Evaluator Assessment
- Review adverse events

### Administer single dose of Period 2 double-blind investigational drug

### Post-dose

- Provide meal approximately 1/2 hour after dosing
- Box and Block test at 3 hr (±20 min), 4 hr (±20 min), and 5 hr (±20 min) (dominant and non-dominant hand, the dominant first)
- Grip test, tip pinch, key pinch and palmar pinch tests at 3 hr (±20 min), 4 hr (±20 min), and 5 hr (±20 min). Each test will be administered three times per each hand at each time point
- Two trials of the T25FW test at 3 hr (±20 min), 4 hr (±20 min), and 5 hr (±20 min)
- Gait analysis at 5 hr (±20 min)
- Brief physical examination after the 5 hr (± 20 min) post-dose time point for the functional assessments.
- Sitting vital sign measurements after the 5 hr (± 20 min) post-dose time point for the functional assessments.
- Complete Evaluator Assessment
- Review adverse events
- Perform investigational product accountability
- Dispense a one-week supply of Period 2 double-blind investigational product with instructions to take the next dose that evening, approximately 12 hours after time of first dose
- Discharge subject and schedule a date and time for the next visit to occur in one week

### Section 5: Day 22, Visit 5 (end of Period 2)

The following assessments and procedures will be performed on Day 22. Assessments will be performed no more than 2 hours after morning dose of investigational product.
- Box and Block (dominant and non-dominant hand, the dominant first)
- Grip test, tip pinch, key pinch and palmar pinch tests. Each test will be administered three times with each hand.
- Two trials of the T25FW test
- Gait analysis
- Brief physical examination after the functional assessments
- Sitting vital sign measurements after the functional assessments
- Evaluator Assessment
- Review adverse events
- Perform investigational product accountability
- Discharge subject and schedule a date and time for a follow-up phone call to occur in one week (± 2 days)

### Section 6: Day 29 (Telephone Follow-up)

The site will make a follow-up telephone call to review any adverse events or changes in medication.
- Final status assessment

### 6.5.8 ANALYSIS OF FUNCTIONAL ASSESSMENTS

### (a) Key Functional Variable

At screening, a battery of tests will be administered to each subject, which includes Box and Block, hand strength as measured by the grip strength, tip pinch, key pinch and palmar pinch tests, and T25FW.

Based on an algorithm to be specified in the SAP, each subject's most pronounced functional deficit based on the deviation from the norm among the Box and Block, hand strength and T25FW assessments will be determined, and will be identified as the key functional variable for that subject. For hand strength and dexterity tests, the screening assessments from dominant hands will be used in the determination. Since the assigned key functional variable may differ across subjects, it will be standardized using Z score prior to the analysis.

### (b) Additional Functional Variables

As stated in Section (a), one of Box and Block, hand strength, and T25FW tests will be determined as the key functional variable for each individual subject; the other tests will be considered as additional variables for that subject. The T25FW, Box and Block, and hand strength component (grip strength, tip pinch, key pinch and palmar pinch tests) variables based on the original scales will be analyzed, and the hand strength variable based on its combined standardized components will also be analyzed. The hand strength and dexterity assessments (i.e., Box and Block, hand strength and its components) will be analyzed using data from both the dominant and non-dominant hand. Also, the test assessed by the evaluator as the one for which the subject shows the most improvement will be analyzed.

Gait parameters will also be analyzed.

### (c) Derived Variables and Data Handling

All available data from the FAP or PPP subjects will be included in the corresponding analysis; missing data will not be imputed.

All functional assessments will be performed at the screening visit. On treatment days, Box and Block, hand strength, and T25FW tests will be administered at 4 time points (pre-dose, and post-dose 3 hours, 4 hours, and 5 hours), while the gait assessment will be administered at 2 time points (pre-dose and 5 hours post-dose).

At each time point at screening and treatment days, two trials will be administered for the T25FW test. Walking speed of an individual trial will be derived by multiplying 25 feet and the reciprocal of the time (in seconds) used to complete the walk. The average walking speed of a particular time point will then be derived by calculating the average of Trial 1 and Trial 2. If either trial is missed, the walking speed from the non-missing trial will be used for that time point.

The hand strength assessments include tip pinch, key pinch, palmar pinch and grip strength. At each time point, the average result of three trials from each of these four measurements will be calculated for each hand. Each of the four averages will then be converted to a Z-score, and the sum of these Z-scores will be used as a single, aggregate assessment of hand strength. The Z-scores for each of the four averages will be based on the mean and standard deviation of the averages, calculated using all the subjects within each time point and within each period.

Within each treatment period, the baseline value of a particular functional variable will be the pre-dose assessment on the treatment day. Changes from baseline will then be calculated for each derived functional variable at each post-dose analysis time point. An average post-treatment value will be derived by calculating the average of the post-dose changes from baseline at 3 hours, 4 hours, and 5 hours on the treatment days (except for the gait parameters, which are assessed only at 5 hours post-dose on the treatment days).

Each of the baseline values and changes from baseline for the Box and Block, hand strength, and T25FW variables will be converted to Z-scores, based on the mean and standard deviation of these baseline and change from baseline values, calculated using all the subjects within each time point and within each period. The Z-scores corresponding to each subject's key functional test will be the subject's key functional variable for analysis.

### (d) Statistical Methods

Since the assigned key variable will differ across subjects, it will be standardized using Z score prior to the analysis (as will all functional variables). Additional analyses will also be performed, based on their original scales for each functional variable. Gait will also be analyzed.

Differences between the treatments on each of the functional variables will be assessed by comparing the period differences (Period 2 - Period 1) across the two sequence groups, using the change from baseline values. This controls for any period effects.

Hand tests including hand strength and Box and Block will be analyzed by hand (dominant and non-dominant).

One analysis of the functional assessments will be based upon an adaptation of the Brown-Mood median test (Senn S. In: Cross-over Trials in Clinical Research, 2nd edition, LTD. John Wiley & Sons. 2002). Within each variable and time point, each of the period differences will be classified as being above or below the median period difference. The treatment effect will be assessed by comparing these classifications across the sequence groups via Fisher's exact test. In an additional analysis, the period differences will be compared across the sequence groups via a two-sample t-test. This test is equivalent to an analysis of variance with effects for subject, period and treatment. If the usual parametric assumptions are grossly violated, then the sequences will be compared via the Wilcoxon rank-sum test. Within each period, the distributions of the evaluator's assessment of the test as the one for which the subject shows the most improvement will be compared across the treatment groups and the equality of the distributions will be tested via Fisher's Exact test. In addition, the test assessed as the one for which each subject displays the most pronounced deficit will be cross-classified according to the treatment received.

Additional sensitivity analyses will be performed, which will be detailed in the SAP.

### 6.5.9 REFERENCES FOR EXAMPLE 5

- Lin JP. The cerebral palsies: a physiological approach. J Neurol Neurosurg Psychiatry. 2003;74(Suppl 1):i23-i29.
- Krigger KW. Cerebral palsy: an overview. Am Fam Physician. 2006;73(1):91-100.
- Odding E, Roebroeck ME, Stam HJ. The epidemiology of cerebral palsy: incidence, impairments and risk factors. Disabil Rehabil. 2006;28(4):183-191.
- Koman LA, Smith BP, Shilt JS. Cerebral palsy. Lancet. 2004;363(9421):1619-1631.
- Carlsson M, Hagberg G, Olsson I. Clinical and aetiological aspects of epilepsy in children with cerebral palsy. Dev Med Child Neurol. 2003;45(6):371-376.
- Humphreys P, Deonandan R, Whiting S, et al. Factors associated with epilepsy in children with periventricular leukomalacia. J Child Neurol. 2007;22(5):598-605.
- Zelnik N, Konopnicki M, Bennett-Back O, Castel-Deutsch T, Tirosh E. Risk factors for epilepsy in children with cerebral palsy. Eur J Paediatr Neurol. 2010;14(1):67-72.
- Johnston MV, Hoon AH Jr. Cerebral palsy. Neuromolecular Med. 2006;8(4):435-450.
- Bax M, Tydeman C, Flodmark O. Clinical and MRI correlates of cerebral palsy: the European Cerebral Palsy Study. JAMA. 2006;296(13):1602-1608.
- Folkerth RD. Neuropathologic substrate of cerebral palsy. J. Child Neurol. 2005;20(12):940-949.
- Wu YW, Croen LA, Shah SJ, Newman TB, Najjar DV. Cerebral palsy in a term population: risk factors and neuroimaging findings. Pediatrics. 2006;118(2):690-697.
- Mikkola K, Ritari N, Tommiska V, et al. Neurodevelopmental outcome at 5 years of age of a national cohort of extremely low birth weight infants who were born in 1996-1997. Pediatrics. 2005; 116(6):1391-1400.
- Robertson CM, Watt MJ, Yasui Y. Changes in the prevalence of cerebral palsy for children born very prematurely within a population-based program over 30 years. JAMA. 2007;297(24):2733-2740.
- Beaino G, Khoshnood B, Kaminski M, et al. Predictors of cerebral palsy in very preterm infants: the EPIPAGE prospective population-based cohort study. Dev Med Child Neurol. 2010;52(6):e119-e125.
- O'Shea TM, Preisser JS, Klinepeter KL, Dillard RG. Trends in mortality and cerebral palsy in a geographically based cohort of very low birth weight neonates born between 1982 to 1994. Pediatrics. 1998;101(4 Pt 1):642-647.
- Strauss D, Shavelle R. Life expectancy of adults with cerebral palsy. Dev Med Child Neurol. 1998;40(6):369-375.
- Hemming K, Hutton JL, Pharoah PO. Long-term survival for a cohort of adults with cerebral palsy. Dev Med Child Neurol. 2006;48(2):90-95.
- Cerebral Palsy: Hope through research: National Institute of Neurological Disorders and Stroke (NINDS). Accessed online April 20, 2011, at: http://www.ninds.nih.gov/disorders/cerebral_palsy/detail_cerebral_palsy.htm#154443104
- Rapp CE, Torres M. The adult with cerebral palsy. Arch Fam Med. 2000;9:466-472
- Volpe JJ. Neurobiology of periventricular leukomalacia in the premature infant. Pediatric Res. 2001;50(5):553-562.
- Back SA, Riddle A, McClure MM. Maturation-dependent vulnerability of perinatal white matter in premature birth. Stroke. 2007;38(2 Suppl):724-730.
- Deng W, Pleasure J, Pleasure D. Progress in periventricular leukomalacia. Arch Neurol. 2008;65(10):1291-1295.
- Thomas B, Eyssen M, Peeters R, et al. Quantitative diffusion tensor imaging in cerebral palsy due to periventricular white matter injury. Brain. 2005;128(Pt 11):2562-2577.
- Hoon AH Jr, Stashinko EE, Nagae LM, et al. Sensory and motor deficits in children with cerebral palsy born preterm correlate with diffusion tensor imaging abnormalities in thalamocortical pathways. Dev Med Child Neurol. 2009;51(9):697-704.
- Beckung E, Hagberg G, Uldall P, Cans C. Surveillance of Cerebral Palsy in Europe. Probability of walking in children with cerebral palsy in Europe. Pediatrics. 2008;121(1):e187-e192.
- Strauss D, Ojdana K, Shavelle R, Rosenbloom L. Decline in function and life expectancy of older persons with cerebral palsy. NeuroRehabilitation. 2004;19(1):69-78.
- Goodman AD, Brown TR, Cohen JA, et al. Dose comparison trial of sustained-release fampridine in multiple sclerosis. Neurology. 2008;71:1134-1141.
- Goodman AD, Brown TR, Krupp LB, et al. Sustained-release oral fampridine in multiple sclerosis: a randomized, double-blind, controlled trial. Lancet. 2009;373: 732-38.
- Goodman AD, Brown TR, Edwards KR, et al. A Phase 3 trial of extended release oral dalfampridine in multiple sclerosis. Ann Neurol. 2010; 373: 494-502.
- Stolp HB, Dziegielewaka KM. Review: Role of developmental inflammation and blood-brain barrier dysfunction in neurodevelopmental and neurodegenerative diseases. Neuropathol Appl Neurobiol. 2009; 35:132-146.
- Aksu F. Nature and prognosis of seizures in patients with cerebral palsy. Dev Med Clin Neurol. 1990;32:661-668.
- McDermott S, Moran R, Platt T, et al. Prevalence of epilepsy in adults with mental retardation and related disabilities in primary care. Am J Ment Retard. 2005;110:48-56.
- Ashwal S, Russman RS, Blasco PA, et al. Practice parameter: Diagnostic assessment of the child with cerebral palsy: report of the Quality Standards Subcommittee of the American Academy of Neurology and the Practice Committee of the Child Neurology Society. Neurology. 2004; 62(6):851-63.
- Fischer J, et al. The Multiple Sclerosis Functional Composite Administration and Scoring Manual. National Multiple Sclerosis Society. 2001; 1-41.
- Bohannon R. Comfortable and maximum walking speed of adults aged 20-79 years: reference values and determinants. Age and Ageing. 1997; 26: 15-19.
- Mathiowetz V, Volland G, Kashman N , et al. Adult norms for the Box and Block Test of manual dexterity. Am J Occup Ther. 1985; 36(6): 386-391.
- Mathiowetz V, Kashman N, Volland G, et al. Grip and pinch strength; normative data for adults. Arch Phys Med Rehabil. 1985; 66:69-72.
- Senn S. In: Cross-over Trials in Clinical Research, 2nd edition, LTD. John Wiley & Sons. 2002.
- Code of Federal Regulations, Title 21 Food and Drugs. In: Selected Regulations and Guidance for Drug Studies. Philadelphia, PA: Clinical Research Resources; Rev. April 1, 2006.
- World Medical Association. Declaration of Helsinski: Ethical Principles for Medical Research Involving Human Subjects. Helsinki, Finland, June 1964.

### 6.6 Example 6: A double-blind, placebo-controlled, crossover study in subjects with CP to evaluate the effect of Dalfampridine on sensorimotor function

The abbreviations and specialist terms used in this example are identical to those used in Example 5 (Table 9).

### 6.6.1 STUDY OBJECTIVES

The effect of dalfampridine-ER 10 mg (i.e., a sustained release formulation of 10 mg 4-aminopyridine), given as both single and multiple doses to patients with CP, will be reported on the following functions as measured by any of the following tests:
- Hand strength as measured by a composite Z-score derived from the grip test, and key, tip and palmar pinch tests
- Manual dexterity as measured by the Box and Block Test
- Walking speed as measured by the Timed 25 Foot Walk (T25FW)
- Gait as measured by gait analysis equipment (to be performed by sites that have the capability to perform it)
- For Part B only, subjective impressions of treatment as measured by:
   Subject Global Impression (SGI)
   Clinician Global Impression;
in order to provide an indication of the efficacy of dalfampridine-ER 10 mg given as a once daily dosage or a twice daily dosage.

### 6.6.2 INVESTIGATIONAL PLAN

This is a multi-center double-blind, placebo-controlled, crossover study. The study will be divided into two parts, A and B, with different subjects enrolled into each part. In Part A, subjects will receive a single dose of investigational product; in Part B, subjects will receive multiple doses of investigational product. Part B will not begin until all the subjects from Part A have completed all visits and it has been confirmed that there is no safety signal that would preclude administration of multiple doses.

For both parts of the study, eligibility will be determined at a screening visit (Visit 1) through a review of medical history, and results from physical examination, vital sign measurements, SMA-12 chemistry tests including calculated creatinine clearance, urinalysis, and a urine pregnancy test for women.

If a subject meets eligibility requirements, a battery of functional tests will be administered at screening to determine manual dexterity (Box and Block), hand strength (grip test, tip pinch, key pinch and palmar pinch tests), and walking speed (T25FW). The test which captures his or her most pronounced functional deficit as determined by a pre-specified algorithm will be assigned as the subject's key variable for analysis; all the other tests will become the subject's additional variables for analysis. An assessment of gait analysis will also be performed if feasible, but will not be used to determine the subject's key functional variable.

### PART A (Cohort 1):

Within a week after the screening visit, qualified subjects in Cohort 1 will check into the clinic to be enrolled into study Period 1. On this study Day 1, Visit 2, subjects will be randomized 1:1 to one of two blinded treatment sequences: AB (dalfampridine-ER followed by placebo), or BA (placebo followed by dalfampridine-ER). Subjects will undergo the full battery of tests to provide pre-dose baseline measurements. The tests will be administered by an evaluator who is blind to the assessment of the subject's most pronounced functional deficit as determined by a different evaluator at the screening visit. Each subject will then take a witnessed single dose of double-blind investigational product (dalfampridine-ER or placebo tablet) with water. Box and Block, hand strength tests (grip strength, tip pinch, key pinch and the palmar pinch), and T25FW will be re-administered at three more time points at 3, 4, and 5 hours following the dose of investigational product, to correspond to approximate peak central nervous system concentration of dalfampridine. There will be a ±20 min window from each time point to administer the tests. Gait analysis will be performed only at pre-dose and at the 5-hour post-dose time point. Subjects will be discharged after completion of Day 1 study procedures.

There will be a washout period (Day 2).

On Day 3, Visit 3, subjects will return to the clinic. Subjects will undergo the full battery of tests prior to dosing. They will receive a witnessed single dose of their Period 2 (cross-over) treatment. The battery of tests will be repeated at 3, 4, and 5 hours post-dose. Gait analysis will be performed only at pre-dose and at the last time point after dosing, as described for Day 1. Subjects will be discharged after completion of study procedures. Visit 3 will be the last clinic visit for Cohort 1.

Adverse events will be monitored through the duration of the study, including a follow-up telephone call on Day 5 (+3 days). Additionally, on the treatment days a brief physical examination will be performed and vital sign measurements taken at pre-dose, and again after the functional assessments 5 hours post-dose, to assess potential changes from baseline.

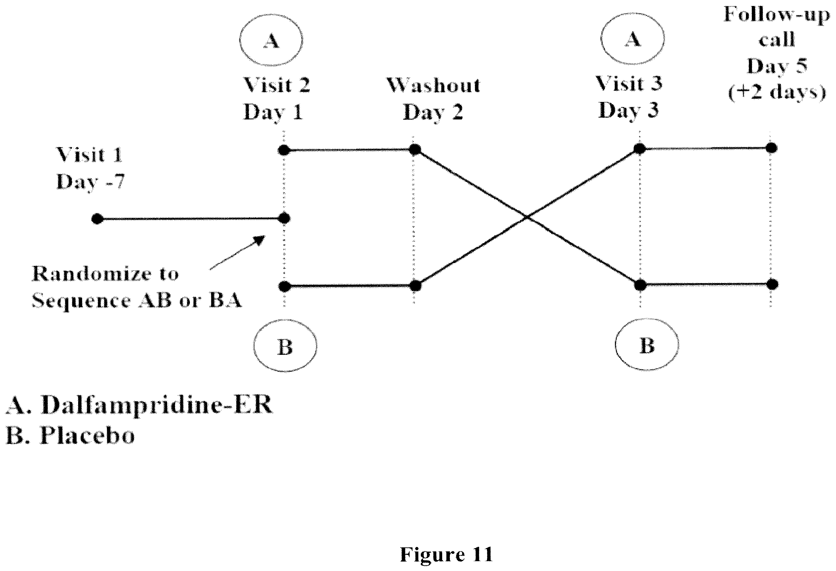

The study design for Part A is displayed graphically in Figure 11. The timing of assessments is presented in Table 12 and details of the study measurements and procedures can be found below.

**Table 12: Schedule of Assessments Part A (Cohort 1)**

| | | **Period 1** | | **Period 2** | |
|---|---|---|---|---|---|
| **Procedure** | **Screening** | **Treatment A or B** | **Wash-out** | **Treatment B or A** | **Follow-up call** |
| | **Visit 1** | **Visit 2** | | **Visit 3** | |
| | **Day -7 to Day -1** | **Day 1** | **Day 2** | **Day 3** | **Day 5 (+2 days)** |
| Written informed consent | X | | | | |
| Medical history | X | | | | |
| Concomitant meds/therapy | X | | | | X |
| Physical exam¹ | X | X | | X | |
| Vital signs² | X | X | | X | |
| SMA-12³,⁴ and creatinine clearance calculation | X | | | | |
| Urinalysis⁴ | X | | | | |
| Urine pregnancy test⁴ | X | | | | |
| Adverse event review | X | X | | X | X |
| Box and Block ⁵ | X | X | | X | |
| Hand strength ^{5,6} | X | X | | X | |
| T25FW^{5,7} | X | X | | X | |
| Gait analysis⁸ | X | X | | X | |
| Evaluator Assessment | X | X | | X | |
| Randomize | | X | | | |
| Administer dose | | X | | X | |
| Discharge subject | | X | | X | |
| Final status assessment | | | | | X |

| | | | | | |
|---|---|---|---|---|---|
| ¹Full physical only at screening. Brief physical on the treatment days: pre-dose and after the 5 hr (± 20 min) post-dose time point for functional assessments. ²Measured at 2 time points on the treatment days: pre-dose (after functional assessments) and after the 5 hr (± 20 min) post-dose time point for functional assessments. Height and weight only at Screening. ³Labs can also be performed at other visits to follow up on an adverse event. ⁴All sampling is to be done after the functional assessments have been performed ⁵Administered at 4 time points on the treatment days: pre-dose, and post-dose hours 3, 4, and 5 (± 20 min) ⁶Three trials for each hand of the grip test, tip pinch, key pinch and palmar tests at each time point. ⁷Two trials of the T25FW at each time point. ⁸For sites that have the capability to perform this testing, performed at 2 time points on the treatment days: pre-dose and 5 hr (± 20 min) post-dose. | | | | | |

The investigational plan for Part B of the study, with a second cohort of new subjects, follows:

### PART B (Cohort 2):

Within a week after the screening visit, qualified subjects from Cohort 2 will check into the clinic to be enrolled into study Part B, Period 1. On this study Day 1, Visit 2, subjects will be randomized 1:1 to one of two blinded treatment sequences: AB (dalfampridine-ER then placebo) or BA (placebo then dalfampridine-ER). Subjects will undergo the full battery of functional tests to provide pre-dose baseline measurements for Period 1. The tests will be administered by an evaluator who is blind to the assessment of the subject's most pronounced functional deficit as determined by a different evaluator at the screening visit. A witnessed single dose of double-blind investigational product (dalfampridine-ER or placebo tablet) will be administered after the baseline assessments. Box and Block, hand strength tests (grip strength, tip pinch, key pinch and the palmar pinch), and T25FW will be re-administered at three more time points at 3, 4, and 5 hours following the dose of investigational product, to correspond to approximate peak central nervous system concentration of dalfampridine. For each time point, all the tests are to be done within a ± 20 minutes time window. Gait analysis will be performed only at pre-dose and the 5-hour post-dose time point. Subjects will be discharged after completion of Day 1 study procedures. They will receive a supply of their assigned Period 1 treatment, with instructions to take one dose approximately every 12 hours at home, beginning the evening of Day 1 (next dose to be taken approximately 12 hours after the witnessed dose in the clinic).

Subjects will return to the clinic for Visit 3 on Day 8 after taking the last dose of their Period 1 treatment in the morning. Subjects will undergo one set of the full battery of functional tests. In addition, a CGI and SGI will be obtained. Subjects will then be discharged with a supply of placebo, to begin a washout period. They will be instructed to take one dose of placebo approximately every 12 hours at home, beginning the evening of Day 8, with last dose taken on the evening of Day 14.

Subjects will return to the clinic for Visit 4 on Day 15, the start of Period 2. They will undergo the same schedule of baseline and post-dose assessments as described for Day 1, and in addition, a CGI and SGI will be added to the baseline assessments. They will receive a witnessed single dose of their Period 2 (cross-over) treatment, per the sequence established at randomization. After completion of all post-dose assessments, subjects will be discharged with a supply of their assigned treatment, to be taken at home, beginning the evening of Day 15 (next dose to be taken approximately 12 hours after the witnessed dose in the clinic).

Subjects will return to the clinic for Visit 5 on Day 22, after taking the last dose of their Period 2 treatment in the morning. Subjects will undergo one set of the full battery of functional assessments. In addition, a CGI and SGI will be obtained. Subjects will then be discharged, and Visit 5 will be the last clinic visit for the Cohort 2.

Cohort 2 will be instructed to have a dosing schedule targeted towards taking a morning dose of investigational product 2 hours prior to the start of scheduled study assessments at Visits 3 and 5.

Blood samples will be obtained for determination of plasma study drug concentration at the beginning and end of Periods 1 and 2.

Adverse events will be monitored through the duration of the study, including a follow-up telephone call on Day 29 (±2 days). Additionally, brief physical examinations will be performed and vital sign measurements taken after functional assessments during each study period to assess potential changes from baseline.

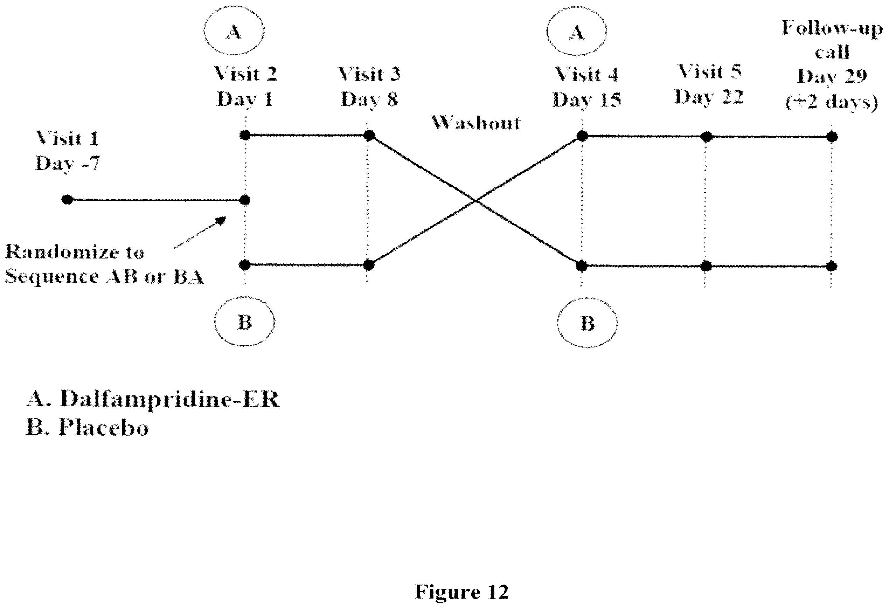

The study design for Part B is displayed graphically in Figure 12. The timing of assessments is presented in Table 13 and details of the study measurements and procedures can be found below.

**Table 13: Schedule of Assessments Part B (Cohort 2)**

| | | **Period 1** | | **Wash-out** | **Period 2** | | |
|---|---|---|---|---|---|---|---|
| **Procedure** | **Screening** | **Treatment A or B** | | **Placebo** | **Treatment B or A** | | **Follow-up call** |
| | **Visit 1** | **Visit 2** | **Visit 3** | | **Visit 4** | **Visit 5** | |
| | **Day -7 to Day -1** | **Day 1** | **Day 8¹** | | **Day 15** | **Day 22¹** | **Day 29 (±2 days)** |
| Written informed consent | X | | | | | | |
| Medical history | X | | | | | | |
| Concomitant meds/therapy | X | | | | | | X |
| Physical exam | X² | X³ | X⁴ | | X³ | X⁴ | |
| Vital signs | X⁵ | X³ | X⁴ | | X³ | X⁴ | |
| SMA-12^{6,7} and creatinine clearance calculation | X | | | | | | |
| Plasma dalfampridine concentration⁷ | | X⁸ | X | | X⁸ | X | |
| Urinalysis⁷ | X | | | | | | |
| Urine pregnancy test⁷ | X | | | | | | |
| Adverse event review | X | X | X | | X | X | X |
| SGI | | | X | | X | X | |
| Box and Block | X | X⁹ | X | | X⁹ | X | |
| Hand strength ¹⁰ | X | X⁹ | X | | X⁹ | X | |
| T25FW¹¹ | X | X⁹ | X | | X⁹ | X | |
| Gait analysis¹² | X | X¹³ | X | | X¹² | X | |
| CGI | | | X | | X | X | |
| Evaluator Assessment | X | X | X | | X | X | |
| Randomize | | X | | | | | |
| Administer witnessed dose | | X | | | X | | |
| Dispense investigational product to take at home | | X | X | | X | | |
| Discharge subject | | X | X | | X | X | |
| Final status assessment | | | | | | | X |

| | | | | | | | |
|---|---|---|---|---|---|---|---|
| ¹All measurements will be performed after the last dose of Period 1 treatment on Day 8 and last dose of Period 2 treatment on Day 22. ²Full physical only at screening; brief physical at subsequent visits. ³Measured at 2 time points: pre-dose (after functional assessments) and after the 5 hr (± 20 min) post-dose time point for functional assessments. ⁴Measured after functional assessments. ⁵Height and weight only at Screening. ⁶ Labs can also be performed at other visits to follow up on an adverse event. ⁷All sampling is to be done after the functional assessments have been performed. ⁸ At Visits 2 and 4, the sample will be obtained before the witnessed dose is administered. ⁹Administered at 4 time points: pre-dose, and post-dose hours 3, 4, and 5 (± 20 min) ¹⁰Three trials for each hand of the grip test, tip pinch, key pinch and palmar tests at each time point. ¹¹Two trials of the T25FW at each time point. ¹²For sites that have the capability to perform this testing ¹³Performed at 2 time points: pre-dose and 5 hr (± 20 min) post-dose. | | | | | | | |

For the study, a sufficient number of subjects will be randomized to ensure that 10 subjects complete Part A and 20 subjects complete Part B.

### 6.6.3 SELECTION AND WITHDRAWAL OF SUBJECTS

### (a) Inclusion Criteria

Subjects must fulfill all of the inclusion criteria listed below to be eligible for participation in the study:
- A diagnosis of CP
- Man or woman 18 to 55 years of age, inclusive
- Body mass index (BMI) ranging between 18.0 - 30.0 kg/m,² inclusive
- No previous use of any dalfampridine formulation
- Provide a signed Informed Consent Form (provided by subject or subject's legally acceptable representative)
Ability to perform all the required study procedures. Subjects should be capable of fully extending and flexing both hands.

### (b) Exclusion Criteria

Subjects who meet any of the following exclusion criteria are not eligible for participation in the study:
1. Presence of any progressive neurological disease
2. Severe CP defined as the requirement to use a wheelchair at all times and a care taker for constant assistance in daily activities. This definition includes spastic quadriplegia.
3. Sexually active women of childbearing potential defined as: not surgically sterile or < two years postmenopause who is not using effective birth control methods
4. Pregnant or breastfeeding
5. History of seizures, excluding simple febrile seizure, which by definition is a single, generalized seizure of less than 15 minutes of duration occurring between the ages of 6 months to 5 years
6. Moderate or severe renal impairment as defined by a calculated creatinine clearance of ≤ 50 mL/minute using the Cockcroft-Gault Equation
7. Presence of active urinary tract infection (UTI) at the Screening Visit or within the 4 weeks prior to the Screening Visit
8. Initiation of concomitant prescription medication regimen or therapy within the 3 weeks prior to the Screening Visit
9. Initiation of baclofen, tizanidine or any other antispasticity medications, or any change in the dosing regimen of these drugs, within the 4 weeks prior to the Screening Visit
10. Botulinum toxin administered within two months prior to the Screening Visit
11. History of drug or alcohol abuse within the past year
12. An abnormal laboratory value at the Screening Visit that, in the Evaluator's judgment, is both clinically significant and has the potential to affect the subject's ability to safely complete the study
13. Any medical condition that would interfere with conduct of study or interpretation of the study results
14. Participation in an investigational trial within the 4 weeks prior to the Screening Visit

### (c) Withdrawal Criteria

The withdrawal criteria, which are optional, include one or more of the following reasons:
- Subject experiences an adverse event
- Pregnancy
- Subject is non-compliant with the protocol
- Subject is lost to follow-up
- Subject abuses alcohol or drugs or no longer meets another eligibility criterion

### 6.6.4 TREATMENT OF SUBJECTS

### (a) Treatments to be Administered

### PART A (Cohort 1):

Each subject will receive a single witnessed dose of (A) dalfampridine-ER 10 mg, and a single witnessed dose of (B) placebo. The tablets will be taken with water. The order of treatment will be determined as described below.

### PART B (Cohort 2):

Each subject will receive 15 doses of (A) dalfampridine-ER 10 mg, and 28 doses of (B) placebo (including 13 doses to be taken during the placebo washout period). The tablets will be taken at with water. The order of treatment will be determined as described below.

At Visit 2 (start of Period 1) and Visit 4 (start of Period 4), the first dose of investigational product will be a witnessed dose in the clinic (the first dose from the 16-count tablet bottle). The bottle containing the remaining 15 tablets (14 doses plus 1 extra tablet) of investigational product will be dispensed to the subject at these visits after assessments have been completed. Subjects will be instructed to take a dose from the bottle in the evening of the visit, approximately 12 hours after the witnessed dose in the clinic, and the next dose the following morning, approximately 12 hours later. Subjects will be instructed to continue dosing every 12 hours at times that are as consistent as possible. Subjects will be told that they must not make up for missed doses.

Subjects will be instructed to have a dosing schedule targeted towards taking a morning dose of investigational product 2 hours prior to the start of scheduled study assessments at Visits 3 and 5.

At Visit 3, the start of the washout period, subjects will receive a 16-count bottle (containing 13 doses plus 3 extra tablets) of placebo, with the same instructions to take a dose approximately every 12 hours beginning the evening of Visit 3. Subjects will be instructed to take their last dose from this bottle in the evening prior to Visit 4.

### (b) Method of Assigning Subject to Treatment Group

Subjects will be randomized at Visit 1 to one of two treatment sequences in a 1:1 ratio according to a randomization created prior to the start of the study:
AB: Dalfampridine-ER then Placebo
BA: Placebo then Dalfampridine-ER

### (c) Blinding

Drug administration for Periods 1 and 2 will be double-blind, meaning that the treatment sequence is not known to the subject or the study site personnel.

The washout period in Part B will be single-blind, meaning that the study site personnel, but not the subject, will know that placebo is being administered during this period.

### (d) Treatment Compliance

In Parts A and B, the time of administration of the witnessed dose will be recorded in the source document. In Part B, subjects will be encouraged to take all doses (except for the first dose of each period) at home as prescribed. Treatment compliance in Part B will be monitored through inventory of tablet count in returned bottles, and by obtaining blood samples for determination of plasma dalfampridine concentration at the beginning and end of each period. Any reasons for non-compliance will be documented.

### (e) Prior and Concomitant Medications

The following medications are excluded for the duration of the study, and also for some period of time prior to the study, for the purpose of maintaining stable symptoms:
- Baclofen, tizanidine or any other antispasticity medications initiated or dosing modified within less than 4 weeks before the Screening Visit
- Botulinum toxin administered less than two months before the Screening Visit
- Other prescription medications or therapies initiated or changed less than 3 weeks before the Screening Visit

No changes will be made to the concomitant treatment during the study, except as required for the safety of the subject.

### 6.6.5 DESCRIPTION OF INVESTIGATIONAL PRODUCT

### Part A

*Active:* Dalfampridine-ER will be supplied as unmarked, film coated, white to off-white, biconvex, oval-shaped, non-scored tablets with a flat edge, containing 10 mg of dalfampridine. Inactive ingredients consist of colloidal silicon dioxide, hydroxypropyl methylcellulose, magnesium stearate, microcrystalline cellulose, polyethylene glycol, and titanium dioxide.

*Placebo:* The placebo tablets will be identical in appearance to the dalfampridine-ER tablets and contain the same inactive ingredients.

### Part B

*Active:* Commercial drug will be used. AMPYRA (dalfampridine) Extended Release tablets are a white to off-white, biconvex, oval shaped, film-coated, non-scored tablet with flat edge, debossed with "A10" on one side, containing 10 mg of dalfampridine. Inactive ingredients consist of colloidal silicon dioxide, hydroxypropyl methylcellulose, magnesium stearate, microcrystalline cellulose, polyethylene glycol, and titanium dioxide.

*Placebo:* The placebo tablets will be identical in appearance to the AMPYRA tablets and contain the same inactive ingredients.

### 6.6.6 STUDY PROCEDURES

The following sections describe the baseline and clinical functional measurements that will be obtained in this study. A detailed schedule of procedures by study visit is provided in the section entitled "Study Sequence," below, and summarized in Table 12 for Part A and Table 13 for Part B.

Prior to engaging in any study procedure, subjects must provide written informed consent.

### (a) Plasma Dalfampridine Concentration

For Part B, only, blood samples for determination of plasma dalfampridine concentration will be obtained at Visits 2, 3, 4, and 5. At each of these visits, a single blood sample will be obtained after functional assessments have been performed. At Visits 2 and 4, the sample will be obtained before the witnessed dose is adminstered. The time of sampling and the time of the administration of the witnessed dose of investigational product (or approximate time the last morning dose was taken at home), will be recorded

A minimum of 7 mL of whole blood will be collected into an appropriately labeled heparin tube and kept cold (i.e., on wet ice) until centrifuged. Immediately after collection, the tube will be centrifuged at low speed and approximately 3 mL of plasma will be transferred from each sample into a labeled tube. The plasma will be stored at -20° C until the shipment to the central laboratory is requested. At that time, frozen plasma samples will be collected together and sent in an insulated container, on dry ice, overnight by express carrier to the designated central laboratory.

### (b) Functional Assessments

This section describes the clinical assessments of CP to be used in this study.

For a given subject, the evaluator administering the T25FW, Box and Block, and Grip and Pinch tests at the screening visit (Visit 1) will not be the same person who administers these tests at subsequent visits. At subsequent visits, the tests will be administered by a new evaluator who is blind to the first evaluator's assessment of the subject's most pronounced functional deficit as determined at the screening visit. The new evaluator will administer the functional tests for all subsequent visits for a given subject.

### Timed 25 Foot Walk (T25FW)

The T25FW test is a quantitative measure of ambulatory function. The subject is instructed to walk as quickly as he or she can from one end to the other end of a clearly marked, unobstructed, 25-foot course. The T25FW will be performed according to the detailed instructions provided in the Administration and Scoring Manual published by the National Multiple Sclerosis Society (Fischer J, et al., National Multiple Sclerosis Society. 2001; 1-41). The instruction booklet will be provided, and is summarized here. The subject will stand with the tip of their shoes on a marked starting line, and timing will begin when any part of the subject's foot crosses the line. Timing will end when any part of the subject's foot crosses the marked finish line. Time will be recorded in seconds and rounded to the nearest tenth of a second using a stopwatch provided for this study. The task is administered again, with a maximum five-minute rest period allowed between trials, by having the subject walk back the same distance. If required, the subject may use an appropriate assistive device. The subject will be instructed to maintain his or her normal activities without rehearsal or practice measures to unfairly improve their performance scores between visits. Every effort will be made to use the same testing room and the same designated area for the T25FW at each assessment. Potential for external distractions will be kept to a minimum as much as possible.

Normative data for walking speed are available (Bohannon R., Age and Ageing. 1997; 26: 15-19). For subjects ≥18 years of age and <20 years of age, the normative data for the 20s decade age group will be used.

### Box and Block

The Box and Block test (Mathiowetz V, Volland G, Kashman N , et al., Am J Occup Ther. 1985; 36(6): 386-391) has been used as a valid and reliable measure of manual dexterity. The subject is instructed to quickly pick up blocks one at a time from one side of a box, transport each block over a partition to the other side of the box, and drop it. The test was originally developed to evaluate the gross manual dexterity of adults with cerebral palsy. Data for normal adults are available (see Bohannon R., Age and Ageing. 1997; 26: 15-19). For subjects ≥18 years of age and <20 years of age, the normative data for the 20-24 years age group will be used.

The Box and Block test will be consistently performed before the Pinch and Grip tests to minimize the effects of fatigue.

### Hand Strength by the Grip and Pinch tests

The Grip Test (Mathiowetz V, Kashman N, Volland G, et al., Arch Phys Med Rehabil. 1985; 66:69-72) is used as a simple, valid and reliable measure to identify hand strength problems, to detect the change which may result from an occupational therapy program, the course of a disease or injury, or to show the relation of the patient's strength to the general population. Hand strength is measured using a dynamometer.

The Pinch Tests (Mathiowetz V, Kashman N, Volland G, et al., Arch Phys Med Rehabil. 1985; 66:69-72) are used as a simple, valid and reliable measure to indentify pinch strength problems, to detect the change which may result from an occupational therapy program, the course of a disease or injury, or to show the relation of the patient's strength to the general population. It has three components, the tip, key and palmar pinch. Pinch strength is measured using a pinch gauge.

Normative data on the Grip and Pinch tests in adults are available (see Mathiowetz V, Kashman N, Volland G, et al., Arch Phys Med Rehabil. 1985; 66:69-72). For subjects ≥18 years of age and <20 years of age, the normative data for the 20-24 years age group will be used.

### Gait Analysis

Gait analysis will be performed at sites that have the capability to perform it. The parameters of interest for gait analysis are: stride length, cadence, single support time, and double support time. Stride length variability and swing time variability may also be assessed. For Part B, only, velocity will be added as an additional measurement..

### Subject Global Impression (SGI)

The SGI is a commonly used measure of treatment response that asks the subject to rate the effects of the investigational drug on his or her physical well-being during the preceding week, using a 7-point scale ranging from "terrible" to "delighted."

The subjects is be given a form to complete stating the following: "We want to find out how you feel about the effects of the study medication on your physical well-being. How do you feel about the effects of the study medication over the past 7 days?" The subject is given the following choices for a response: "terrible," "unhappy," "mostly dissatisfied," "neural/mixed," "mostly satisfied," "pleased," and "delighted." The subject is asked to explain the response given in their own words.

This scale will be administered only in Part B.

### Clinician Global Impression (CGI)

The CGI is a commonly used measure of treatment response that asks the clinician to provide an overall impression of the changes in the subject's neurological status and general state of health following treatment with the investigational product, as compared to the subject's condition at baseline (and not compared to the preceding visit). The CGI is rated according to a 7-point scale ranging from "very much improved" to "very much worse."

The clinician is given a form to complete stating the following: "Overall, taking into account the subject's symptoms and other neurological functions, how would you rate the subject's neurological status today, relative to their Screening Visit? Please consider neurological changes only, without respect to other factors." The clinician is given the following choices for a response: "very much improved," "much improved," "somewhat improved," "no change," "somewhat worse," "much worse," and "very much worse." The clinician is asked to explain any indication of change, if possible.

This scale will be administered only in Part B.

### Evaluator Assessment

The first evaluator will answer the following question about each subject after administration of the battery of tests at the Screening Visit: Which test, according to the evaluator's assessment, captures the subject's most pronounced functional deficit?
D. Box and Block
E. Grip and Pinch Tests
F. T25FW

A different evaluator will answer the following question about each subject after last administration of the battery of tests on dosing days for Part A and at the beginning and end of each period for Part B: On which test, according to evaluator's assessment, does the subject show the most improvement?
E. Box and Block
F. Grip and Pinch Tests
G. T25FW
D. None

### (c) Study Sequence

### 1.1.1. Part A (Cohort 1)

The following sections describe the assessments to be performed at each clinic visit during Part A of the study.

### Day -7 to Day -1 (Visit 1, Screening)

The Evaluator will assess eligibility after the following procedures have been performed in the order presented:
- Obtain signed informed consent
- Assign subject number
- Obtain medical history, demographic information, and CP history including date of initial diagnosis and current clinical presentation
- Record all concomitant medications and therapies taken within three weeks of this visit
- Administer Box and Block test (dominant and non-dominant hand, the dominant first)
- Administer grip test, tip pinch, key pinch and palmar pinch tests. These tests will be administered three times with each hand.
- Administer two trials of the T25FW
- Perform gait analysis (if feasible)
- Complete a full physical examination
- Obtain sitting vital sign measurements and height and weight
- Take blood and urine samples for laboratory evaluations (SMA-12, calculated creatinine clearance, urinalysis, and urine pregnancy test for women of childbearing potential)
- Complete Evaluator Assessment
- Review adverse events
- Discharge subject and schedule a date and time for the next visit to occur within one week

### Day 1, Visit 2 (Period 1)

The following assessments and procedures will be performed in the order outlined below:

### Pre-dose

- Box and Block test (dominant and non-dominant hand, the dominant first)
- Grip test, tip pinch, key pinch and palmar pinch tests. These tests will be administered three times with each hand.
- Two trials of the T25FW
- Gait analysis (if feasible)
- Brief physical examination
- Sitting vital sign measurements
- Review adverse events
- Randomize to one of two treatment sequences

### Administer single dose of Period 1 double-blind investigational drug

### Post-dose

- Box and Block test at 3 hr (±20 min), 4 hr (±20 min), and 5 hr (±20 min) (dominant and non-dominant hand, the dominant first)
- Grip test, tip pinch, key pinch and palmar pinch tests at 3 hr (±20 min), 4 hr (±20 min), and 5 hr (±20 min). Each test will be administered three times per each hand at each time point.
- Two trials of the T25FW test at 3 hr (±20 min), 4 hr (±20 min), and 5 hr (±20 min)
- Gait analysis, if feasible, at 5 hr (±20 min)
- Brief physical examination after the 5 hr (± 20 min) post-dose time point for the functional assessments.
- Sitting vital sign measurements after the 5 hr (± 20 min) post-dose time point for the functional assessments.
- Complete Evaluator Assessment
- Review adverse events
- Discharge subject after completing all 5-hour post-dose procedures

### Day 2 (Washout)

There will be no study assessments on Day 2.

### Day 3, Visit 3 (Period 2)

The following assessments and procedures will be performed in the order outlined below:

### Pre-dose

- Box and Block test (dominant and non-dominant hand, the dominant first)
- Grip test, tip pinch, key pinch and palmar pinch tests. Each test will be administered three times with each hand.
- Two trials of the T25FW
- Gait analysis (if feasible)
- Brief physical examination
- Sitting vital sign measurements
- Review adverse events

### Administer single dose of Period 2 double-blind investigational drug

### Post-dose

- Box and Block at 3 hr (±20 min), 4 hr (±20 min), and 5 hr (±20 min) (dominant and non-dominant hand, the dominant first)
- Grip test, tip pinch, key pinch and palmar pinch tests at 3 hr (±20 min), 4 hr (±20 min), and 5 hr (±20 min). Each test will be administered three times per each hand at each time point.
- Two trials of the T25FW test at 3 hr (±20 min), 4 hr (±20 min), and 5 hr (±20 min)
- Gait analysis, if feasible, at 5 hr (±20 min)
- Brief physical examination after the 5 hr (± 20 min) post-dose time point for the functional assessments
- Sitting vital sign measurements after the 5 hr (± 20 min) post-dose time point for the functional assessments
- Complete Evaluator Assessment
- Review adverse events
- Discharge subject after completing all 5-hour post-dose procedures

### Day 5 (+ 3 days) (Telephone Follow-up)

The site will make a follow-up telephone call to review any adverse events or changes in medication. The follow-up call may be performed up to 3 days after Day 5 to account for the weekend and/or holidays.
- Final status assessment

### Part B (Cohort 2)

The following sections describe the assessments to be performed at each clinic visit during Part B of the study.

### Day -7 to Day -1 (Visit 1, Screening)

The Evaluator will assess eligibility after the following procedures have been performed in the order presented:
- Obtain signed informed consent
- Assign subject number
- Obtain medical history, demographic information, and CP history including date of initial diagnosis and current clinical presentation
- Record all concomitant medications and therapies taken within three weeks of this visit
- Administer Box and Block test (dominant and non-dominant hand, the dominant first)
- Administer grip test, tip pinch, key pinch and palmar pinch tests. Each test will be administered three times with each hand.
- Administer two trials of the T25FW
- Perform gait analysis (if feasible)
- Complete a full physical examination
- Obtain sitting vital sign measurements and height and weight
- Take blood and urine samples for laboratory evaluations (SMA-12, calculated creatinine clearance, urinalysis, and urine pregnancy test for women of childbearing potential).
- Complete Evaluator Assessment
- Review adverse events
- Discharge subject and schedule a date and time for the next visit to occur within one week

### Day 1, Visit 2 (start of Period 1)

The following assessments and procedures will be performed in the order outlined below:

### Pre-dose

- Box and Block test (dominant and non-dominant hand, the dominant first)
- Grip test, tip pinch, key pinch and palmar pinch tests. Each test will be administered three times with each hand.
- Two trials of the T25FW
- Gait analysis (if feasible)
- Brief physical examination
- Sitting vital sign measurements
- Obtain blood sample for plasma dalfampridine concentration
- Review adverse events and concomitant medications
- Randomize to one of two treatment sequences

### Administer single dose of Period 1 double-blind investigational drug

### Post-dose

- Box and Block test at 3 hr (±20 min), 4 hr (±20 min), and 5 hr (±20 min) (dominant and non-dominant hand, the dominant first)
- Grip test, tip pinch, key pinch and palmar pinch tests at 3 hr (±20 min), 4 hr (±20 min), and 5 hr (±20 min). Each test will be administered three times per each hand at each time point.
- Two trials of the T25FW test at 3 hr (±20 min), 4 hr (±20 min), and 5 hr (±20 min)
- Gait analysis, if feasible, at 5 hr (±20 min)
- Brief physical examination after the 5 hr (± 20 min) post-dose time point for the functional assessments.
- Sitting vital sign measurements after the 5 hr (± 20 min) post-dose time point for the functional assessments.
- Complete Evaluator Assessment
- Review adverse events
- Dispense a bottle of Period 1 double-blind investigational product with instructions to take the next dose that evening, approximately 12 hours after time of first dose.
- Discharge subject and schedule a date and time for the next visit to occur in one week

### Day 8, Visit 3 (end of Period 1, start of washout)

The following assessments and procedures will be performed in the order outlined below:
- Subject to complete the SGI
- Box and Block (dominant and non-dominant hand, the dominant first)
- Grip test, tip pinch, key pinch and palmar pinch tests. Each test will be administered three times with each hand.
- Two trials of the T25FW test
- Gait analysis (if feasible)
- Brief physical examination after the functional assessments
- Sitting vital sign measurements after the functional assessments
- Obtain blood sample for plasma dalfampridine concentration
- Complete the CGI
- Complete Evaluator Assessment
- Review adverse events and concomitant medications
- Perform investigational product accountability
- Dispense a bottle of single-blind placebo with instructions to take first dose that evening at approximately the same time as the evening doses taken during Period 1. Subjects will be instructed to take their last dose from this bottle in the evening before Visit 4.
- Discharge subject and schedule a date and time for the next visit to occur in one week

### Day 15, Visit 4 (end of washout, start of Period 2)

The following assessments and procedures will be performed in the order outlined below:

### Pre-dose

- Subject to complete the SGI
- Box and Block test (dominant and non-dominant hand, the dominant first)
- Grip test, tip pinch, key pinch and palmar pinch tests. Each test will be administered three times with each hand.
- Two trials of the T25FW
- Gait analysis (if feasible)
- Brief physical examination
- Sitting vital sign measurements
- Obtain blood sample for plasma dalfampridine concentration
- Complete the CGI
- Review adverse events

### Administer single dose of Period 2 double-blind investigational drug

### Post-dose

- Box and Block test at 3 hr (±20 min), 4 hr (±20 min), and 5 hr (±20 min) (dominant and non-dominant hand, the dominant first)
- Grip test, tip pinch, key pinch and palmar pinch tests at 3 hr (±20 min), 4 hr (±20 min), and 5 hr (±20 min). Each test will be administered three times per each hand at each time point.
- Two trials of the T25FW test at 3 hr (±20 min), 4 hr (±20 min), and 5 hr (±20 min)
- Gait analysis, if feasible, at 5 hr (±20 min)
- Brief physical examination after the 5 hr (± 20 min) post-dose time point for the functional assessments.
- Sitting vital sign measurements after the 5 hr (± 20 min) post-dose time point for the functional assessments.
- Complete Evaluator Assessment
- Review adverse events and concomitant medications
- Perform investigational product accountability
- Dispense a bottle of Period 2 double-blind investigational product with instructions to take the next dose that evening, approximately 12 hours after time of first dose.
- Discharge subject and schedule a date and time for the next visit to occur in one week

### Day 22, Visit 5 (end of Period 2)

The following assessments and procedures will be performed in the order outlined below:
- Subject to complete the SGI
- Box and Block (dominant and non-dominant hand, the dominant first)
- Grip test, tip pinch, key pinch and palmar pinch tests. Each test will be administered three times with each hand.
- Two trials of the T25FW test
- Gait analysis (if feasible)
- Brief physical examination after the functional assessments
- Sitting vital sign measurements after the functional assessments
- Obtain blood sample for plasma dalfampridine concentration
- Complete the CGI
- Complete Evaluator Assessment
- Review adverse events and concomitant medications
- Perform investigational product accountability
- Discharge subject and schedule a date and time for a follow-up phone call to occur in one week (± 2 days)

### Day 29 (Telephone Follow-up)

The site will make a follow-up telephone call to review any adverse events or changes in medication.
- Final status assessment

### 6.6.7 STATISTICS

### (a) Statistical Power

A sufficient number of subjects will be randomized to ensure that 10 subjects complete Part A and 20 subjects complete Part B. This sample size should provide adequate variance estimates for future studies.

### (b) Analyses of Functional Assessments

### Part A (Cohort 1)

At screening, a battery of tests will be administered to each subject, which includes Box and Block, hand strength as measured by the grip strength, tip pinch, key pinch and palmar pinch tests, and T25FW.

Based on an algorithm to be specified in the SAP, each subject's most pronounced functional deficit based on the deviation from the norm among the Box and Block, hand strength and T25FW assessments will be determined, and will be identified as the key functional variable for that subject. For hand strength and dexterity tests, the screening assessments from dominant hands will be used in the determination. Since the assigned key functional variable may differ across subjects, it will be standardized using Z score prior to the analysis.

One of Box and Block, hand strength, and T25FW tests will be determined as the key functional variable for each individual subject; the other tests will be considered as additional variables for that subject. The T25FW, Box and Block, and hand strength component (grip strength, tip pinch, key pinch and palmar pinch tests) variables based on the original scales will be analyzed, and the hand strength variable based on its combined standardized components will also be analyzed. The hand strength and dexterity assessments (i.e., Box and Block, hand strength and its components) will be analyzed using data from both the dominant and non-dominant hand. Also, the test assessed by the evaluator as the one for which the subject shows the most improvement will be analyzed.

Gait parameters, SGI and CGI will also be analyzed.

### Derived Variables and Data Handling

All available data from the FAP and PPP (if applicable) subjects will be included in the corresponding analysis; missing data will not be imputed.

All functional assessments will be performed at the screening visit. On treatment days, Box and Block, hand strength, and T25FW tests will be administered at 4 time points (pre-dose, and post-dose 3 hours, 4 hours, and 5 hours), while the gait assessment will be administered at 2 time points (pre-dose and 5 hours post-dose).

At each time point at screening and treatment days, two trials will be administered for the T25FW test. Walking speed of an individual trial will be derived by multiplying 25 feet and the reciprocal of the time (in seconds) used to complete the walk. The average walking speed of a particular time point will then be derived by calculating the average of Trial 1 and Trial 2. If either trial is missed, the walking speed from the non-missing trial will be used for that time point.

The hand strength assessments include tip pinch, key pinch, palmar pinch and grip strength. At each time point, the average result of three trials from each of these four measurements will be calculated for each hand. Each of the four averages will then be converted to a Z-score, and the sum of these Z-scores will be used as a single, aggregate assessment of hand strength. The Z-scores for each of the four averages will be based on the mean and standard deviation of the averages, calculated using all the subjects within each time point and within each period.

Within each treatment period, the baseline value of a particular functional variable will be the pre-dose assessment on the treatment day. Changes from baseline will then be calculated for each derived functional variable at each post-dose analysis time point. An average post-treatment value will be derived by calculating the average of the post-dose changes from baseline at 3 hours, 4 hours, and 5 hours on the treatment days (except for the gait parameters, which are assessed only at 5 hours post-dose on the treatment days).

Each of the baseline values and changes from baseline for the Box and Block, hand strength, and T25FW variables will be converted to Z-scores, based on the mean and standard deviation of these baseline and change from baseline values, calculated using all the subjects within each time point and within each period. The Z-scores corresponding to each subject's key functional test will be the subject's key functional variable for analysis.

### Statistical Methods

Since the assigned key variable will differ across subjects, it will be standardized using Z score prior to the analysis (as will all functional variables). Additional analyses will also be performed, based on their original scales for each functional variable. Gait will also be analyzed.

Differences between the treatments on each of the functional variables will be assessed by comparing the period differences (Period 2 - Period 1) across the two sequence groups, using the change from baseline values. This controls for any period effects.

Hand tests including hand strength and Box and Block will be analyzed by hand (dominant and non-dominant).

One analysis of the functional assessments will be based upon an adaptation of the Brown-Mood median test (Senn S. In: Cross-over Trials in Clinical Research, 2nd edition, LTD. John Wiley & Sons. 2002). Within each variable and time point, each of the period differences will be classified as being above or below the median period difference. The treatment effect will be assessed by comparing these classifications across the sequence groups via Fisher's exact test. In an additional analysis, the period differences will be compared across the sequence groups via a two-sample t-test. This test is equivalent to an analysis of variance with effects for subject, period and treatment. If the usual parametric assumptions are grossly violated, then the sequences will be compared via the Wilcoxon rank-sum test. Within each period, the distributions of the evaluator's assessment of the test as the one for which the subject shows the most improvement will be compared across the treatment groups and the equality of the distributions will be tested via Fisher's Exact test.

Additional sensitivity analyses will be performed, which will be detailed in the SAP.

### Part B (Cohort 2)

In Cohort 2, the same functional assessments as those in Cohort 1 will be performed for screening visit and the first visit within each period.

The analyses for the first visit within each period will be analogous to the treatment visits of Cohort 1. In addition, the functional assessments from the first treatment visits of both Cohort 1 and Cohort 2 will be pooled together for analysis.

The functional assessments following multiple doses of the study drug will be analyzed based on the changes from baseline to the assessment values at the last visit within the period. However, for the last visit within each period, there will be only one post-dose assessment for functional variables. The baseline within each period is defined as the pre-dose assessment from the first visit within the period. For these changes from baseline of key functional variables and additional variables, the same variable derivation, data handling and statistical methods will be implemented as those described for Cohort 1.

In Cohort 2, SGI and CGI, will also be administered and two types of analyses will be performed for these. The first type of analysis will be based on the intra-subject change from Visit 3 to Visit 5 for the subjects randomized to Sequence BA. The second type of analysis will be based on between-treatment group comparisons at Visit 3.

### 6.6.8 REFERENCES FOR EXAMPLE 6

- Lin JP. The cerebral palsies: a physiological approach. J Neurol Neurosurg Psychiatry. 2003;74(Suppl 1):i23-i29.
- Krigger KW. Cerebral palsy: an overview. Am Fam Physician. 2006;73(1):91-100.
- Odding E, Roebroeck ME, Stam HJ. The epidemiology of cerebral palsy: incidence, impairments and risk factors. Disabil Rehabil. 2006;28(4):183-191.
- Koman LA, Smith BP, Shilt JS. Cerebral palsy. Lancet. 2004;363(9421):1619-1631.
- Carlsson M, Hagberg G, Olsson I. Clinical and aetiological aspects of epilepsy in children with cerebral palsy. Dev Med Child Neurol. 2003;45(6):371-376.
- Humphreys P, Deonandan R, Whiting S, et al. Factors associated with epilepsy in children with periventricular leukomalacia. J Child Neurol. 2007;22(5):598-605.
- Zelnik N, Konopnicki M, Bennett-Back O, Castel-Deutsch T, Tirosh E. Risk factors for epilepsy in children with cerebral palsy. Eur J Paediatr Neurol. 2010;14(1):67-72.
- Johnston MV, Hoon AH Jr. Cerebral palsy. Neuromolecular Med. 2006;8(4):435-450.
- Bax M, Tydeman C, Flodmark O. Clinical and MRI correlates of cerebral palsy: the European Cerebral Palsy Study. JAMA. 2006;296(13):1602-1608.
- Folkerth RD. Neuropathologic substrate of cerebral palsy. J. Child Neurol. 2005;20(12):940-949.
- Wu YW, Croen LA, Shah SJ, Newman TB, Najjar DV. Cerebral palsy in a term population: risk factors and neuroimaging findings. Pediatrics. 2006;118(2):690-697.
- Mikkola K, Ritari N, Tommiska V, et al. Neurodevelopmental outcome at 5 years of age of a national cohort of extremely low birth weight infants who were born in 1996-1997. Pediatrics. 2005;116(6):1391-1400.
- Robertson CM, Watt MJ, Yasui Y. Changes in the prevalence of cerebral palsy for children born very prematurely within a population-based program over 30 years. JAMA. 2007;297(24):2733-2740.
- Beaino G, Khoshnood B, Kaminski M, et al. Predictors of cerebral palsy in very preterm infants: the EPIPAGE prospective population-based cohort study. Dev Med Child Neurol. 2010;52(6):e119-e125.
- O'Shea TM, Preisser JS, Klinepeter KL, Dillard RG. Trends in mortality and cerebral palsy in a geographically based cohort of very low birth weight neonates born between 1982 to 1994. Pediatrics. 1998;101(4 Pt 1):642-647.
- Strauss D, Shavelle R. Life expectancy of adults with cerebral palsy. Dev Med Child Neurol. 1998;40(6):369-375.
- Hemming K, Hutton JL, Pharoah PO. Long-term survival for a cohort of adults with cerebral palsy. Dev Med Child Neurol. 2006;48(2):90-95.
- Cerebral Palsy: Hope through research: National Institute of Neurological Disorders and Stroke (NINDS). Accessed online April 20, 2011, at: http://www.ninds.nih.gov/disorders/cerebral_palsy/detail_cerebral_palsy.htm#154443104
- Rapp CE, Torres M. The adult with cerebral palsy. Arch Fam Med. 2000;9:466-472
- Volpe JJ. Neurobiology of periventricular leukomalacia in the premature infant. Pediatric Res. 2001;50(5):553-562.
- Back SA, Riddle A, McClure MM. Maturation-dependent vulnerability of perinatal white matter in premature birth. Stroke. 2007;38(2 Suppl):724-730.
- Deng W, Pleasure J, Pleasure D. Progress in periventricular leukomalacia. Arch Neurol. 2008;65(10):1291-1295.
- Thomas B, Eyssen M, Peeters R, et al. Quantitative diffusion tensor imaging in cerebral palsy due to periventricular white matter injury. Brain. 2005;128(Pt 11):2562-2577.
- Hoon AH Jr, Stashinko EE, Nagae LM, et al. Sensory and motor deficits in children with cerebral palsy born preterm correlate with diffusion tensor imaging abnormalities in thalamocortical pathways. Dev Med Child Neurol. 2009;51(9):697-704.
- Beckung E, Hagberg G, Uldall P, Cans C. Surveillance of Cerebral Palsy in Europe. Probability of walking in children with cerebral palsy in Europe. Pediatrics. 2008;121(1):e187-e192.
- Strauss D, Ojdana K, Shavelle R, Rosenbloom L. Decline in function and life expectancy of older persons with cerebral palsy. NeuroRehabilitation. 2004;19(1):69-78.
- Goodman AD, Brown TR, Cohen JA, et al. Dose comparison trial of sustained-release fampridine in multiple sclerosis. Neurology. 2008;71:1134-1141.
- Goodman AD, Brown TR, Krupp LB, et al. Sustained-release oral fampridine in multiple sclerosis: a randomized, double-blind, controlled trial. Lancet. 2009;373: 732-38.
- Goodman AD, Brown TR, Edwards KR, et al. A Phase 3 trial of extended release oral dalfampridine in multiple sclerosis. Ann Neurol. 2010; 373: 494-502.
- Stolp HB, Dziegielewaka KM. Review: Role of developmental inflammation and blood-brain barrier dysfunction in neurodevelopmental and neurodegenerative diseases. Neuropathol Appl Neurobiol. 2009; 35:132-146.
- Aksu F. Nature and prognosis of seizures in patients with cerebral palsy. Dev Med Clin Neurol. 1990;32:661-668.
- McDermott S, Moran R, Platt T, et al. Prevalence of epilepsy in adults with mental retardation and related disabilities in primary care. Am J Ment Retard. 2005;110:48-56.
- Ashwal S, Russman RS, Blasco PA, et al. Practice parameter: Diagnostic assessment of the child with cerebral palsy: report of the Quality Standards Subcommittee of the American Academy of Neurology and the Practice Committee of the Child Neurology Society. Neurology. 2004; 62(6):851-63.
- Fischer J, et al. The Multiple Sclerosis Functional Composite Administration and Scoring Manual. National Multiple Sclerosis Society. 2001; 1-41.
- Bohannon R. Comfortable and maximum walking speed of adults aged 20-79 years: reference values and determinants. Age and Ageing. 1997; 26: 15-19.
- Mathiowetz V, Volland G, Kashman N , et al. Adult norms for the Box and Block Test of manual dexterity. Am J Occup Ther. 1985; 36(6): 386-391.
- Mathiowetz V, Kashman N, Volland G, et al. Grip and pinch strength; normative data for adults. Arch Phys Med Rehabil. 1985; 66:69-72.
- Senn S. In: Cross-over Trials in Clinical Research, 2nd edition, LTD. John Wiley & Sons. 2002.
- Code of Federal Regulations, Title 21 Food and Drugs. In: Selected Regulations and Guidance for Drug Studies. Philadelphia, PA: Clinical Research Resources; Rev. April 1, 2006.
- World Medical Association. Declaration of Helsinski: Ethical Principles for Medical Research Involving Human Subjects. Helsinki, Finland, June 1964.

## Claims

1. An aminopyridine or a pharmaceutically acceptable salt thereof for use in a method for treating a sign or symptom of cerebral palsy ("CP") or an impairment or alteration consequent to CP, said method comprising administering the aminopyridine or pharmaceutically acceptable salt thereof to a patient with CP, wherein the symptom of CP or the impairment or alteration consequent to CP is not an impairment in mental status, preferably wherein the patient is human.

2. The aminopyridine or pharmaceutically acceptable salt thereof for the use of claim 1, wherein said aminopyridine or pharmaceutically acceptable salt thereof is present in a pharmaceutical composition comprising a therapeutically effective amount of the aminopyridine or a pharmaceutically acceptable salt thereof and a pharmaceutically acceptable carrier.

3. The aminopyridine or pharmaceutically acceptable salt thereof for the use of claim 1 or 2, wherein said aminopyridine or pharmaceutically acceptable salt thereof is a mono-aminopyridine or a di-aminopyridine, preferably wherein the aminopyridine or pharmaceutically acceptable salt thereof is 4-aminopyridine, 3-aminopyridine or 3,4-diaminopyridine.

4. The aminopyridine or pharmaceutically acceptable salt thereof for the use of any one of claims 1-3, further comprising a step of assessing a CP sign or symptom before administration of the aminopyridine or pharmaceutically acceptable salt thereof, and a step of assessing the same CP sign or symptom after administration of the aminopyridine or pharmaceutically acceptable salt thereof.

5. The aminopyridine or pharmaceutically acceptable salt thereof for the use of any one of claims 1-4, wherein the aminopyridine or pharmaceutically acceptable salt thereof is in a sustained release composition.

6. The aminopyridine or pharmaceutically acceptable salt thereof for the use of any one of claims 1-5, wherein the aminopyridine or pharmaceutically acceptable salt thereof is administered to the patient once daily or twice daily.

7. The aminopyridine or pharmaceutically acceptable salt thereof for the use of any one of claims 1-6, wherein the aminopyridine is administered in an amount in the range of 1 to 20 mg, 5 to 20 mg, 5 to 15 mg, 5 to 10 mg, or 7.5 to 10 mg, once or twice daily.

8. The aminopyridine or pharmaceutically acceptable salt thereof for the use of claim 7, wherein the aminopyridine is administered in the amount of 10 mg, once daily or twice daily.

9. The aminopyridine or pharmaceutically acceptable salt thereof for the use of claim 5, wherein the aminopyridine or pharmaceutically acceptable salt thereof is administered in a single dose of the sustained release composition.

10. The aminopyridine or pharmaceutically acceptable salt thereof for the use of any one of claims 1-9, wherein the aminopyridine or pharmaceutically acceptable salt thereof is administered orally, preferably wherein the aminopyridine or pharmaceutically acceptable salt thereof is formulated in a form of a tablet or a capsule.

11. The aminopyridine or pharmaceutically acceptable salt thereof for the use of any one of claims 1-10, wherein the sign or symptom of CP or the impairment or alteration consequent to CP is a sensorimotor impairment, preferably wherein the sensorimotor impairment is an impairment in walking, impairment in limb function, impairment in lower extremity function, impairment in lower extremity muscle strength, impairment in muscle tone, impairment in upper extremity function, impairment in upper extremity strength, impairment in hand function, impairment in fine hand coordination, impairment in grip strength, impairment in balance or coordination, impairment in global body control, dysarthria, impairment in jaw function, impairment in chewing, or impairment in jaw articulation.

12. The aminopyridine or pharmaceutically acceptable salt thereof for the use of any one of claims 1-10, wherein the sign or symptom of CP or the impairment or alteration consequent to CP is a motor dysfunction, preferably wherein the motor dysfunction is of the upper extremities or of the lower extremities, or in a muscle group of the body core, or in postural musculature.

13. The aminopyridine or pharmaceutically acceptable salt thereof for the use of any one of claims 1-10, wherein the sign or symptom of CP or the impairment or alteration consequent to CP is a walking or ambulation dysfunction, preferably wherein the sign or symptom of CP or the impairment or alteration consequent to CP is an impairment in walking speed; or wherein the sign or symptom of CP or the impairment or alteration consequent to CP is at least one of: impaired muscle tone, impaired reflexes, impaired coordination, spasticity, involuntary movements, impaired gait, impaired balance, decreased muscle mass, a dysfunctional muscle strength, a speech dysfunction, or an impairment in hand strength or manual dexterity.

14. The aminopyridine of claim 1 for the use of any one of claims 1-13.

15. The pharmaceutically acceptable salt of the aminopyridine of claim 1 for the use of any one of claims 1-6 or 9-13.

## Patentansprüche

1. Aminopyridin oder pharmazeutisch akzeptables Salz davon zur Verwendung bei einem Verfahren zur Behandlung eines Anzeichens oder Symptoms von Zerebralparese ("CP") oder einer Beeinträchtigung oder Veränderung infolge von CP, wobei das Verfahren die Verabreichung eines Aminopyridins oder eines pharmazeutisch akzeptablen Salzes davon an einen Patienten mit CP umfasst, wobei das Symptom von CP oder die Beeinträchtigung oder Veränderung infolge von CP keine Beeinträchtigung des Geisteszustands ist, wobei der Patient vorzugsweise ein Mensch ist.

2. Aminopyridin oder pharmazeutisch akzeptables Salz davon zur Verwendung nach Anspruch 1, wobei das Aminopyridin oder das pharmazeutisch akzeptable Salz davon in einer pharmazeutischen Zusammensetzung vorliegt, die eine therapeutisch wirksame Menge des Aminopyridins oder eines pharmazeutisch akzeptablen Salzes davon und einen pharmazeutisch akzeptablen Träger umfasst.

3. Aminopyridin oder pharmazeutisch akzeptables Salz davon zur Verwendung nach Anspruch 1 oder 2, wobei das Aminopyridin oder das pharmazeutisch akzeptable Salz davon ein Monoaminopyridin oder ein Diaminopyridin ist, wobei das Aminopyridin oder das pharmazeutisch akzeptable Salz davon vorzugsweise 4-Aminopyridin, 3-Aminopyridin oder 3,4-Diaminopyridin ist.

4. Aminopyridin oder pharmazeutisch akzeptables Salz davon zur Verwendung nach einem der Ansprüche 1 bis 3, zudem umfassend einen Schritt des Beurteilens eines Anzeichens oder Symptoms von CP vor der Verabreichung des Aminopyridins oder des pharmazeutisch akzeptablen Salzes davon und einen Schritt des Beurteilens des gleichen Anzeichens oder Symptoms von CP nach der Verabreichung des Aminopyridins oder des pharmazeutisch akzeptablen Salzes davon.

5. Aminopyridin oder pharmazeutisch akzeptables Salz davon zur Verwendung nach einem der Ansprüche 1 bis 4, wobei das Aminopyridin oder das pharmazeutisch akzeptable Salz davon in einer Retard-Zusammensetzung vorliegt.

6. Aminopyridin oder pharmazeutisch akzeptables Salz davon zur Verwendung nach einem der Ansprüche 1 bis 5, wobei das Aminopyridin oder das pharmazeutisch akzeptable Salz davon dem Patienten einmal täglich oder zweimal täglich verabreicht wird.

7. Aminopyridin oder pharmazeutisch akzeptables Salz davon zur Verwendung nach einem der Ansprüche 1 bis 6, wobei das Aminopyridin in einer Menge im Bereich von 1 bis 20 mg, 5 bis 20 mg, 5 bis 15 mg, 5 bis 10 mg oder 7,5 bis 10 mg einmal oder zweimal täglich verabreicht wird.

8. Aminopyridin oder pharmazeutisch akzeptables Salz davon zur Verwendung nach Anspruch 7, wobei das Aminopyridin in einer Menge von 10 mg einmal täglich oder zweimal täglich verabreicht wird.

9. Aminopyridin oder pharmazeutisch akzeptables Salz davon zur Verwendung nach Anspruch 5, wobei das Aminopyridin oder das pharmazeutisch akzeptable Salz davon in einer Einzeldosis der Retard-Zusammensetzung verabreicht wird.

10. Aminopyridin oder pharmazeutisch akzeptables Salz davon zur Verwendung nach einem der Ansprüche 1 bis 9, wobei das Aminopyridin oder das pharmazeutisch akzeptable Salz davon oral verabreicht wird, wobei das Aminopyridin oder das pharmazeutisch akzeptable Salz davon vorzugsweise in Form einer Tablette oder einer Kapsel formuliert wird.

11. Aminopyridin oder pharmazeutisch akzeptables Salz davon zur Verwendung nach einem der Ansprüche 1 bis 10, wobei das Anzeichen oder Symptom von CP oder die Beeinträchtigung oder Veränderung infolge von CP eine sensomotorische Beeinträchtigung ist, wobei die sensomotorische Beeinträchtigung vorzugsweise eine Beeinträchtigung des Gehens, Beeinträchtigung der Funktion der Gliedmaßen, Beeinträchtigung der Funktion der unteren Extremitäten, Beeinträchtigung der Muskelkraft der unteren Extremitäten, Beeinträchtigung des Muskeltonus, Beeinträchtigung der Funktion der oberen Extremitäten, Beeinträchtigung der Kraft der oberen Extremitäten, Beeinträchtigung der Handfunktion, Beeinträchtigung der Feinkoordination der Hand, Beeinträchtigung der Griffkraft, Beeinträchtigung des Gleichgewichts oder der Koordination, Beeinträchtigung der globalen Körperkontrolle, Dysarthrie, Beeinträchtigung der Kieferfunktion, Beeinträchtigung des Kauens oder Beeinträchtigung der Kieferartikulation ist.

12. Aminopyridin oder pharmazeutisch akzeptables Salz davon zur Verwendung nach einem der Ansprüche 1 bis 10, wobei das Anzeichen oder Symptom von CP oder die Beeinträchtigung oder Veränderung infolge von CP eine motorische Funktionsstörung ist, wobei die motorische Funktionsstörung vorzugsweise an den oberen Extremitäten oder unteren Extremitäten oder in einer Muskelgruppe des Körperkerns oder in der posturalen Muskulatur vorliegt.

13. Aminopyridin oder pharmazeutisch akzeptables Salz davon zur Verwendung nach einem der Ansprüche 1 bis 10, wobei das Anzeichen oder Symptom von CP oder die Beeinträchtigung oder Veränderung infolge von CP eine Geh- oder Gangstörung ist, wobei das Anzeichen oder Symptom von CP oder die Beeinträchtigung oder Veränderung infolge von CP vorzugsweise eine Beeinträchtigung der Gehgeschwindigkeit ist; oder wobei das Anzeichen oder Symptom von CP oder die Beeinträchtigung oder Veränderung infolge von CP mindestens eine(s) von beeinträchtigtem Muskeltonus, beeinträchtigten Reflexen, beeinträchtigter Koordination, Spastik, unwillkürlichen Bewegungen, beeinträchtigtem Gang, beeinträchtigtem Gleichgewicht, verringerter Muskelmasse, einer gestörten Muskelkraft, einer Sprachstörung oder einer Beeinträchtigung der Handkraft oder der manuellen Geschicklichkeit ist.

14. Aminopyridin nach Anspruch 1 zur Verwendung nach einem der Ansprüche 1 bis 13.

15. Pharmazeutisch akzeptables Salz des Aminopyridins nach Anspruch 1 zur Verwendung nach einem der Ansprüche 1 bis 6 oder 9 bis 13.

## Revendications

1. Aminopyridine ou sel pharmaceutiquement acceptable de celle-ci destinés à être utilisés dans une méthode pour le traitement d'un signe ou symptôme de paralysie cérébrale (« PC ») ou d'une déficience ou altération consécutive à une PC, ladite méthode comprenant l'administration de l'aminopyridine ou du sel pharmaceutiquement acceptable de celle-ci à un patient atteint de PC, le symptôme de PC ou la déficience ou altération consécutive à une PC n'étant pas une déficience de l'état mental, de préférence le patient étant humain.

2. Aminopyridine ou sel pharmaceutiquement acceptable de celle-ci destinés à être utilisés selon la revendication 1, ladite aminopyridine ou ledit sel pharmaceutiquement acceptable de celle-ci étant présents dans une composition pharmaceutique comprenant une quantité thérapeutiquement efficace de l'aminopyridine ou d'un sel pharmaceutiquement acceptable de celle-ci et un véhicule pharmaceutiquement acceptable.

3. Aminopyridine ou sel pharmaceutiquement acceptable de celle-ci destinés à être utilisés selon la revendication 1 ou 2, ladite aminopyridine ou ledit sel pharmaceutiquement acceptable de celle-ci étant une monoaminopyridine ou une diaminopyridine, de préférence l'aminopyridine ou le sel pharmaceutiquement acceptable de celle-ci étant la 4-aminopyridine, la 3-aminopyridine ou la 3,4-diaminopyridine.

4. Aminopyridine ou sel pharmaceutiquement acceptable de celle-ci destinés à être utilisés selon l'une quelconque des revendications 1-3, l'utilisation comprenant en outre une étape consistant à évaluer un signe ou symptôme de PC avant l'administration de l'aminopyridine ou du sel pharmaceutiquement acceptable de celle-ci et une étape consistant à évaluer le même signe ou symptôme de PC après l'administration de l'aminopyridine ou du sel pharmaceutiquement acceptable de celle-ci.

5. Aminopyridine ou sel pharmaceutiquement acceptable de celle-ci destinés à être utilisés selon l'une quelconque des revendications 1-4, l'aminopyridine ou le sel pharmaceutiquement acceptable de celle-ci étant dans une composition à libération prolongée.

6. Aminopyridine ou sel pharmaceutiquement acceptable de celle-ci destinés à être utilisés selon l'une quelconque des revendications 1-5, l'aminopyridine ou le sel pharmaceutiquement acceptable de celle-ci étant administrés au patient une fois par jour ou deux fois par jour.

7. Aminopyridine ou sel pharmaceutiquement acceptable de celle-ci destinés à être utilisés selon l'une quelconque des revendications 1-6, l'aminopyridine étant administrée en une quantité dans la plage de 1 à 20 mg, 5 à 20 mg, 5 à 15 mg, 5 à 10 mg ou 7,5 à 10 mg, une fois ou deux fois par jour.

8. Aminopyridine ou sel pharmaceutiquement acceptable de celle-ci destinés à être utilisés selon la revendication 7, l'aminopyridine étant administrée en quantité de 10 mg, une fois par jour ou deux fois par jour.

9. Aminopyridine ou sel pharmaceutiquement acceptable de celle-ci destinés à être utilisés selon la revendication 5, l'aminopyridine ou le sel pharmaceutiquement acceptable de celle-ci étant administrés en une seule dose de la composition à libération prolongée.

10. Aminopyridine ou sel pharmaceutiquement acceptable de celle-ci destinés à être utilisés selon l'une quelconque des revendications 1-9, l'aminopyridine ou le sel pharmaceutiquement acceptable de celle-ci étant administrés par voie orale, de préférence l'aminopyridine ou le sel pharmaceutiquement acceptable de celle-ci étant formulés sous une forme de comprimé ou de capsule.

11. Aminopyridine ou sel pharmaceutiquement acceptable de celle-ci destinés à être utilisés selon l'une quelconque des revendications 1-10, le signe ou symptôme de PC ou la déficience ou altération consécutive à une PC étant une déficience sensori-motrice, de préférence la déficience sensori-motrice étant une déficience de la marche, une déficience de la fonction des membres, une déficience de la fonction des extrémités inférieures, une déficience de la force musculaire des extrémités inférieures, une déficience du tonus musculaire, une déficience de la fonction des extrémités supérieures, une déficience de la force des extrémités supérieures, une déficience de la fonction de la main, une déficience de la coordination fine de la main, une déficience de la force de préhension, une déficience de l'équilibre ou de la coordination, une déficience du contrôle global du corps, une dysarthrie, une déficience de la fonction de la mâchoire, une déficience de la mastication ou une déficience de l'articulation de la mâchoire.

12. Aminopyridine ou sel pharmaceutiquement acceptable de celle-ci destinés à être utilisés selon l'une quelconque des revendications 1-10, le signe ou symptôme de PC ou la déficience ou altération consécutive à une PC étant un dysfonctionnement moteur, de préférence le dysfonctionnement moteur en étant un des extrémités supérieures ou un des extrémités inférieures, ou d'un groupe musculaire du tronc ou de la musculature de posture.

13. Aminopyridine ou sel pharmaceutiquement acceptable de celle-ci destinés à être utilisés selon l'une quelconque des revendications 1-10, le signe ou symptôme de PC ou la déficience ou altération consécutive à une PC étant un dysfonctionnement de la marche ou de l'ambulation, de préférence le signe ou symptôme de PC ou la déficience ou altération consécutive à une PC étant une déficience de la vitesse de marche ; ou le signe ou symptôme de PC ou la déficience ou altération consécutive à une PC en étant au moins un parmi : un tonus musculaire atténué, des réflexes atténués, une coordination altérée, une spasticité, des mouvements involontaires, une démarche altérée, un équilibre atténué, une masse musculaire diminuée, une force musculaire dysfonctionnelle, un dysfonctionnement du langage ou une déficience de la force dans la main ou de la dextérité manuelle.

14. Aminopyridine selon la revendication 1 destinée à être utilisée selon l'une quelconque des revendications 1-13.

15. Sel pharmaceutiquement acceptable de l'aminopyridine selon la revendication 1 destiné à être utilisé selon l'une quelconque des revendications 1-6 ou 9-13.
